Europäisches Patentamt

European Patent Office

Office européen des brevets

(11) Publication number: **0 231 883 B1**

(12) **EUROPEAN PATENT SPECIFICATION**

(45) Date of publication of patent specification: **02.09.92**

(51) Int. Cl.⁵: **C12N 15/58**, C12N 9/64, C12N 9/72, C12N 1/21, //(C12N1/21,C12R1:13)

(21) Application number: **87101209.2**

(22) Date of filing: **29.01.87**

The file contains technical information submitted after the application was filed and not included in this specification

(54) **Hybrid plasminogen activator-like polypeptide.**

(30) Priority: **31.01.86 JP 17734/86**

(43) Date of publication of application:
**12.08.87 Bulletin 87/33**

(45) Publication of the grant of the patent:
**02.09.92 Bulletin 92/36**

(84) Designated Contracting States:
**BE CH DE ES FR GB IT LI NL SE**

(56) References cited:
**EP-A- 0 093 619**
**EP-A- 0 151 308**
**EP-A- 0 155 387**

**BIOTECHNOLOGY, vol. 3, no. 10, October 1985, pages 923-929, New York, US; W.E. HOLMES et al.: "Cloning and expression of the gene for pro-urokinase in Escherichia coli"**

(73) Proprietor: **SAGAMI CHEMICAL RESEARCH CENTER**
**4-5, Marunouchi 1-chome**
**Chiyoda-ku Tokyo 100(JP)**

Proprietor: **CENTRAL GLASS COMPANY, LIMITED**
**5253, Oaza Okiube Ube-shi**
**Yamaguchi 755(JP)**

Proprietor: **HODOGAYA CHEMICAL COMPANY, LIMITED**
**4-2, Toranomon 1-chome Minato-ku**
**Tokyo 105(JP)**

Proprietor: **NIPPON SODA CO., LTD.**
**2-1, Ohtemachi 2-chome**
**Chiyoda-ku, Tokyo 100(JP)**

Proprietor: **NISSAN CHEMICAL INDUSTRIES LTD.**
**3-7-1, Kanda Nishiki-cho**
**Chiyoda-ku Tokyo(JP)**

Rank Xerox (UK) Business Services

PROCEEDINGS OF THE NATIONAL ACADEMY OF SCIENCES OF THE USA, vol. 81, no. 17, September 1984, pages 5355-5359, Washington, US; T. NY et al.: "The structure of the human tissue-type plasminogen activator gene: Correlation of intron and exon structures to functional and structural domains"

Proprietor: **Tosoh Corporation**
**4560, Oaza Tonda**
**Shinnanyo-shi Yamaguchi-ken(JP)**

(72) Inventor: **Tagawa, Michito**
**3-16, Sakae-cho**
**Sagamihara-shi Kanagawa(JP)**
Inventor: **Wada, Masakatsu**
**Miyahaimu 102, 2-2-62 Higashionuma**
**Sagamihara-shi, Kanagawa(JP)**
Inventor: **Yamada, Masayuki**
**4-41, Nishionuma**
**Sagamihara-shi Kanagawa(JP)**
Inventor: **Yokoyama, Midori**
**Daiichi Tachibana-so 201 5-4-7, Sagamiono**
**Sagamihara-shi Kanagawa(JP)**
Inventor: **Numao, Naganori**
**1-9-2, Minamidai**
**Sagamihara-shi Kanagawa(JP)**

(74) Representative: **Patentanwälte Grünecker,**
**Kinkeldey, Stockmair & Partner**
**Maximilianstrasse 58**
**W-8000 München 22(DE)**

## Description

### BACKGROUND OF THE INVENTION

#### 1. Field of the Invention

The present invention relates to hybrid plasminogen activator-like polypeptides, and a process for production thereof using genetic engineering. The hybrid plasminogen activator-like polypeptides according to the present invention can be used for the prevention and treatment of various kinds of cardiovascular disorders or diseases.

#### 2. Description of the Related Art

Plasminogen activator hydrolyses, in a limited fashion, inactive-type enzyme plasminogen present in plasma, to convert it into active-type enzyme plasmin. The plasmin can hydrolyse fibrin clots generated in a blood vessel and resulting in lysis of thrombus, i.e., fibrinolysis. Since the plasmin is rapidly inactivated in vivo by a plasmin inhibitor present in plasma, a plasminogen activator is clinically used as a thrombolytic agent.

As a plasminogen activator, at present, urokinase isolated from human urine is clinically used. However, urokinase has a disadvantage in that, when administered in a large amount, it can result in systemic hemorrhage. The systemic hemorrhage depends on the ratio of fibrinogen-degradation activity and fibrin-degradation activity of the plasminogen activator. Namely, the systemic hemorrhage depends on the affinity of the plasminogen activator to fibrin. Therefore, attempts have been made to produce urokinase having an affinity to fibrin.

On the other hand, a tissue plasminogen activator has a high affinity to fibrin, and therefore, has been considered as a replacement for urokinase. However, the tissue plasminogen activator has an enzyme activity lower than the urokinase, and is less stable in the blood.

Therefore, there is a demand for a new type of plasminogen activator having a satisfactory affinity to fibrin and a sufficient enzyme activity, as well as a satisfactory stability in vivo.

### SUMMARY OF THE INVENTION

The present invention, therefore, provides hybrid plasminogen activator-like polypeptides comprising a polypeptide region responsible for an affinity to fibrin derived from tissue plasminogen activator joined by a peptide bond to a polypeptide region responsible for enzyme activity derived from prourokinase.

The present invention also provides a gene system used for the production of the above-mentioned polypeptide, including DNA segments coding for the polypeptide, plasmids containing the DNA segment, and microorganisms transformed with the plasmid.

There is also provided a process for the production of the hybrid plasminogen activator-like polypeptide comprising culturing the above-mentioned microorganism, and recovering the polypeptide from the cultured product.

### BRIEF DESCRIPTION OF THE DRAWINGS

Figures 1-1 to 1-5 represent a nucleotide sequence of a cDNA insert coding for human tissue plasminogen activator in plasmid pDPA3, and a corresponding amino acid sequence in an open reading frame contained in the cDNA insert. In the amino acid sequence, a part of the sequence shown by lower-case letters represents a leader sequence, and a part of the sequence shown by capital letters represents an amino acid sequence of the tissue plasminogen activator;

Figs. 2-1 to 2-5 represent a nucleotide sequence of a cDNA insert coding for human prourokinase in plasmid pKU22, and a corresponding amino acid sequence in an open reading frame contained in the cDNA insert. In the amino acid sequence, a part of the sequence shown by lower-case letters represents a leader sequence, and a part of the sequence represented by capital letters represents an amino acid sequence of human prourokinase;

Fig. 3 is a schematic illustration of embodiments of the hybrid polypeptide of the present invention;

Fig. 4 is a flow chart showing the construction of plasmids containing a gene coding for human prourokinase;

Fig. 5 is a flow chart showing the construction of plasmids containing a gene coding for a hybrid polypeptide of the present invention;

Fig. 6 represents the construction of plasmid pKYU22 from plasmids pPE3 and pKYU21;

Fig. 7 represents the restriction endonuclease map of plasmid pPE3;

Fig. 8 represents the construction of plasmid pKMU1 from plasmid pKYU22 and a synthetic oligodeoxyribonucleotide;

Fig. 9 represents the construction of plasmid pMUT4L;

Fig. 10 represents the construction of a single-stranded phage DNA containing a 1200 bp insert from plasmid pKYU22 and a phage M13pm8 RF DNA;

Fig. 11 represent a process for the introduction of a point mutation using a primer;

Fig. 12 represents the construction of plasmid pMUT4Lpm3 from a mutant phage M13 double stranded DNA (pm3) and plasmid pMUT4L;

Fig. 13 represents the construction of plasmid pDPAT2 from plasmids pYTU3, pDPA3 and pK12;

Fig. 14 represents the construction of plasmid pHA00 from plasmids pDPAT2 and pMUT4L (also designated as pPE3p), and the construction of plasmid pHA03 from plasmids pDPAT2 and pMUT4Lpm3 (also designated as pPE3pm3);

Fig. 15 represents the construction of plasmid pHA20 from plasmids pDPAT2 and pHA00, and the construction of plasmid pHA23 from plasmids pDPAT2 and pHA03;

Fig. 16 represents the construction of plasmid pHA13 from plasmid pHA23;

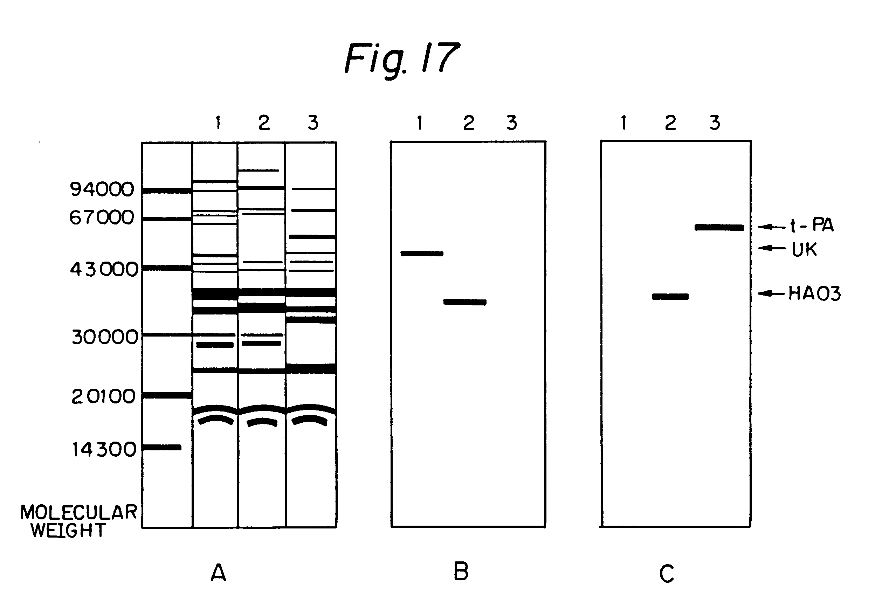

Fig. 17 represents a result of electrophoresis wherein an expression product from Escherichia coli JM103/pHA03 (lane 2) is compared with an expression product from E. coli JM103/pPE3 (pMUT4L) producing native human prourokinase (UK) (lane 1), and an expression product from E. coli JM103/pDPAT2 producing native human tissue plasminogen activator (t-PA) (lane 3). In this figure, A represents a composition of proteins stained by Coomassie Blue; B represents products reactive with an anti-urokinase antibody developed by a enzyme stain method; and C represents products reactive with an anti-tissue plasminogen activator antibody developed by a enzyme stain method;

Figs. 18-1 to 18-4 represent elution profiles of affinity chromatography showing the fibrin-affinity of various kinds of plasminogen activators;

Fig. 19 represents the construction of plasmid pMUT9Lpml from plasmids pMUT4L and pMUT4pml;

Fig. 20 represents the construction of plasmid pHA21L from plasmid pHA20 and pMUT9Lpml, and plasmid pHA24L from plasmids pHA20 and pMUT4Lpm4;

Fig. 21 represents the construction of plasmid pHA11L from plasmids pHA21L and pMUT9Lpml;

Fig. 22 represents the construction of plasmid pHAO1L from plasmid pHA21L; and

Fig. 23 represents SDS polyacrylamide gel electrophoresis for an insoluble fraction (lane 2) and supernatant (lane 1) of an expression product of plasmid pHA24L.

DESCRIPTION OF THE PREFERRED EMBODIMENT

A. Hybrid plasminogen activator-like polypeptide

The hybrid plasminogen activator-like polypeptide of the present invention comprises a polypeptide region responsible for an affinity to fibrin derived from tissue plasminogen activator and a polypeptide region responsible for enzyme activity derived from prourokinase.

The tissue plasminogen activator is a protein comprising a polypeptide consisting of about 500 amino acids, and including a polypeptide region responsible for the affinity to fibrin at its N-terminal half. The hybrid plasminogen activator-like polypeptide of the present invention includes, as its N-terminal part, a polypeptide region responsible for the affinity to fibrin. This part of the present polypeptide is derived from a native tissue plasminogen activator polypeptide. That is, this part of the present polypeptide may be a whole polypeptide of the native tissue plasminogen activator, or a part thereof responsible for its affinity to fibrin, or a modification thereof. This modification is carried out by the addition of one or same amino acids to the native form, or the deletion of one or some amino acids from the native form, or the replacement of one or some amino acids in the native form, substantially maintaining the fibrin affinity of the native form. When a part of the native tissue plasminogen activator polypeptide is used for the region responsible for the fibrin affinity, the length of that part can differ widely to an extent wherein the fibrin affinity is maintained.

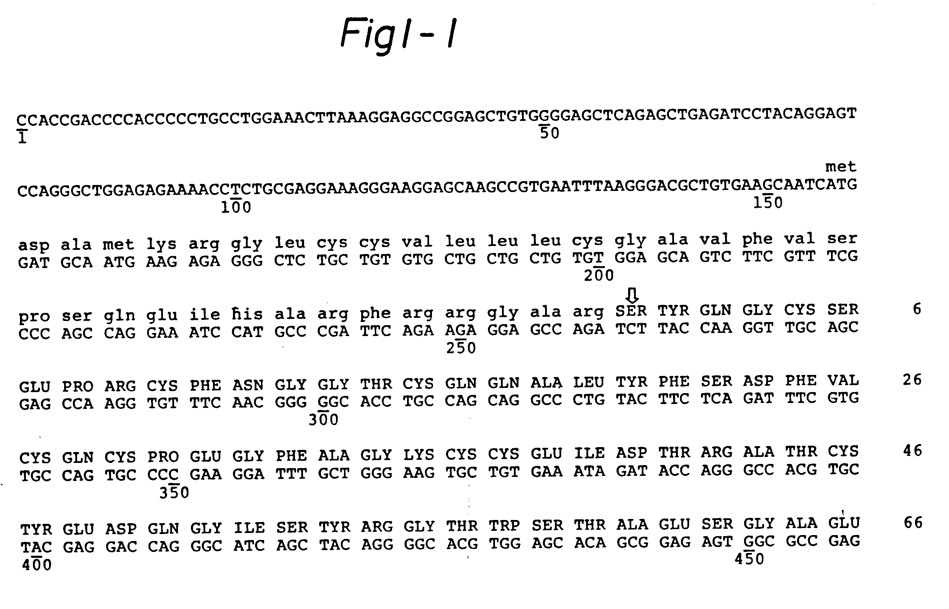

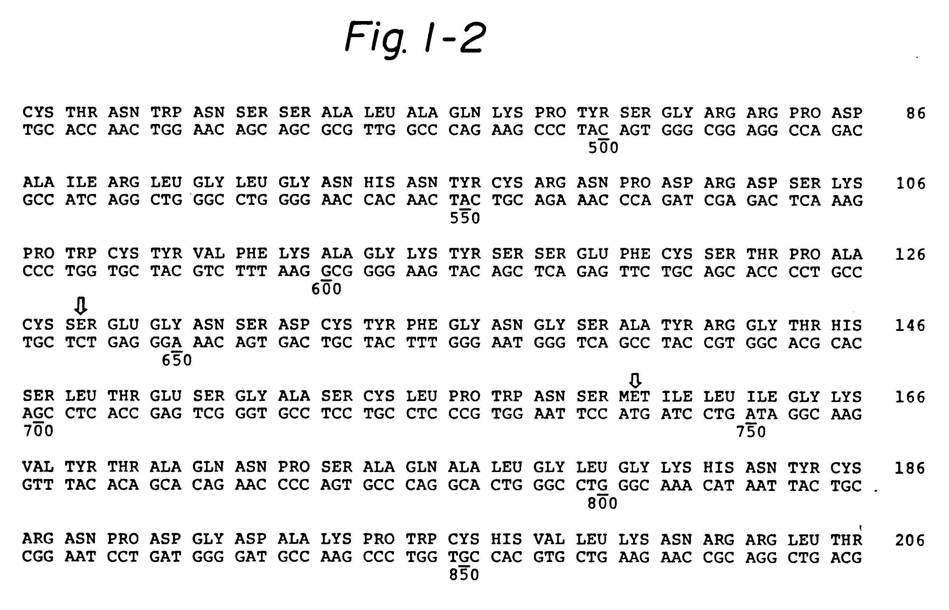

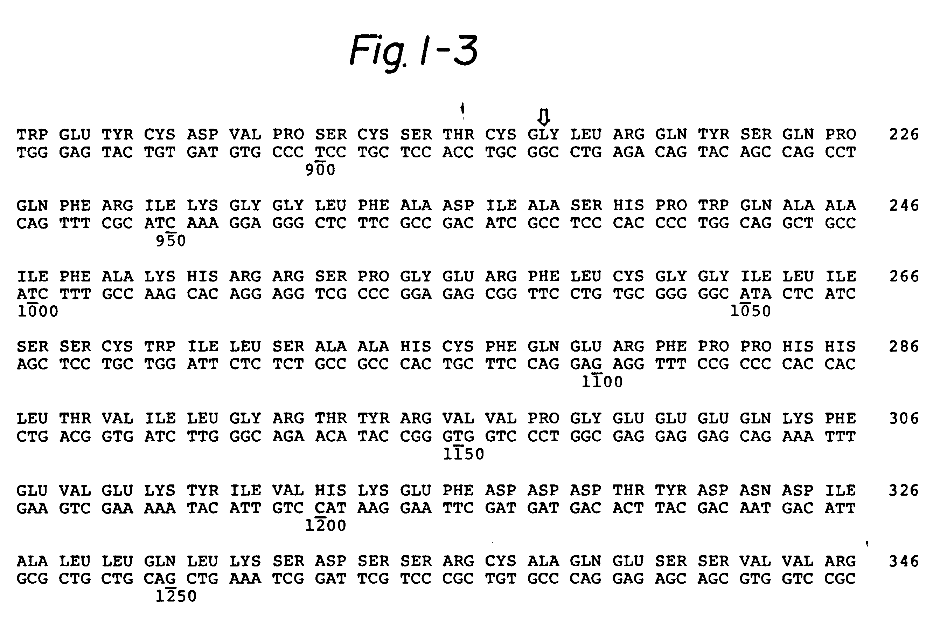

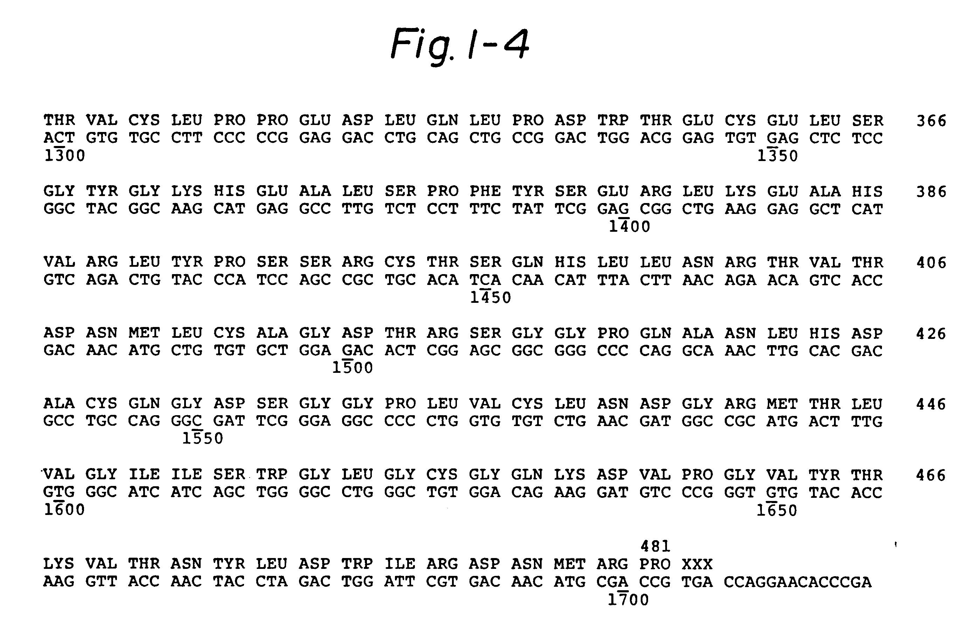

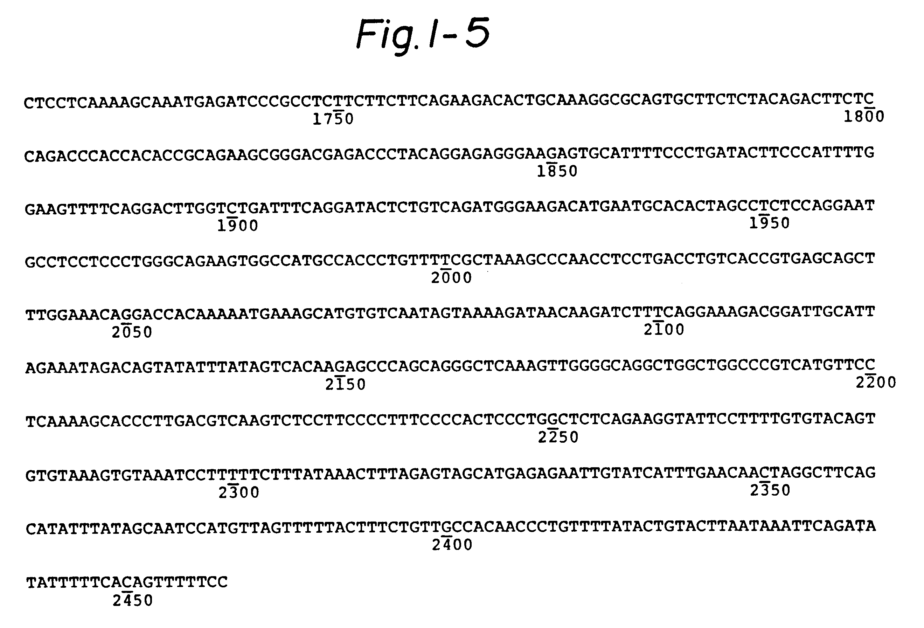

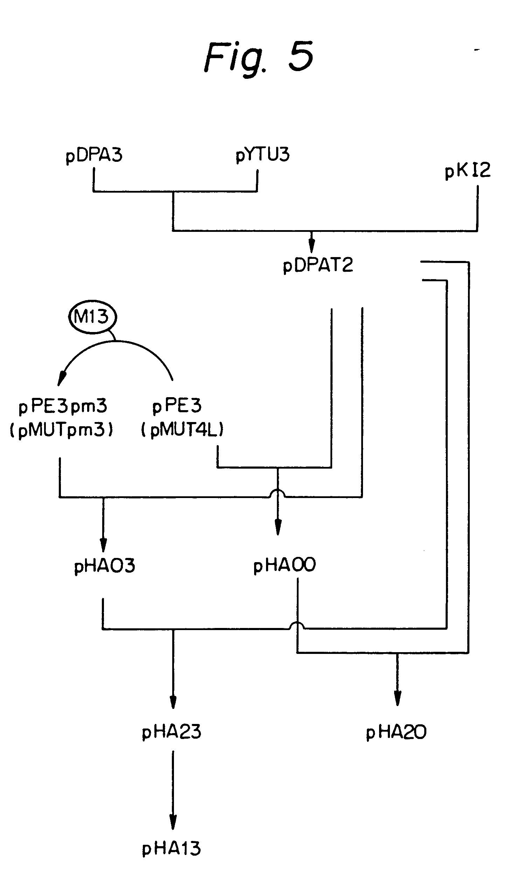

For example, the present inventors prepared hybrid polypeptides which contain, as an N-terminal half, about two kringles consisting of an amino acid sequence from N-terminal first serine to 219th glycine of the human tissue plasminogen activator calculated from the N-terminal serine as shown in Figs. 1-1 to 1-3; about one kringle consisting of an amino acid sequence from 128th serine to 219th glycine calculated from N-terminal serine as the first amino acid, as shown in Figs. 1-2 to 1-3; or about a half of a kringle consisting of an amino acid sequence from 161th methionine to 219th glycine calculated from N-terminal serine as the first amino acid, as shown in Figs. 1-2 to 1-3. These polypeptides were then tested for their affinity to fibrin, and it was confirmed that all of these polypeptides have fibrin affinity. Therefore, the fibrin affinity region of the present hybrid polypeptide can be any polypeptide region of the tissue plasminogen activator containing a region responsible for the fibrin affinity. Figs. 1-1 to 1-5 represent an nucleotide sequence coding for human tissue plasminogen activator and a corresponding amino acid sequence used as the fibrin affinity region of the present hybrid polypeptide, wherein first serine, 128th serine, 161th methionine, and 219th glycine are shown by arrows.

The prourokinase is a protein comprising a polypeptide consisting of 411 amino acids from N-terminal serine to C-terminal leucine, and including a polypeptide region responsible for enzyme activity at its C-terminal half. The hybrid plasminogen activator-like polypeptide of the present invention includes, as its C-terminal half, a polypeptide region responsible for enzyme activity. This part of the present polypeptide is derived from a prourokinase polypeptide. That is, this part of the present polypeptide may be a whole polypeptide of the native prourokinase or a part thereof responsible for its enzyme activity, or a modification thereof. This modification is carried out by the addition of one or some amino acids to the native form, or the deletion of one or some amino acids from the native form, or the replacement of one or some amino acids in the native form, substantially maintaining the enzyme activity. When, a part of the prourokinase polypeptide is used for the region responsible for the enzyme activity, the length of the part can differ widely to an extent wherein the enzyme activity is maintained.

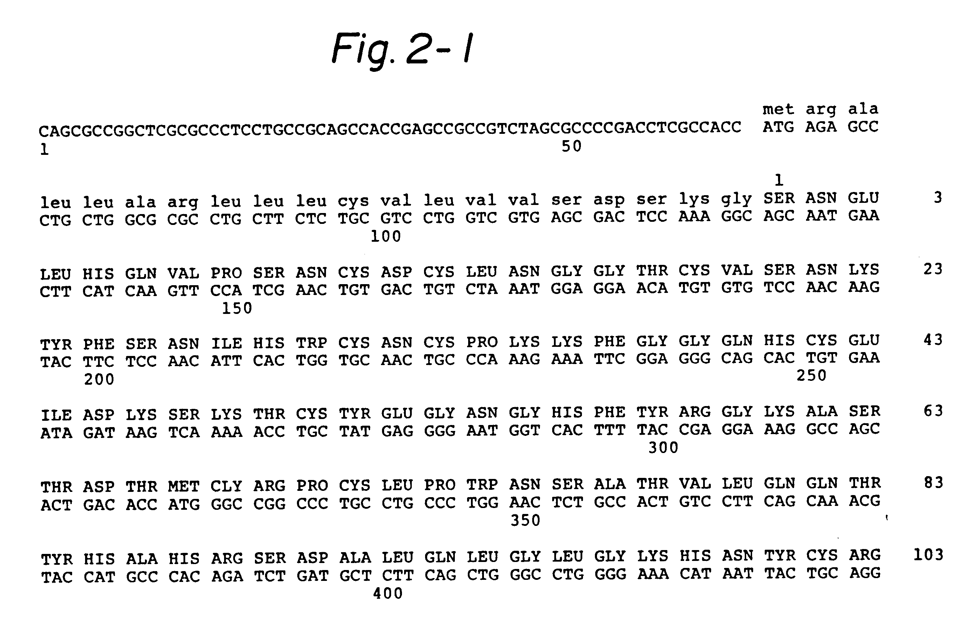

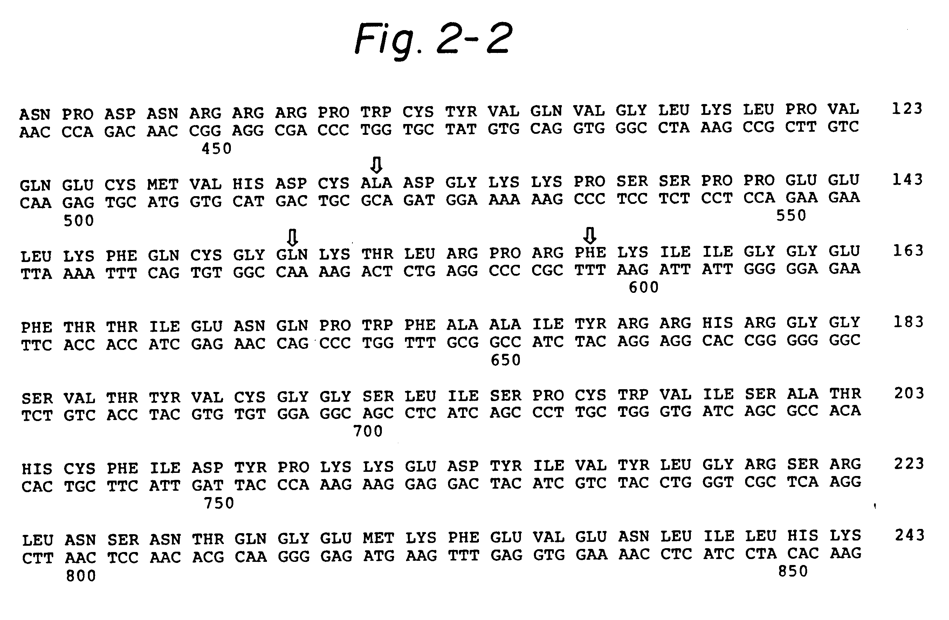

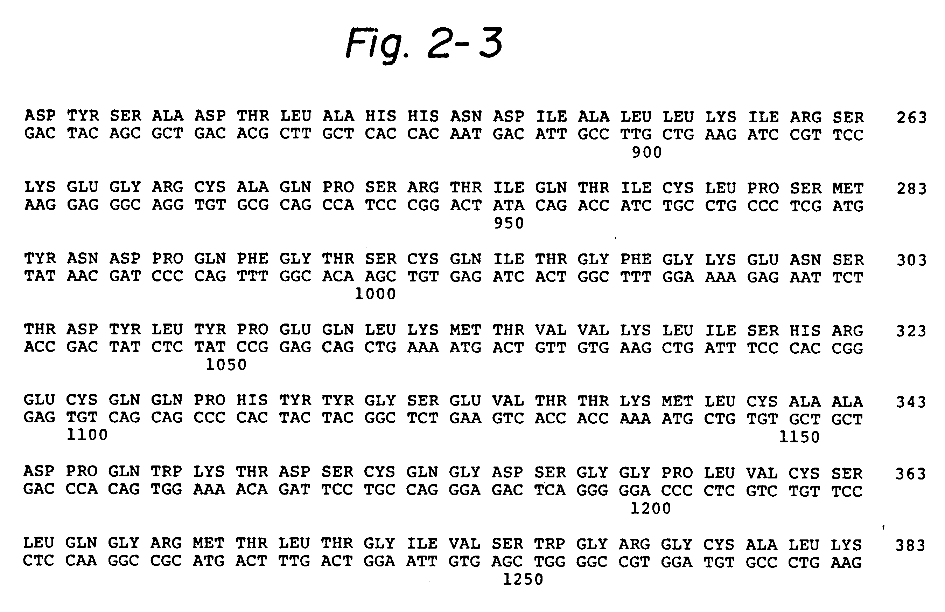

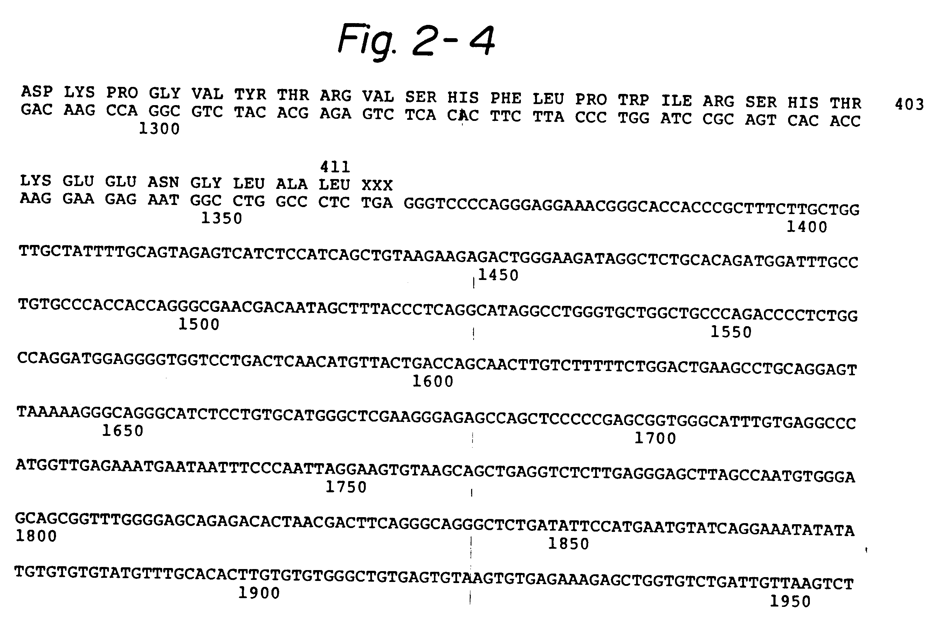

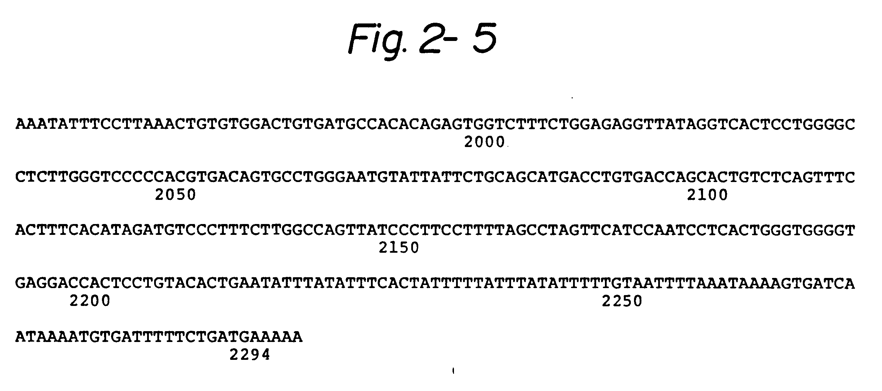

For example, considering the convenience of gene manipulation, the present inventor prepared hybrid polypeptides which contain, as a C-terminal half, an amino acid sequence from 150th glutamine to C-terminal 411th leucine calculated from N-terminal serine as the first amino acid, as shown in Figs. 2-2 to 2-4, or an amino acid sequence from 132th alanine to C-terminal 411th leucine calculated from N-terminal serine as the first amino acid, as shown in Figs. 2-2 to 2-4. These hybrid polypeptides were then tested for enzyme activity, and it was confirmed that these polypeptides have enzyme activity.

Prourokinase, which is an inactive form, is activated by cleavage of the polypeptide between 158th lysine and 159th isoleucine to generate active urokinase, and the active urokinase is degraded rather rapidly. Therefore, it is expected that a hybrid plasminogen activator-like polypeptide, which it is difficult to cleave at the above-mentioned position, is sustained in vivo. The present inventor prepared a hybrid plasminogen-like polypeptide wherein 157th phenylalanine shown in Fig. 2-2 is changed to an acidic amino acid such as aspartic acid or glutamic acid, and confirmed that such polypeptides have an enhanced resistance against proteases such as plasmin, thrombin, and trypsin. Therefore, in a preferable embodiment of the present polypeptide, 157th phenylalanine is replaced by an acidic amino acid such as aspartic acid or glutamic acid. Figures 2-1 to 2-5 represent an nucleotide sequence coding for human prourokinase and a corresponding amino acid sequence used on the enzyme activity region of the present hybrid polypeptide, wherein 132th alanine, 150th glutamine, and 157th phenylalanine are shown by arrows.

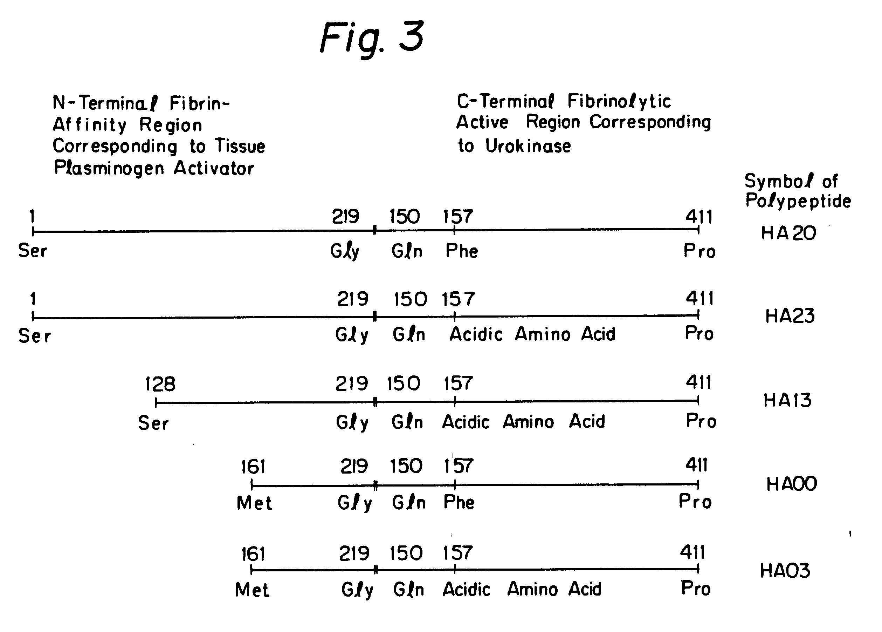

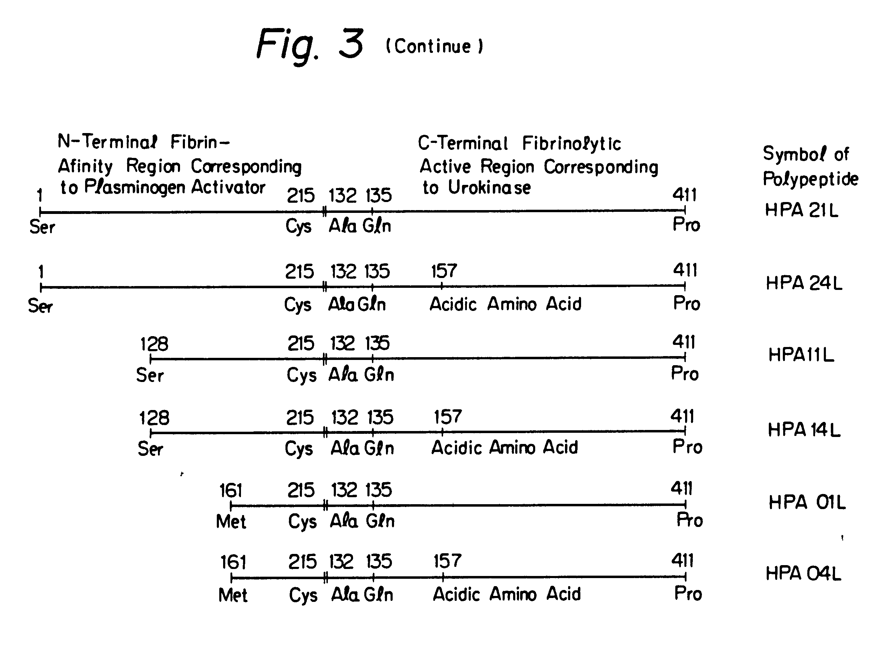

Therefore, in a preferable embodiment, the hybrid plasminogen activator-like polypeptide of the present invention comprises an N-terminal region corresponding to an N-terminal region of human tissue plasminogen activator containing a polypeptide region responsible for its affinity to fibrin and a C-terminal region corresponding to the C-terminal region of human prourokinase containing a polypeptide region responsible for its enzyme activity, wherein 157th phenylalanine shown in Fig. 2-2 is optionally replaced by aspartic acid or glutamic acid. In the prourokinase, a peptide bond between 135th lysine and 136th lysine is susceptible to trypsin-type protease such as plasmin. Therefore, to prevent the cleavage of the peptide bond, in a preferable embodiment of the present invention, 135th amino acid lysine is replaced with an amino acid other than basic amino acid. As the amino acid other than basic amino acid, any neutral amino acid or acidic amino acid which does not adversely affect the physiological property of the desired polypeptide may be used and, for example, alanine, asparagine, aspartic acid, glutamine, glutamic acid, phenylalanine, glycine, isoleucine, leucine, methionine, serine, threonine, valine, tryptophan, tyrosine, and proline may be cited. Embodiments of the present hybrid polypeptides are shown in Fig. 3.

B. Gene systems

The present invention also provides gene systems for the production of the above-mentioned hybrid plasminogen activator-like polypeptide, including gene segments coding for the hybrid polypeptides, plasmides containing such gene segments, and microorganisms transformed with such plasmids.

Gene coding for the hybrid polypeptide

The gene codes for the above-mentioned hybrid plasminogen activator-like polypeptide consist of codons which can be expressed in a host microorganism chosen for the gene expression such as E. coli. For convenience in the construction of the gene, it is preferably obtained by linking a relevant part of cDNA derived from mRNA coding for human tissue plasminogen activator to a relevant part of cDNA derived from mRNA coding for human prourokinase. Figures 1-1 to 1-5 show cDNA coding for human tissue plasminogen activator, together with a corresponding amino acid sequence; and Figs. 2-1 to 2-5 show cDNA coding for human prourokinase together with a corresponding amino acid sequence.

Plasmid

Although plasmids that function in yeast or animal cells may be used for expression of the hybrid plasminogen activator-like polypeptide of the present invention, the use of a plasmid containing along with said coding region the expression control region necessary for expressing said genes in microorganisms, particularly in E. coli and the region necessary for replication in E. coli is preferred. As the expression control region any system useful for expressing the foreign gene in E. coli may be used arbitrarily. For example, as the promoter/operator systems, tac, $P_L$, lacUV5, $P_R$, trp, and 1pp are used. In particular, tac promoter/operator, $P_L$ promotor/operator, and trp promoter/operator are preferred. As an example of SD sequences, the SD sequence of the metapyrocatechase gene (C230SD) (literature 1) and lacSD may be used.

Host Microorganism

Although animal cells and such microorganisms as bacteria and yeast may be used as hosts for introducing said plasmids, the use of bacteria, particularly E. coli, is preferred.

As host E. coli of the present invention, such strains of nonenterositic, nontoxic E. coli as those derived from E. coli K-12 may be used, e.g., JM83, JM103, JM105, RB791, SM32, N99, RR1, W3110, and $_x$1776.

Construction of Gene System

An example of the process for constructing genes involved in the present invention comprises the following steps:

(1) preparing a gene coding for a tissue plasminogen activator, and obtaining a DNA fragment coding for a polypeptide region having an affinity to fibrin;

(2) preparing a gene coding for prourokinase, and obtaining a DNA fragment coding for a polypeptide region having enzyme activity; and

(3) joining together the DNA fragments obtained in steps (1) and (2) in an appropriate plasmid to construct a gene coding for the hybrid plasminogen activator-like polypeptide.

In the case wherein 157th phenylalanine is replaced by an acidic amino acid such as aspartic acid or glutamic acids, during step (2) a codon coding for the 157th phenylalanine is converted to a codon coding for aspartic acid or glutamic acid.

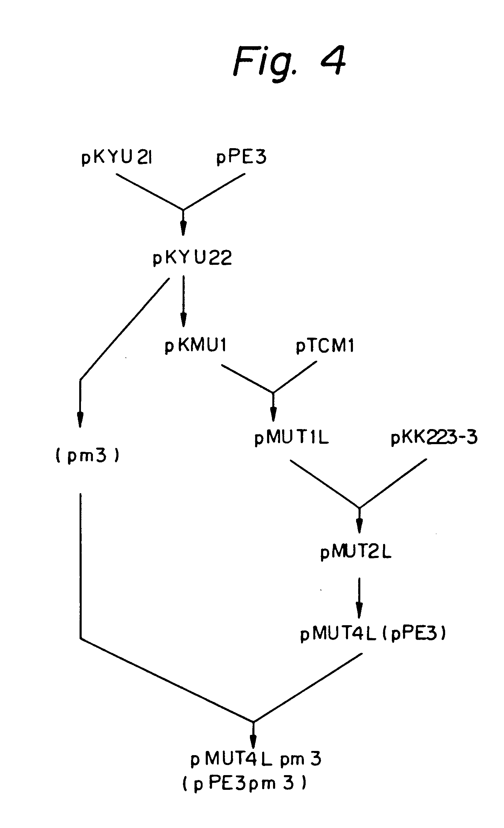

As an origin of a DNA region coding for a polypeptide region having enzyme activity, plasmid pMUT4L (also designated as pPE3) containing a gene coding for a native human prourokinase, and plasmid pMUT4Lpm3 (also designated as pPE3pm3) containing a gene coding for a stabilized human prourokinase-like polypeptide wherein the 157th amino acid is an acidic amino acid are constructed according to a flow chart shown in Fig. 4. Detailed construction processes for these plasmids are disclosed in reference examples 4 to 15.

As an origin of a DNA region coding for a polypeptide region having the fibrin affinity, plasmid pDPA3 containing a gene coding for a human tissue plasminogen activator is constructed according to processes disclosed in detail in reference examples 1 to 3.

Plasmids containing a gene coding for the hybrid plasminogen activator-like polypeptide of the present invention can be constructed from the above-mentioned plasmids pDPA3, pMUT4L (pPE3) and pMUT4Lpm3 (pPE3pm3), and vector plasmids pYTU3 and pK12.

The plasmid pYTU3 contains $P_L$ promoter and C230 SD sequence, and was constructed according to a procedure described in Japanese Unexamined Patent Publication No. 61-158787; and E. coli HB101/pYTU3 containing the plasmid pYTU3 was deposited with the Fermentation Research Institute, Agency of Industrial Science and Technology (1-3, Higashi 1-chome, Yatabe-cho, Tsukuba-gun, Ibaraki-ken, Japan) (F.R.I.), on December 11, 1984, as FERM P-7992. The plasmid pKl2 contains tac promoter/operator and C230 SD sequence, and was constructed according to a procedure described in Japanese Unexamined Patent Publication No. 61-158787; and E. coli JM103/pK12 containing the plasmid pK12 was deposited with the F.R.I. on December 11, 1984 as FERM P-7996.

Plasmid pDPAT2 constructed in Example 1 contains a gene coding for mature human tissue plasminogen activator under the control by the tac promoter/operator and C230 SD sequence, and can express the mature human tissue plasminogen activator, or is used as an origin of a gene region coding for a polypeptide having the fibrin affinity for the construction of plasmids of the present invention, as described hereinafter. E. coli JM103/pDPAT2 containing the plasmid pDPAT2 was deposited with the Deutsche Sammlung von Microorganismen Gesellschaft fur Biotechnologische Forschung mbH (DSM) as an international deposition under the Budapest Treaty on the International Recognition of the Deposit of Microorganisms for the Purpose of Patent Procedure (Budapest Treaty) on December 28, 1985, and has the deposition number DSM 3629.

Plasmid pHA00 constructed in Example 2 contains a gene coding for a hybrid plasminogen activator-like polypeptide (HA00) comprising as its N-terminal half, a polypeptide region from 161th methionine to 219th glycine of a human tissue plasminogen activator calculated from N-terminal serine as the first amino acid, and as its C-terminal half, a polypeptide region from 150th glutamine to 411 C-terminal leucine of human prourokinase calculated from N-terminal serine as the first amino acid, under the control by the tac promoter and C230 SD sequence, and can express the above-mentioned hybrid polypeptide in E. coli. E. coli JM103/pHA00 containing the plasmid pHA00 was deposited with DSM as DSM3628 on December 28, 1985 under the Budapest Treaty.

Plasmid pHA03 constructed in Example 3 contains a gene coding for a hybrid plasminogen activator-like polypeptide comprising as its N-terminal half, a polypeptide region from 161th methionine to 219th glycine of human tissue plasminogen activator calculated from N-terminal serine as the first amino acid, and as its C-terminal half, a polypeptide region from 150th glutamine to 411th N-terminal leucine of human prourokinase calculated from N-terminal serine as the first amino acid wherein 157th phenylalanine is replaced by aspartic acid, under the control by the tac promoter/operator and C230 SD sequence, and can express the above-mentioned hybrid polypeptide in E. coli. E. coli JM103/pHA03 containing the plasmid pHA03 was deposited with DSM as DSM3627 on December 28, 1985 under the Budapest Treaty.

Plasmid pHA20 constructed in Example 4 contains a gene coding for a hybrid plasminogen activator-like polypeptide (HA20) comprising as its N-terminal half, a polypeptide region from N-terminal serine at amino acid position 1 to 219th glycine of human tissue plasminogen activator, and as its C-terminal half, a polypeptide region from 150th glutamine to 411th C-terminal leucine of human prourokinase calculated from N-terminal serine as the first amino acid, under the control by the tac promoter/operator and C230 SD sequence, and can express the above-mentioned hybrid polypeptide in E. coli. E. coli JM103/pHA20 containing the plasmid pHA20 was deposited with DSM as DSM3626 on December 28, 1985 under the Budapest Treaty.

Plasmid pHA23 constructed in Example 5 contains a gene coding for a hybrid plasminogen activator-like polypeptide (HA23) comprising as its N-terminal half, a polypeptide region from N-terminal serine at amino acid position 1 to 219th glycine of human tissue plasminogen activator, and as its C-terminal half, a polypeptide region from 150th glutamine to 411th C-terminal leucine of human prourokinase calculated from N-terminal serine as the first amino acid wherein 157th phenylalanine is replaced by aspartic acid, under the control by the tac promoter and C230 SD sequence, and can express the above-mentioned hybrid polypeptide in E. coli. E. coli JM103/pHA23 containing the plasmid pHA23 was deposited with DSM as DSM3625 on December 28, 1985 under the Budapest Treaty.

Plasmid pHA13 constructed in Example 6 contains a gene coding for a hybrid plasminogen activator-like polypeptide comprising as its N-terminal half, a polypeptide region from 128th serine to 219th glycine of human tissue plasminogen activator calculated from N-terminal serine as the first amino acid, and as its C-terminal half, a polypeptide region from 150th glutamine to 411th C-terminal leucine of human prourokinase calculated from N-terminal serine on the first amino acid wherein 157th phenylalanine is replaced by aspartic acid, under the control by the tac promoter/operator and C230 SD sequence, and can express the above-mentioned hybrid polypeptide.

Plasmid pHA21L constructed in Example 11 contains a gene coding for a hybrid plasminogen activator-like polypeptide (HPA21L) comprising as its N-terminal half, a polypeptide region from N-terminal serine at amino acid position 1 to 215th cysteine of human tissue plasminogen activator calculated from N-terminal serine as the first amino acid, and as its C-terminal half, a polypeptide region from 132th alanine to 411th N-terminal leucine of human prourokinase calculated from N-terminal serine as the first amino acid wherein 135th lysine is replaced by glutamine, under the control by the tac promoter/operator and C230 SD sequence, and can express the above-mentioned hybrid polypeptide in E. coli. E. coli JM103/pHA21L containing the plasmid pHA21L was deposited with DSM as DSM 3951 on January 6, 1987 under the Budapest Treaty.

Plasmid pHA24L constructed in Example 12 contains a gene coding for a hybrid plasminogen activator-like polypeptide (HPA24L) comprising as its N-terminal half, a polypeptide region from N-terminal serine at amino acid position 1 to 215th cysteine of human tissue plasminogen activator, and as its C-terminal half, a polypeptide region from 132th alanine to 411th C-terminal leucine of human prourokinase calculated from N-terminal serine as the first amino acid, under the control by the tac promoter/operator and C230 SD sequence wherein 135th lysine is replaced by glutamine and 157th phenylalanine is replaced by aspartic acid, and can express the above-mentioned hybrid polypeptide in E. coli. E. coli JM103/pHA24L containing the plasmid pHL24L was deposited with DSM as DSM 3952 on January 6, 1987 under the Budapest Treaty.

Plasmid pHA11L constructed in Example 13 contains a gene coding for a hybrid plasminogen activator-like polypeptide (HPA11L) comprising as its N-terminal half, a polypeptide region from 128th serine to 215th cysteine of human tissue plasminogen activator, and as its C-terminal half, a polypeptide region from 132th alanine to 411th C-terminal leucine of human prourokinase calculated from N-terminal serine as the first amino acid wherein 135th lysine is replaced by glutamine, under the control by the tac promoter and C230 SD sequence, and can express the above-mentioned hybrid polypeptide in E. coli.

Plasmid pHA14L constructed in Example 14 contains a gene coding for a hybrid plasminogen activator-like polypeptide (HPA14L) comprising as its N-terminal half, a polypeptide region from 128th serine to 215th cysteine of human tissue plasminogen activator calculated from N-terminal serine as the first amino acid, and as its C-terminal half, a polypeptide region from 132th alanine to 411th C-terminal leucine of human prourokinase calculated from N-terminal serine as the first amino acid wherein 135th lysine is replaced by glutamine and 157th phenylalanine is replaced by aspartic acid, under the control by the tac promoter/operator and C230 SD sequence, and can express the above-mentioned hybrid polypeptide.

Plasmid pHA01L constructed in Example 15 contains a gene coding for a hybrid plasminogen activator-like polypeptide (HPA01L) comprising as its N-terminal half, a polypeptide region from 161th methionine to 215th cysteine of human tissue plasminogen activator calculated from N-terminal serine as the first amino acid, and as its C-terminal half, a polypeptide region from 132th alanine to 411th N-terminal leucine of human prourokinase calculated from N-terminal serine as the first amino acid wherein 135th lysine is replaced by glutamine, under the control by the tac promoter/operator and C230 SD sequence, and can express the above-mentioned hybrid polypeptide in E. coli.

Plasmid pHA04L constructed in Example 16 contains a gene coding for a hybrid plasminogen activator-like polypeptide (HPA04L) comprising as its N-terminal half, a polypeptide region from 161th methionine to 215th cysteine of human tissue plasminogen activator calculated from N-terminal serine as the first amino acid, and as its C-terminal half, a polypeptide region from 132th alanine to 411th N-terminal leucine of human prourokinase calculated from N-terminal serine as the first amino acid wherein 135th lysine is replaced by glutamine and 157th phenylalanine is replaced by aspartic acid, under the control by the tac promoter/operator and C230 SD sequence, and can express the above-mentioned hybrid polypeptide in E. coli.

D. Expression of gene and purification of hybrid polypeptides

E. coli transformed with the above-mentioned plasmid is cultured, for example, in L-medium supplemented with 100 $\mu$g/ml of ampicillin, and when the cell concentration reaches an absorbance of 0.3 to 0.6 at 550 mm, an inducer isopropyl-$\beta$-D-thiogalactopyranoside is added to the medium as 1 mM inducer, and culturing is carried out for an additional several hours to accumulate the hybrid polypeptide in the cultured cells.

To recover and purify the target hybrid polypeptide, any conventional procedure used for recovery and purification of a polypeptide accumulated in cells may be used. For example, cultured cells are collected, and the collected cells are disrupted by any conventional means, and the disrupted cells are centrifuged to collect a precipitate. The target polypeptide is recovered in the precipitate. Next, the precipitate is treated with guanidine hydrochloride to dissolve the target polypeptide. The target polypeptide is then purified by an appropriate combination of conventional procedures for the purification of protein, such as liquid chromatography, ion exchange chromatography, affinity chromatography and the like. An embodiment of recovery and purification of the hybrid polypeptide of the present invention is described in Example 7.

E. Property of hybrid polypeptide

(1) Analysis by electrophoresis

Samples of the expression products prepared as above-mentioned are subjected to an SDS-polyacrylamide gel electrophoresis, and the gel is stained with Coomassie Blue. An example of the result is shown in Fig. 17A. In Fig. 17A, lanes 1, 2, and 3 represent the results of expression products obtained from E. coli JM103/pPE3, E. coli JM103/pHA03, and E. coli JM101/pDPAT2, respectively.

(2) Immunological confirmation

Next, the reactivity of the products separated by the above-mentioned electrophoresis with an anti-tissue plasminogen activator antiserum or with an anti-urokinase antiserum was tested on a nitrocellulose sheet, and the reaction patterns shown in Fig. 17 B and C were obtained. As seen from the figures, it was confirmed that the hybrid polypeptide HA03 of the present invention is reactive with both the above-mentioned antisera.

For other hybrid polypeptides, similar results were obtained.

(3) Affinity to fibrin

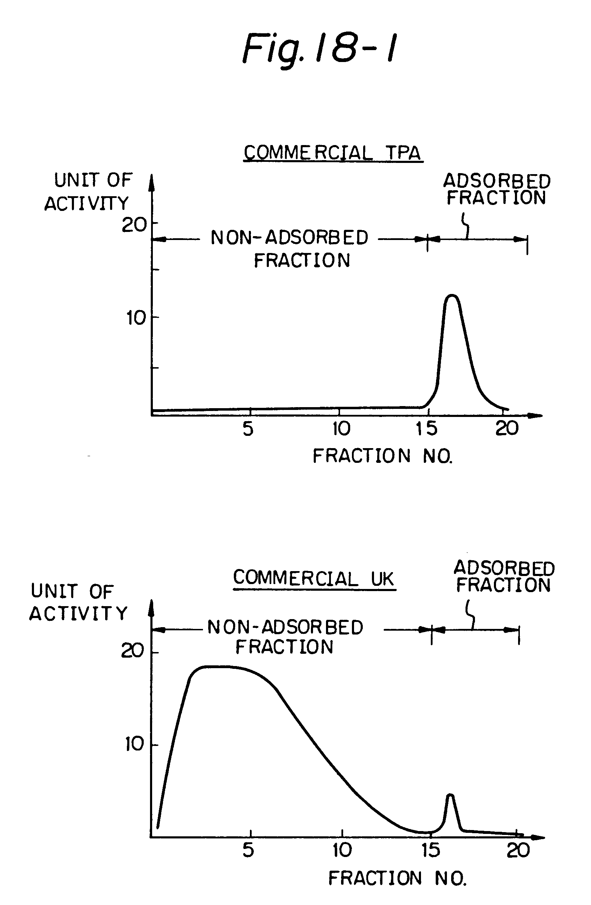

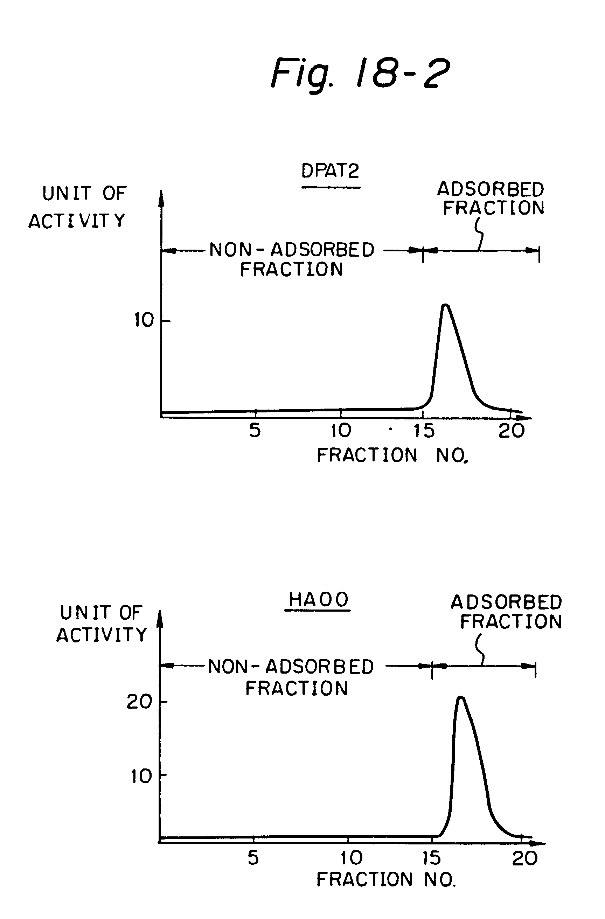

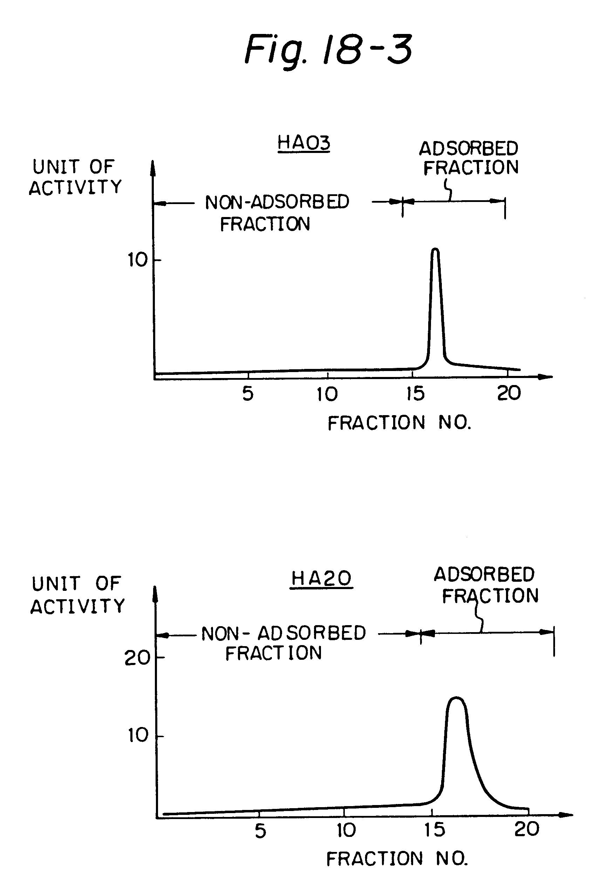

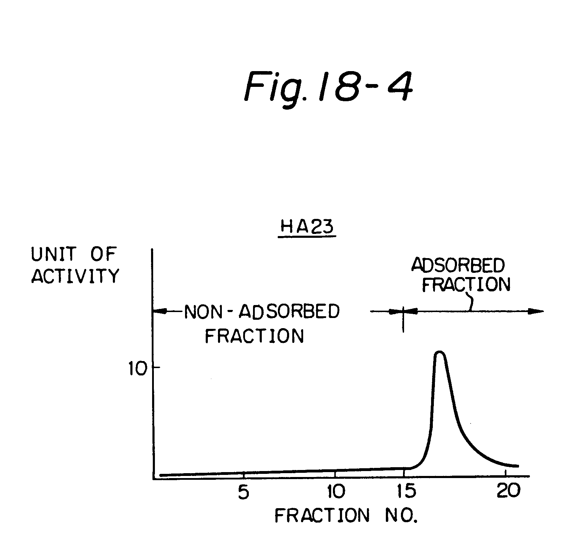

Fibrin affinity columns were prepared, and the sample of the expression product applied to the column, and elution was carried out first with Tris-HC1 buffer (A) and then with Tris-HC1 buffer containing 2M KSCN(B). The elute was fractionated, and fractions eluted with (A) designated as non-adsorbed fractions; and fractions eluted with (B) designated as adsorbed fractions. Each fraction thus obtained was tested for plasminogen activating action on a fibrin plate containing plasminogen. The results are shown in Figs. 18-1 to 18-4. As seen from these figures, while a commercially available urokinase (UK) was eluted in non-adsorbed fractions, hybrid polypeptides of the present invention and a commercially available tissue plasminogen activator (TPA) were eluted in adsorbed fractions. Therefore, it is evident that the hybrid polypeptide of the present invention have the same level of fibrin affinity as that of native tissue plasminogen activator.

(4) Measurement of Km value on synthetic substrate S-2288

To confirm enzyme activity of the present hybrid polypeptide, Km values of an expression product HA03 from plasmid pHA03 and an expression product HA20 from plasmid pHA20 after activation with plasmin, which are representatives of hybrid polypeptides containing as its fibrin affinity region about two kringles or about a half kringle of polypeptide, and as its enzyme activity region, an enzyme activity region of native human prourokinase or a corresponding region wherein 157th phenylalanine is replaced by aspartic acid, were compared with the Km value of a commercially available urokinase. The Km values of three polypeptides were the same, $2.0 \times 10^{-4}$ mol/l. Therefore, it was confirmed that the hybrid polypeptides of the present invention, after activation, exhibit the same level of enzyme activity as that of native urokinase.

(5) Stability against plasmin and thrombin

Among the hybrid polypeptides of the present invention, those wherein 157th phenylalanine is replaced by aspartic acid, i.e., hybrid polypeptide HA03, HA13, and HA23, are difficult to inactivate by thrombin, and are activated by plasmin at a lower speed.

As seen from the above description, the hybrid plasminogen activator-like polypeptides of the present invention exhibit a fibrin affinity at the same level as a tissue plasminogen activator, and when activated, exhibit an enzyme activity at the same level as urokinase. Moreover, the hybrid plasminogen activator-like polypeptides wherein 157th phenylalanine is replaced by an acidic amino acid are stable against plasmin, thrombin and other proteases, and therefore, when administered in vivo, are expected to exhibit a sustained fibrinolytic activity.

At present, plasminogen activators having such properties have not been known, and the present plasminogen activator-like polypeptides are a new type.

Examples

The present invention will now be further illustrated by, but is by no means limited to, the following Reference examples and Examples.

Reference Example 1. Isolation of poly A⁺ RNA

Cells of human pharyngeal cancer cell line Detroit 562 were grown in a modified Eagle's medium supplemented with 10% fetal calf serum, 0.1 mM nonessential amino acids, 1 mM sodium pyruvate, and 0.1% lactalbumin on a plastic plate under the presence of carbon dioxide at a concentration of 5%. When a confluent state was reached, the medium was removed, and 2 ml each of a denatured solution (6 M guanidinium thiocyanate, 5 mM sodium citrate, 0.5% sarcosine, and 0.1 M 2-mercaptoethanol) was added to the plastic plate 9 cm in diameter to denature a cellular material to obtain an extract. According to a method of Chirgwin et al (literature 2), the extract thus obtained was overlaid on a 5.7 M CsCl solution, and the whole was centrifuged to isolate RNA. Typically, 12 mg of RNA was recovered from 60 plastic plates.

Next, poly A⁺ RNA was isolated by oligo dT cellulose chromatography (literature 3). Finally, about 600 μg of an RNA preparation was obtained.

Reference Example 2. Preparation of cDNA library

According to a method of Okayama and Berg (literature 4), cDNA was synthesized. That is, 2 μg of a vector primer and 3 μg of the above-mentioned poly A⁺ RNA were mixed, and cDNA extention reaction was carried out at 37°C for 40 minutes using 12 units of reverse transcriptase. Subsequently, according to the literature, the addition of oligo dC, digestion with Hind III, annealing and cyclization with a linker DNA, and replacement of RNA chain were carried out. The final reaction mixture thus prepared was stored as a cDNA library at -20°C. This preparation can be thawed immediately before use for transformation of E. coli. Typically, about 100,000 transformants were obtained using E. coli ₓ1776 as competent cells, from the above-mentioned reaction mixture.

Reference Example 3. Screening

To isolate clones which contain plasmid carrying a gene coding for a plasminogen activator-like protein from the above-mentioned transformants, synthetic DNA fragments were used as a probe. The DNA fragments have the following nucleotide sequences:

**Probe I**

5'                        3'
**AATCGGGCATGGATTTCCTG**   , and

**Probe II**

5'                        3'
**GCCCCCGCACAGGAACCG.**

These nucleotide sequences are complementary to partial sequences of cDNA coding for a melanoma plasminogen activator reported by Pennica (literature 6) (Probe I: nucleotide number 154-173; Probe II: nucleotide number 1099-1116 in the literature).

These fragments were labeled with ³²P-γ-ATP at their 5′-end. The labeled probes were hybridized with the transformant E. coli grown on a nitrocellulose filter and denatured with an alkali, on the filter in a hybridization buffer of 900 mM NaCl, 90 mM sodium citrate, 10 × Denhart's solution (literature 7), at 45°C, for 20 hours. Next, the nitrocellulose filter was washed in a solution of 300 mM NaCl and 30 mM sodium citrate, and the filter was placed in contact with an X-ray film to obtain an autoradiogram, and hybridization-positive clones were detected. By using a mixture of the probes I and II, from about 50,000 clones more than ten hybridization-positive clones were isolated. Among these hybridization positive clones, one clone E. coli ₓ1776 (pDPA3) was hybridized with both probes. From this clone, plasmid pDPA3 was obtained according to a conventional procedure.

E. coli ₓ1776 (pDPA3) containing the plasmid pDPA3 was deposited with F.R.I. on November 8, 1984 as FERM P-7931.

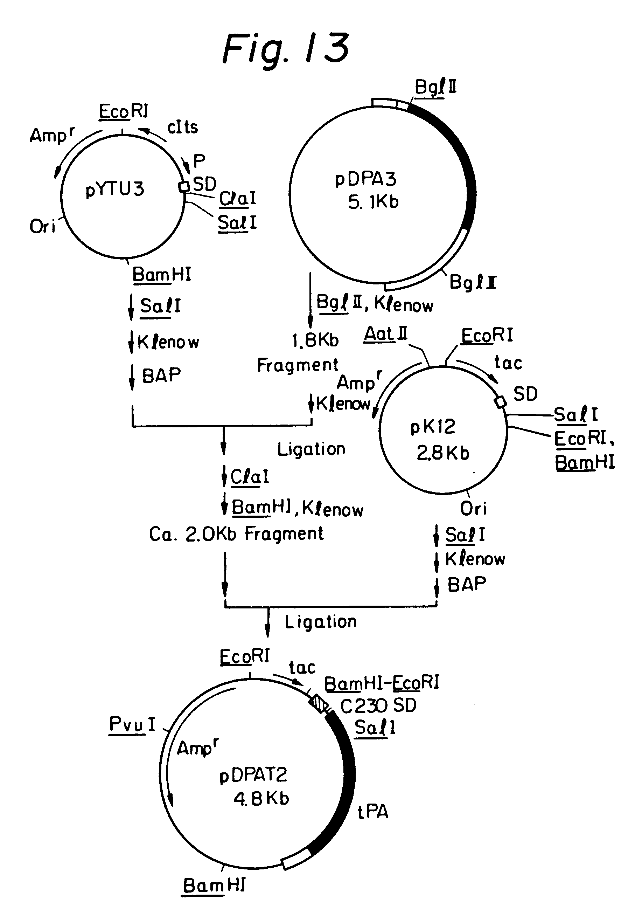

A restriction endonuclease map of the plasmid pDPA3 is shown in Fig. 13. In this figure, a thick circle part corresponds to the cDNA insert. This cDNA insert was sequenced by a method of Maxam and Gilbert (literature 8), and the results thereof are shown in Figs. 1-1 to 1-5. The cDNA sequence consists of 2459 base pairs excluding a poly A sequence present at 3′-terminal. Among them, 1548 bp encode 516 amino acids. This coding region includes, in addition to a sequence coding for a mature plasminogen activator (nucleotide number 261-1703), an upstream preprosequence (nucleotide number 156-260). There is a $5^{'}$-non-coding region (nucleotide number 1-155) upstream of the coding region, and a $3^{'}$-non-coding region (nucleotide number 1704-2459) downstream of the coding region.

Reference Example 4. Preparation of mRNA

mRNA was prepared by the method of J.M. Chirgwin et al (literature 2) using guanidine thiocyanate.

Twenty g of kidney tissue cells frozen at -80°C was crushed with a Waring blender in liquid nitrogen and suspended in 80 ml of a 5 M guanidine thiocyanate solution (5 M guanidine thiocyanate, 0.5% sodium N-lauroylsarcosine, 25 mM sodium tartrate, 0.1 M mercaptoethanol, and 0.1% antifoam A). The suspension was homogenized with a Teflon homogenizer and nucleic acid was shared with a 20G 1/2 injection needle. Twenty-four ml of said solution was layered over 12 ml of 5.7 M CsCl and after centrifugation with a Beckman centrifuge in SW28 rotor for 24 hours at 15°C and at 25,000 rpm the whole crude RNA was recovered.

The total crude RNA was dissolved in 2% potassium acetate solution and twice its volume of ethanol was added. The mixture was allowed to stand overnight at -20°C and centrifuged to recover precipitate.

In accordance with the method of H. Aviv et al (literature 3), poly(A)$^+$ RNA was isolated and purified by oligo (dT) cellulose column chromatography. Ten g of the kidney tissue cells yielded about 3 mg of the total RNA, of which 2 to 3% was poly(A)$^+$ RNA.

Reference Example 5. Construction of cDNA Library (I)

cDNA synthesis was carried out using 40 $\mu$g of the poly(A)$^+$ RNA obtained in Reference Example 4. Using 40 $\mu$g of oligo (dT)$_{12-18}$ as a primer, reaction was carried out with 40 units of reverse transcriptase for 2 hours at 42°C to synthesize the first chain and after removal of the template mRNA by alkali treatment the synthesis of the second chain was carried out with 100 units of E. col[-1zi DNA polymerase I Klenow fragment.

After elimination of the hairpin loop with S1 nuclease, the (dC)$_{10-20}$ chain was bonded to the $3^{'}$ end of the double-stranded cDNA with terminal deoxynucleotidyl transferase, and about 400 ng of (dC) tailed cDNA was obtained.

This material was annealed together with 800 ng of a commercially available (dG) tailed pBR322 (PstI site) (New England Nuclear Inc.), and E. coli $_x$1776 was transformed by the Hanahan method (literature 9). A cDNA library (I) consisting of about $2 \times 10^5$ tetracycline-resistant and ampicillin-sensitive transformants was thus obtained.

The size of cDNA insert fragments was determined by the rapid isolation method (literature 10) using the alkali lytic procedure on these transformants.

Reference Example 6. Preparation of Synthetic DNA Oligomers for Use as cDNA Library Screening Probes

The following 16 different DNA oligomers consisting of 14 nucleotides which are complementary to the mRNA corresponding to the amino acid sequence of the human urokinase: Asn[169], Gln, Pro, Trp, Phe[173] which has been reported by G.J. Steffens et al (literature 11) and Gunzler et al (literature 12) were synthesized by the phosphotriester method:

**AACCAAGGTTGATT**

**AACCAAGGTTGGTT**

**AACCAGGGTTGATT**

**AACCACGGTTGATT**

**AACCACGGTTGGTT**

**AACCATGGTTGATT**

**AACCATGGTTGGTT**

**AACCAAGGCTGATT**

**AACCAAGGCTGGTT**

**AACCAGGGCTGATT**

**AACCAGGGCTGGTT**

**AACCACGGCTGATT**

**AACCACGGCTGGTT**

**AACCATGGCTGATT**

**AACCATGGCTGGTT**

**AACCAGGGTTGGTT**

These 16 different DNA oligomers are referred to hereinafter as UK probe I.

For use as confirmatory probes, the following 8 different DNA oligomers consisting of 14 nucleotides which are complementary to the mRNA corresponding to Met[238], Try, Asn, Asp, Pro[287] were synthesized in the same manner:

**5' GGATCATTATACAT 3'**

**GGATCGTTATACAT**

**GGATCGTTGTACAT**

**GGATCATTGTACAT**

**GGGTCATTATACAT**

**GGGTCGTTATACAT**

**GGGTCGTTGTACAT**

**GGGTCATTGTACAT.**

These 8 different DNA oligomers are referred to hereinafter as UK probe II.

By use of T4 polynucleotidekinase, 200 ng each of the UK probes I and II was radioactively labeled at the 5′ end with 3000 Ci/mmole $^{32}$P-$\gamma$-ATP to prepare probes for colony hybridization.

Reference Example 7. Screening of cDNA Library (I)

(1) Screening

An autoclaved nitrocellulose filter (0.45 $\mu$m, Type-TM-2 made by Toyo Roshi Co.) was placed on an LB agar medium containing 15 $\mu$g/ml of tetracycline and the medium was inoculated with the transformants prepared in Reference Example 5 so as to allow about 2,000 transformant colonies to grow. After 8 hours of incubation at 37°C the colonies were replicated on two nitrocellulose filters, which were incubated further for 3 hours at 37°C. The original nitrocellulose filter was used as a master filter, while two second filters were transferred to LB agar media containing 15 $\mu$g/ml of tetracycline and 100 $\mu$g/ml of chloramphenicol and were subjected to overnight incubation at 37°C. In accordance with the modified method of Grunstein and Hogness (literature 13) the filters were then placed over 0.5 M NaOH and 1.5 M NaCl for 3 minutes to effect colony lysis and DNA denaturation, and after neutralization over 0.5 M Tris-HCl (pH 7.6) and 1.5 M NaCl, were air-dried and baked for 2 hours at 80°C.

After washing the filters in 4 $\times$ SSC for 30 minutes each at 60°C, prehybridization was carried out in 4 $\times$ SSC, 10 $\times$ Denhardt and 50 $\mu$g/ml denatured E. coli-DNA for one hour at 60°C, and after addition of 0.1 mM ATP and the radioactively labeled UK probe I (about 10$^7$ cpm/filter), hybridization was carried out for 16 hours at 37°C. After washing 6 to 8 times in 4 $\times$ SSC at 39°C, the filters were air-dried and screened for

12

transformants hybridizing with the UK probe I to obtain 21 candidate clones from $8 \times 10^4$ colonies. Plasmids of these clones are hereinafter referred to as plasmids pKYU1 to PKYU21.

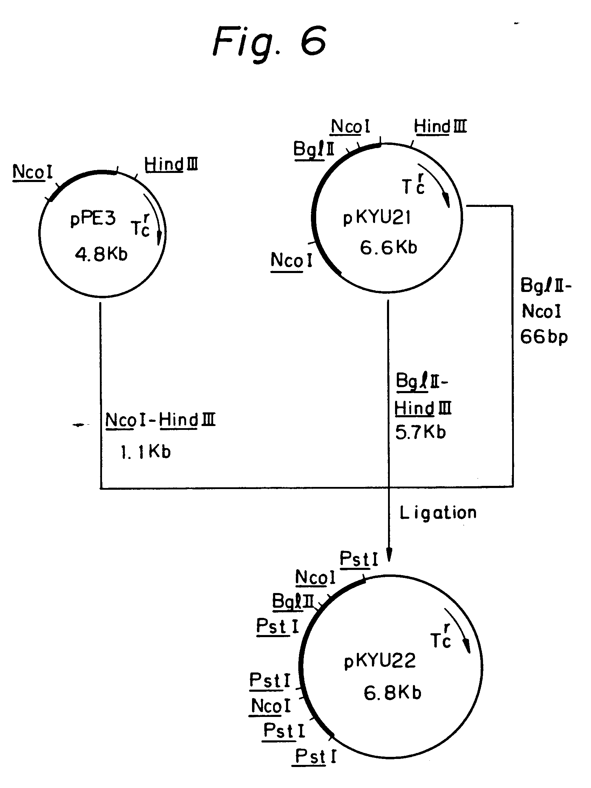

The 21 candidate clones obtained were treated in the same manner as described above and clones hybridizing with the UK probe II were obtained. The plasmids in these clones are hereinafter referred to as plasmid pKYU21 (Fig. 6).

(2) Characteristics of pKYU21 Plasmid DNA

After digesting the pKYU21 plasmid DNA with various restriction endonucleases and subcloning it in M13mp8 by the Maxam-Gilbert method (literature 8), determination of the nucleotide sequence was carried out by the dideoxy chain termination method (literature 14). On contrasting this sequence with the well-known amino acid sequence of urokinase it was confirmed that although the cDNA contains the entire code region of low molecular weight urokinase, about 100 bp long DNA in the $5^{'}$-end region of the coding region of high molecular weight urokinase is lacking.

Reference Example 8. Construction of cDNA Library (II) by Primer Extension Reaction

Based on the nucleotide sequence determined in Paragraph (2) of Reference Example 7, the following DNA oligomer:

## 5' CTGAAGAGCATCAGA 3'

consisting of 15 nucleotides which are complementary to the mRNA sequence corresponding to $^{8}$ $^{9}$Ser, Asp, Ala, Leu, Glu$^{93}$ was synthesized by the phosphotriester method.

Employing 100 $\mu$g of poly(A)$^{+}$ RNA as a template and complying with the method of Agarwall et al (literature 15) or the method of Steward et al (literature 16), the first cDNA chain was synthesized using 100 units of reverse transcriptase together with 1 $\mu$g of $5^{'}$ $^{32}$P-labeled primer followed by the synthesis of the second chain with 100 units of E. coli DNA polymerase I Klenow fragment. Then the single-stranded DNA was digested with S1 nuclease and (dC)n chain was added at the $3^{'}$-end using terminal deoxynucleotidyl transferase. After annealing this dC tailed insert (cDNA) and a dG tailed vector (pBR322) together, E. coli $_{x}1776$ was transformed to obtain a cDNA library (II) consisting of about $5 \times 10^4$ transformants.

Reference Example 9. Screening of cDNA Library (II)

The transformants obtained in Reference Example 8 were subjected to hybridization in the same manner as that described in Paragraph (1) of Reference Example 7. In this case, a 150 bp PstI-BglII $5^{'}$ digestion fragment from pKYU21 radioactively labeled by the nick translation method (literature 9) using $^{32}$P-$\alpha$-dCTP (3000 Ci/mole) was used as a probe. The hybridization was carried out at 60°C.

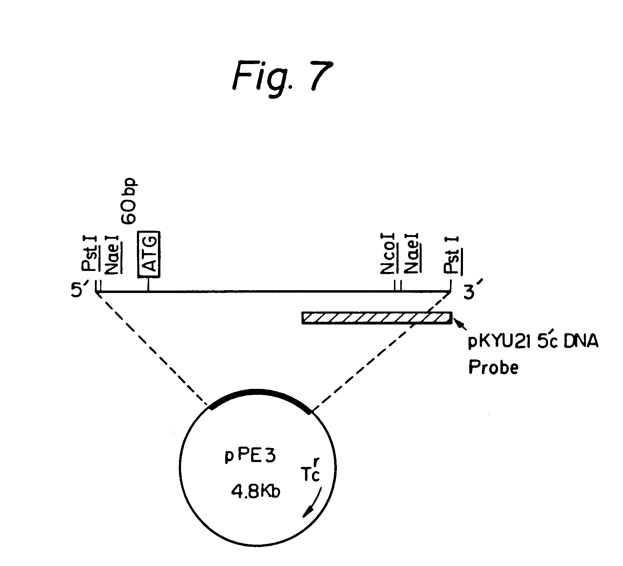

The filter was air-dried after washing two or three times with $2 \times$ SSC at 60°C, and clones hybridizing with said probe were retrieved by autoradiography. Eight positive clones were obtained out of about $3 \times 10^4$ clones. The plasmids in these clones are hereinafter referred to as plasmids pPE1 to pPE8. On digestion of these plasmid DNAs with restriction enzyme PstI it was confirmed that Plasmid pPE3 (Fig. 7) contains an approximately 420 bp long cDNA insert.

The fragments obtained through digestion of DNA of plasmid pPE3 with restriction endonuclease PstI were subcloned into M13pm8, and determination of the base sequence was carried out by the dideoxy chain termination method (literature 14). As the result, it was confirmed that the cDNA contains not only a sufficiently long coding region on the $5^{'}$-end side of the prourokinase gene but also a 66 bp long $5^{'}$-nontranslation region upstream of the translation initiation codon ATG (Fig. 2-1 to 2-5).

Reference Example 10. Construction of Prourokinase Gene (Fig. 6)

Five $\mu$g of DNA of plasmid pKYU21 containing an entire coding region of the low molecular weight urokinase was digested with 10 units each of restriction endonucleases BglII and HindIII and then electrically eluted to give about 5.7 Kbp DNA fragment, while DNA of said plasmid pKYU21 was digested with 10 units each of BglII and NcoI and then eluted to give a 66 bp DNA fragment. Then again 5 $\mu$g of DNA of plasmid pPEP3 obtained in Reference Example 6 was digested with 10 units each of NcoI and HindIII to give about 1.1 Kbp DNA fragment. These three different DNA fragments were repeatedly

EP 0 231 883 B1

extracted with phenol/chloroform, precipitated with 2 volumes of ethanol so as to purify and recover the precipitate. These three different DNA fragments were ligated together with T4 DNA ligase for transformation into E. coli $_x$1776. The resultant transformants were screened by the rapid isolation method using the alkali lysis procedure and a clone carrying plasmid pKYU22 that contains the entire gene for prourokinase was obtained.

The clone, E. coli $_x$1776/pKYU22 was deposited on January 11, 1985 with F.R.I. as FERM P-8041 and on January 22, 1986 was placed under international deposition as FERM BP-968 pursuant to the provisions of the Budapest Treaty.

Reference Example 11. Nucleodide Sequence Determination of Plasmid pKYU 22 Insert Site

The nucleotide sequence at the insert site of plasmid pKYU22 was determined by the Maxam-Gilbert method, and the dideoxy chain termination method preceded by subcloning to M13mp8. The result is presented in Figures 2-1 to 2-5.

As seen from these figures, the insert consists of a 66 bp 5′-non-coding region, a leader sequence ATG(Met)-GGC(Gly), a prourokinase coding region AGC(Ser)-CTC(Lev), a translation stop codon TGA(XXX), and a 3′-non-translation codon downstream of the stop codon.

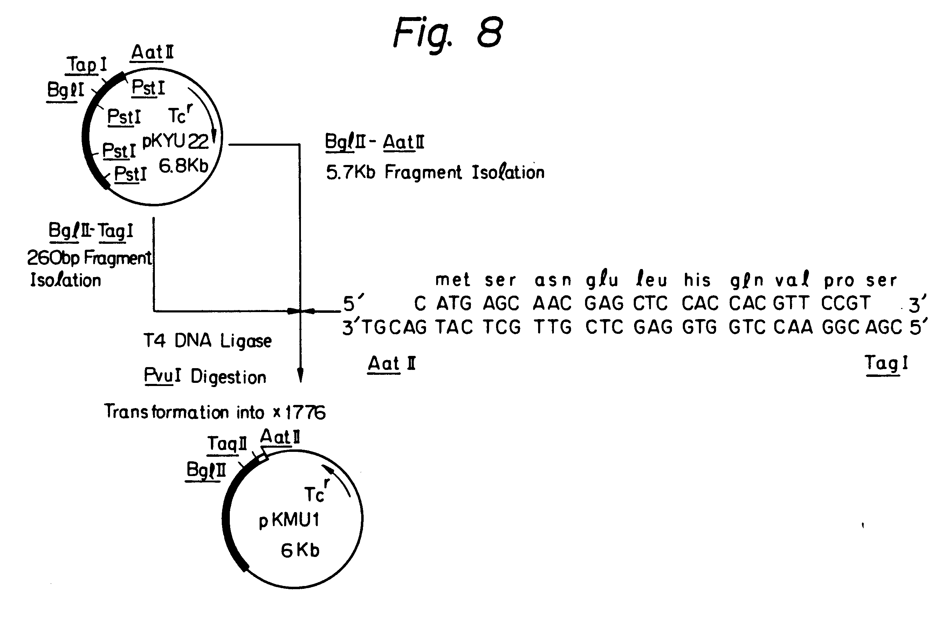

Reference Example 12. Synthesis of Prourokinase Gene Modified at 5′ end Region (Fig. 8)

Codons of a 5′-terminal region of native cDNA coding for prourokinase were replaced so as to permit the prourokinase gene to be efficiently expressed in E. coli under the SD sequence of the Pseudomonas putida-derived C230 gene; a translation start codon ATG(Met) was provided adjacent to and upstream of a codon for the first amino acid (Ser) so that prourokinase could be directly expressed; and a restriction enzyme recognition site AatII was provided to join the coding region near to an SD sequence of the expression vector. For this purpose, a double stranded DNA oligomer shown in Fig. 8 was synthesized by a phosphotriester method. This double stranded synthetic DNA has an AatII site at one end to insert it into a vector, and a TaqI site at another end to join it to the prourokinase gene.

The following three single-chain DNA oligomers comprising 29, 15, and 20 nucleotides respectively were synthesized by the phosphotriester method:

**5' CATGAGCAACGAGCTCCACCAGGTTCCGT 3'**

**3' TGCAGTACTCGTTGC 5'**

**3' TCGAGGTGGTCCAAGGCAGC 5'**

Next, 1 μg each of the synthetic DNA oligomers was heated for 2 minutes at 95°C, phosphorylated at the 5′ end with T4 polynucleotide kinase and purified using a Sep Pak (C18) column (Waters). After drying, the purified material was dissolved in 50 μl of 20 mM Tris-HCl (pH 7.6) and 10 mM MgCl$_2$, and annealed by heating for 2 minutes at 95°C, cooling slowly to room temperature, and then maintaining the solution overnight at 12°C to give the following double-stranded DNA:

**5'       CATGAGCAACGAGCTCCACCAGGTTCCGT 3'**

**3' TGCAGTACTCGTTGCTCGAGGTGGTCCAAGGCAGC**

**AatII          SstI    BstNI    TaqI**

On the one hand, 5 μg of DNA of plasmid pKYU22 was digested with restriction endonucleases BglII and AatII, and about 5.7 Kb DNA fragment was recovered by electric elution. On the other hand, 5 μg of DNA of the same plasmid pKYU22 was digested with restriction endonucleases PstI and BglII and about 400 bp DNA fragment was obtained by electric elution. This fragment was again digested with restriction endonucleases TaqI and about 260 bp DNA fragment was recovered by electric elution. These two different DNA fragments were recovered and purified by phenol/chloroform extraction, and precipitation with 2 volumes of ethanol.

These two different DNA fragments and the aforesaid double-stranded synthetic DNA oligomer were ligated together using T4 DNA ligase and the ligation product was used to transform E. coli $_x$1776. Then the

14

transformants were screened by the rapid isolation method by the alkali lysis procedure, and a clone Escherichia coli ₓ1776/pKMU1 carrying plasmid pKMU1 that contains a modified prourokinase gene was obtained. The clone E. coli ₓ1776/pKMU1 has been deposited with F.R.I. on January 11, 1985 as FERM P-8040.

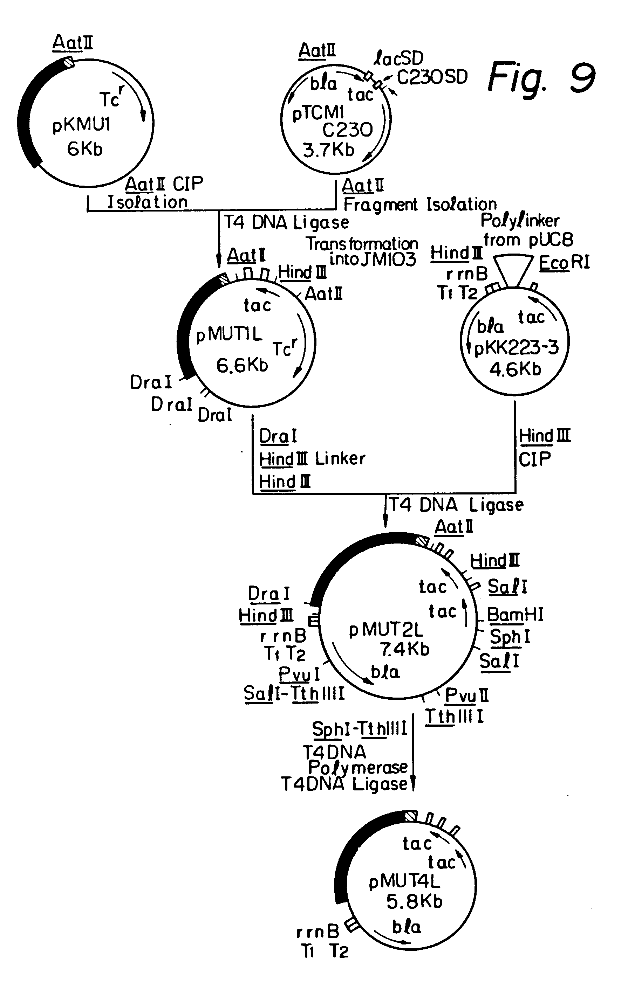

Reference Example 13. Construction of Plasmid pMUT4L (Fig. 9)

Expression plasmid pMUT4L containing a human prourokinase gene without a point mutation was constructed from the above-mentioned plasmid pKMU1, and an expression vector pTCM1. E. coli JM103/pTCM1 containing the plasmid pTCM1 was deposited with F.R.I. on August 17, 1984 as FERM P-7779.

Five μg of plasmid pKMU1 from Reference Example 12 was digested with 10 units of restriction endonuclease AatII and the digest was isolated after treatment with calf intestinal phosphatase (CIP). On the other hand, 5 μg of plasmid pTCM1 was digested with 10 units of restriction endonuclease AatII and about 500 bp DNA fragment was isolated by the electric elution. These two different DNA fragments were purified by repeated phenol/chloroform extraction and ethanol precipitation.

Both of these DNA fragments were joined together using T4 DNA ligase and were transformed into E. coli JM103. The transformants were screened by the rapid isolation method by the alkali lysis procedure, and a clone carrying plasmid pMUT1L in which tac promoter/operator and C230SD sequence having the normal orientation with reference to the prourokinase gene was obtained.

Next, 5 μg of plasmid pKK223-3 (literature 17, 18 and 19) was digested with 10 units of restriction endonuclease HindIII, and the digest was treated with calf intestinal phosphatase (P.L. Biochemicals).

On the other hand, 1 μg of the plasmid pMUT1L obtained as above was digested with 4 units of restriction endonuclease DraI and the digestion fragment and 1 μg of 5′-phosphorylated HindIII linker (dCAAGCTTG) were ligated with T4 DNA ligase. Digestion was carried out using 12 units of restriction endonuclease HindIII, and the digest was dissolved in 0.15 M NaCl. The solution was extracted with an equal volume of phenol/chloroform and the DNA was precipitated by the addition of 2 volumes of ethanol. The precipitate was collected at 16,000 rpm and at 4°C and was dried.

The resultant pMUT1L digestion fragment and the HindIII digestion fragment of aforesaid pKK223-3 were ligated using T4 DNA ligase and were transformed into E. coli JM103. The transformants were screened by the alkali lysis procedure and a clone, E. coli JM103/pMUT2L containing plasmid pMUT2L was obtained.

Five μg of plasmid pMUT2L was digested with 10 units each of restriction endonucleases SphI and Tth111I, and after extraction with phenol/chloroform DNA was precipitated with ethanol. The DNA thus recovered was blunt-ended using T4 polymerase in the presence of 0.1 mM dGTP, dCTP, dATP and TTP, and was recyclized by T4 DNA ligase. The DNA was transformed into E. coli JM103 and colonies were allowed to form on an LB agar medium containing 50 μg/ml of ampicillin. The transformants were screened by the alkali lysis procedure (literature 10) and a clone, E. coli JM103/pMUT4L was obtained.

Reference Example 14. Introduction of Specific Base Substitution Mutation into Codon for Amino Acid 157 Using M13 Phage

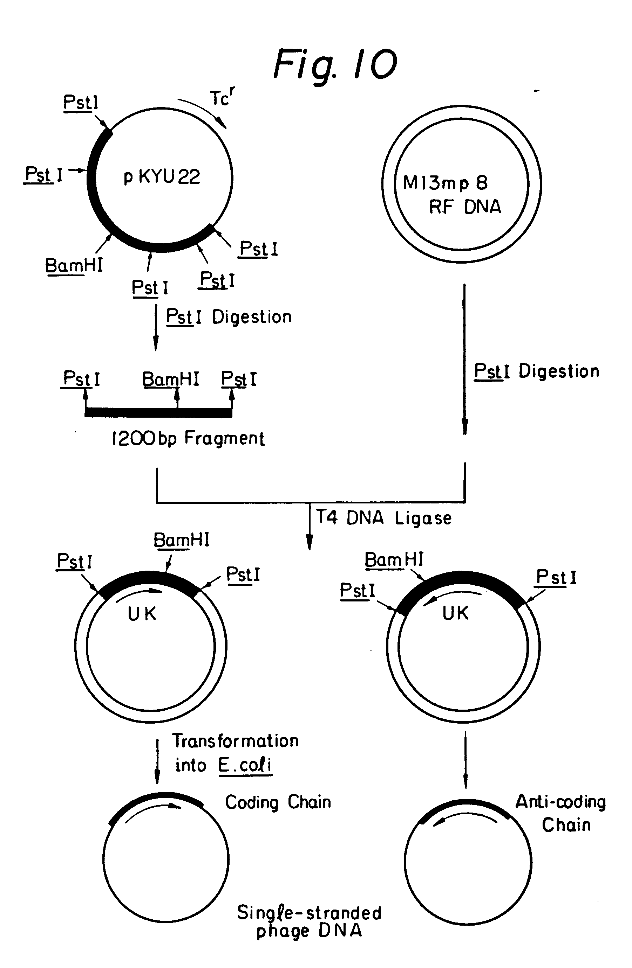

(1) Preparation of Single-strand Template DNA (Fig. 10)

One μg each of plasmid pKYU22 and phage M13mp8 double-stranded DNA was digested in 20 μl of a solution containing 10 mM Tris-HCl (pH 7.5), 7 mM MgCl₂ , 7 mM β-melcaptoethanol, and 50 mM NaCl for one hour at 37°C using 5 units of PstI. The respective DNA fragments were recovered after treatment with phenol and precipitation with ethanol. A mixture of these two different DNA fragments was subjected to ligation reaction in 20 μl of a solution containing 66 mM Tris-RCl (pH 7.5), 5 mM MgCl₂ , 5 mM DTT, and 1 mM ATP for 16 hours at 12°C using T4 DNA ligase. Transformation into E. coli JM103 was carried out using the reaction mixture in accordance with the method of Messing et al (literature 19) and the transformants were plated with soft agar containing 0.02% X-gal and 1 mM IPTG. The plates were incubated overnight at 37°C. Single-stranded DNA was prepared from white plaques formed by the recombinant.

Using some of the resultant single-strand DNA as a template the base sequence was determined using the dideoxy procedure in accordance with the method of Messing et al (literature 20) and sequence of the cloned single-strand DNA was confirmed. The coding chain and anticoding chain were obtained as shown in Fig. 10.

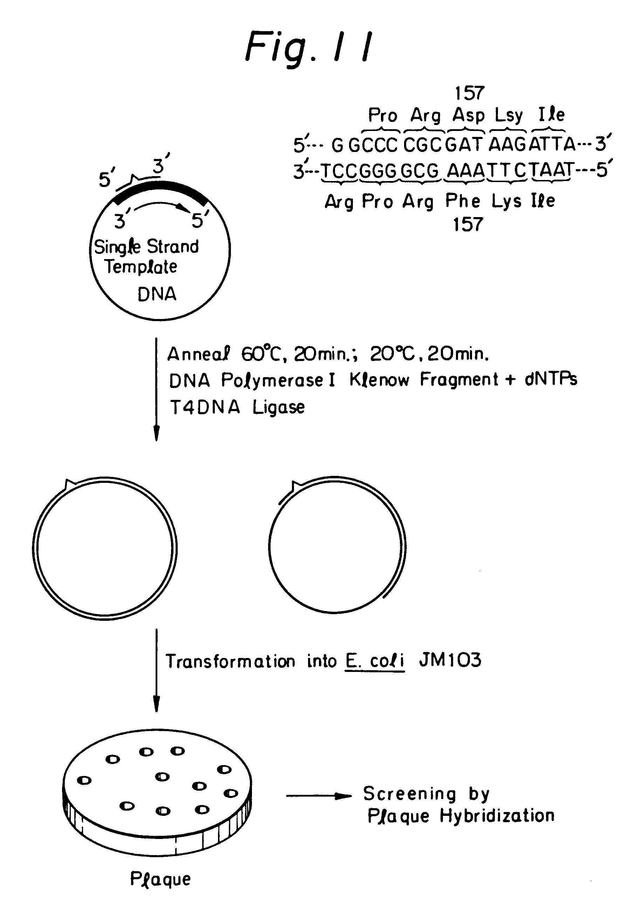

(2) Introduction of Specific Base Substitution Mutation (Fig. 11)

Using the resulting recombinant M13 phage single-strand DNA (an anticoding chain of the urokinase gene has been cloned) as a template, introduction of site-directed mutagenesis was carried with a another oligonucleotide mutagen. In this case, the following synthetic oligonucleotide:

**5' GGCCCCGCG<u>GAT</u>AAGATTA 3'**

was used as a primer. Although this 18-base oligonucleotide is complementary to the urokinase gene in the single-strand template DNA, two bases have undergone changes wherein codon TTT which specifies phenylalanine has changed to codon GAT specifying aspartic acid.

Using the aforesaid oligonucleotide as a primer, double-strand DNA was synthesized in vitro. That is, 2 pmole of 5′-phosphorylated primer was added to 0.5 pmole of the template single-strand DNA and the mixture was incubated in 10 $\mu$l of a solution containing 7 mM Tris-HCl (pH 7.5), 0.1 mM EDTA, 20 mM NaCl, and 7 mM MgCl$_2$ for 20 minutes at 60°C followed by further incubation for 20 minutes at 23°C. Next, to the reaction mixture was added 0.5 mM each of dATP, dGTP, dTTP and dCTP to a combined volume of 20 $\mu$l to which was added 2 units of DNA polymerase Klenow fragment. The mixture was incubated for 20 minutes at 23°C, and after the addition of 1 $\mu$l of 10 mM ATP and 1 unit of T4 DNA ligase the mixture was incubated overnight at 12°C. In accordance with the method of Messing et al (literature 20) the aforesaid reaction mixture was directly transformed into E. coli JM103. About 10,000 phage plaques per microliter of said reaction mixture were thus obtained.

After the transfer of the resulting plaques from a soft agar medium to a nitrocellulose filter in accordance with the method of Benton and Davis et al (literature 21), the filter was baked in vacuo for 2 hours at 80°C. The nitrocellulose filter was hybridized with the primer oligonucleotide labeled with $^{32}$P as a probe in a mixture of 6 × SSC and 10 × Denhardt overnight at 37°C. The filter was then washed in 6 × SSC at 52°C and mutant phage plaques giving positive signals were autoradiographically isolated. From the mutant phage, mutant phage DNA of the double-strand type (pm3) was obtained. Using the mutant phage DNA as a template according to the dideoxy method, the nucleotide sequence of the mutant DNA was determined, and the occurrence of the desired single base substitution mutation was confirmed.

In addition, it is possible to replace codon TTT specifying phenylalanine at 157 position with the Glu-specifying codon GAA using the following oligonucleotide:

**5'GGCCCCGC<u>GAA</u>AAGATTA 3'**

as a mutagen.

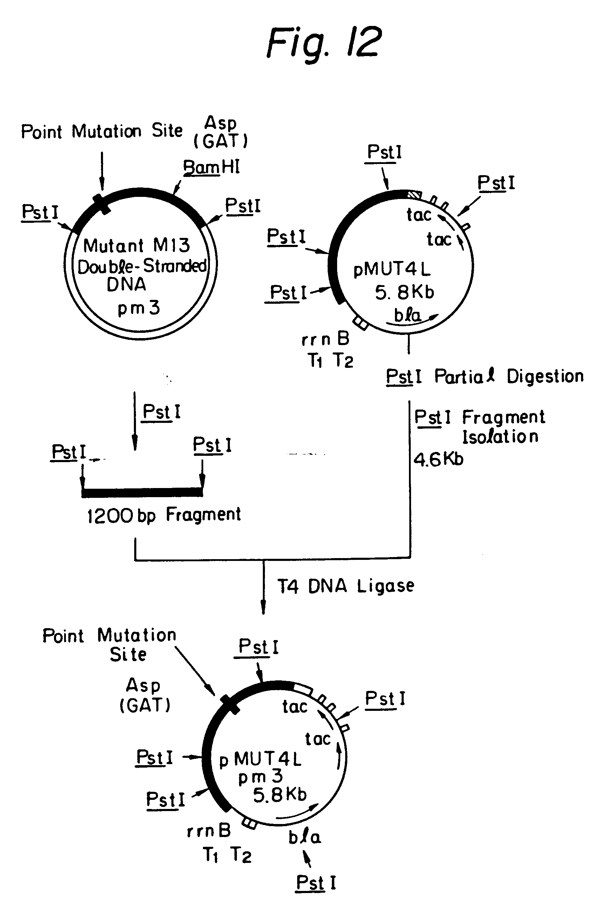

Reference Example 15. Construction of Plasmid pMUT4Lpm3 (Fig. 12)

Expression plasmid pMUT4Lpm3 wherein the human urokinase structural gene having the aforementioned point mutation had been inserted downstream of E. coli tac promoter was constructed in the following manner:

After complete digestion of 10 $\mu$g of mutant M13 double-strand DNA (pm3) with PstI, a fragment about 1.2 Kbp in length was isolated. On the other hand, 10 $\mu$g of plasmid pMUT4L was partially digested with PstI and a fragment about 4.6 Kbp in length was isolated from which a fragment about 1.2 Kbp in length had been eliminated.

Each of the above two fragments was recovered by treating with phenol and precipitating with ethanol and then the precipitates were mixed. The mixture was subjected to ligation reaction overnight at 12°C using T4 DNA ligase and the reaction mixture was used to transform E. coli HB101. The transformants were then screened by the alkali lysis procedure and a clone containing plasmid pMUT4Lpm3 was obtained. Escherichia coli $_x$1776/pMUT4Lpm3 carrying plasmid pMUT4Lpm3 was deposited on July 11, 1985 with F.R.I. as FERM P-8341 and was placed under international deposition on January 22, 1986 as FERM BP-971 pursuant to the provisions of the Budapest Treaty.

Reference Example 16. Construction of Plasmid pMUT4LpmI

Mutant double-stranded phage M13RFpml containing a DNA fragment wherein a codon AAA for 135th lysine is mutated to a codon CAA for glutamine was obtained by the same procedure as described in Reference Example 14, except that the following synthetic oligonucleotide:

$$5'\text{GATGGA}\underline{\text{CAA}}\text{AAGCCC}3'$$

was used. The DNA sequence of the mutant DNA thus obtained was determined by dideoxy method using the mutant phage DNA as a template, and was confirmed to contain a desired point mutation.

Next, from the phage M13RFpml and the plasmid pMUT4L, pMUT4Lpml was constructed according to the same procedure described in Reference Example 15.

### Reference Example 17. Construction of Plasmid pMUT4Lpm4

A mutated double-strand phage containing a DNA fragment wherein a codon AAA for 135th lysine is mutated to a codon for glutamine was obtained by the same method as described in Reference Example 14, except that the following synthetic oligonucleotide:

$$5'\text{GATGGA}\underline{\text{CAA}}\text{AAGCCC}3'$$

was used.

From the mutated phage thus obtained, a single-strand phage was obtained according to the same procedure as described in Reference Example 14. Using the single-strand phage as a template, a codon TTT for 157th phenylalanine was then mutated to a codon GAT for aspartic acid according to the same procedure as described above, except that the following synthetic oligonucleotide:

$$5'\text{GGCCCCGC}\underline{\text{GAT}}\text{AAGATTA}3'$$

was used, to obtain a mutated double-strand phage Ml3RFpm4 containing a DNA fragment wherein a codon AAA for 135th lysine is mutated to a codon CAA for glutamine and a codon for 157th phenylalamine is mutated to a codon for aspargic acid.

Next, from the double mutated phage M13RFpm4 thus obtained and the plasmid PMUT4L, plasmid pMUT4Lpm4 was constructed according to the same procedure as described in Reference Example 15.

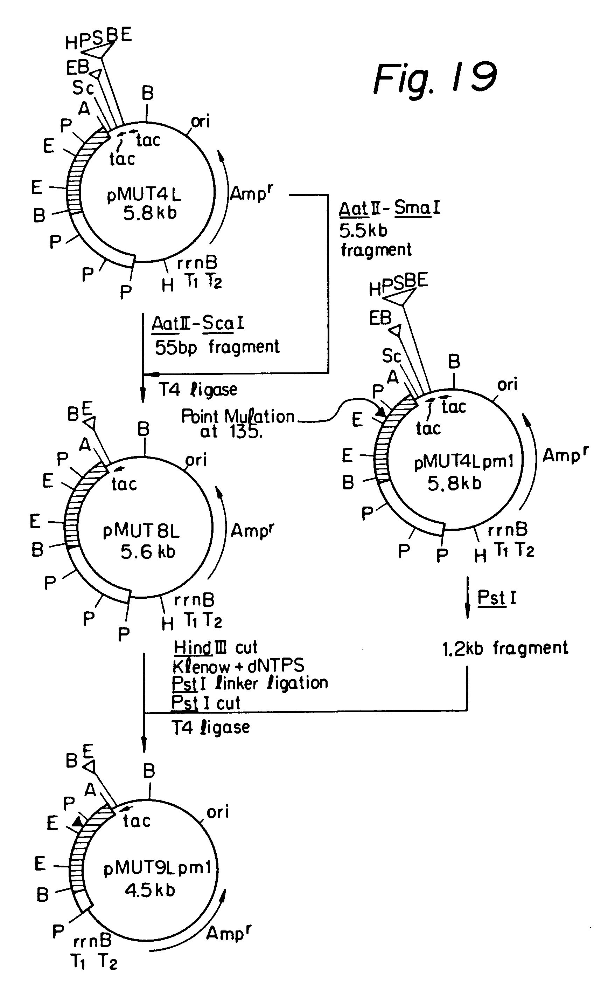

### Reference Example 18. Construction of Plasmid pMUT9Lpml (Fig. 19)

5 $\mu$g of plasmid pMUT4L constructed in Reference Example 13 was digested with 50 units of ScaI and 100 units of AatII in 100 $\mu$l of a buffer containing 10 mM Tris-HCl (pH7.5), 7 mM MgCl$_2$ , 125 mM NaCl, 7 mM $\beta$-mercaptoethanol at 37°C for 6 hours. The reaction mixture was then subjected to 0.7% agarose gel electrophoresis, and a DNA fragment of about 5500 bp was recovered by a conventional means. On the other hand, 10 $\mu$g of the same plasmid pMUT4L was digested with 50 units of AatII and 50 units of SmaI in 100 $\mu$l of a buffer containing 10 mM Tris-HCl (pH7.5), 7 mM MgCl$_2$ , 60 mMKCl, 7 mM $\beta$-mercaptoethanol at 37°C for 6 hours. The reaction mixture was then subjected to 2.0% agarose gel electrophoresis, and a DNA fragment of 55 bp was recovered by a conventional means. Two DNA fragments thus obtained were reacted with 5 units of T4 DNA ligase in 10 $\mu$l of a buffer containing 66 mM Tris-HCl (pH7.6), 6.6 mM MgCl$_2$ , 10 mM DTT and 1 mM ATP at 12°C for 15 hours. The reaciton mixture was used to transform E. coli. JM103 according to a conventional procedure, and ampicillin resistant transformants thus obtained were screemed for a colony containing plasmid pMUT8L shown in Fig. 19, and from the selected colony, the plasmid pMUT8L was isolated by a conventional means.

5 $\mu$g of the plasmid pMUT84 thus obtained was digested with 50 units of HindIII in 100 $\mu$l of a buffer containing 10 mM Tris-HCl (pH7.5), 7 mM MgCl$_2$ and 60 mM NaCl at 37°C for 2 hours. After phenol extraction and ethanol precipitation of the reaction mixture, the precipitate was treated with one unit of Klenow fragment in 20 $\mu$l of a buffer containing 50 mM Tris-HCl (pH7.2), 10 mM MgCl$_2$ , 0.1 mM DTT and 80 $\mu$M dNTPs at 22°C for 30 minutes. Afer phenol extract and ehanol precipitation of the reaction mixture, the precipitate was treated with 5 units of T4 DNA ligase in 20 $\mu$l of a buffer containing 1 $\mu$g of pSsTI linker, and 66 mM Tris-HCl (pH7.6), 6.6 mM MgCl$_2$ , 10 mM DTT and 1 mM ATP at 12°C for 2 hours. After phenol extraction and ethanol precipitation of the reaction mixture, the precipitate was digested with 50 units of PstI in 100 $\mu$l of a solution containing 20 mM Tris-HCl (pH7.5), 10 mM MgCl$_2$ and 100 mM NaCl at 37°C for 2 hours. The reaction mixture was then subjected to 0.7% agarose gel electrophoresis, and a DNA fragment of about 4300 bp was recovered by a conventional means.

On the other hand, 5 $\mu$g of the plasmid pMUT4LpmI constructed in Reference Example 16 was digested with 50 units of PstI in 100 $\mu$l of a buffer containing 20 mM Tris-HCl (pH7.5), 10 mM MgCl$_2$ and 100 mM NaCl at 37°C for 2 hours. The reaction mixture was then subjected to 0.7% agarose gel electrophoresis, and a DNA fragment of about 1200 bp was recovered by a conventional means.

Two DNA fragments thus obtained were mixed, and ligated using 5 units of T4 DNA ligase in 20 $\mu$l of a buffer containing 66 mM Tris-HCl (pH7.6), 6.6 mM MgCl$_2$ , 10 mM DTT and 10 mM ATP for 15 hours at 12°C. The reaction mixture was used to transform E. coli JM103 according to a conventional procedure, and ampicillin resistant transformants thus obtained were screened for a colony containing plasmid pMUT9LpmI, and the plasmid pMUT9LpmI was isolated according to a conventional means.

Example 1. Construction of Plasmid pDPAT2

Fifty $\mu$g of plasmid pDPA3 was digested with 75 units of BglII in 200 $\mu$l of a buffer containing 10 mM Tris-HCl (pH7.5), 7 mM MgCl$_2$ , 100 mM NaCl, and 7 mM $\beta$-mercaptoethanol at 37°C for 4 hours. After ethanol precipitation, the precipitate was reacted with 5 units of Klenow fragment in 50 $\mu$l of a buffer containing 50 mM Tris-HCl (pH7.2), 10 mM MgCl$_2$ , 0.1 mM DTT and 80 $\mu$M dNTP at 22°C for 30 minutes. The reaction mixture was subjected to 0.7% agarose gel electrophoresis, and a DNA fragment of about 1800 bp was recovered by a conventional means. This DNA fragment is designated as DNA fragment (A).

On the other hand, 10 $\mu$g of plasmid pYTU3 was digested with 20 units of SalI in 50 $\mu$l of a buffer containing 10 mM Tris-HCl (pH7.5), 7 mM MgCl$_2$ , 150 mM NaCl, 0.2 mM EDTA and 7 mM $\beta$-mercaptoethanol at 37°C for 4 hours. After ethanold1 precipitation, the precipitate was treated with 2 units of Klenow fragment in 20 $\mu$l of a buffer containing 50 mM Tris-HCl (pH7.2), 10 mM MgCl$_2$ , 0.1 mM DTT, 80 $\mu$M dNTP at 22°C for 30 minutes. After ethanol precipitation, the precipitate was treated with one unit of alkaline phosphatase (from bovine intestine) in 20 $\mu$l of a buffer containing 50 mM Tris-HCl (pH9.0), 1 mM MgCl$_2$ , 0.1 mM ZnCl$_2$ , 1 mM spermidine at 37°C for 30 minutes. Phenol treatment and ethanol precipitation were carried out. The precipitate was dissolved in 20 $\mu$l of a solution containing 10 mM Tris-HCl (pH7.5) and 1 mM EDTA. The solution contains 0.5 $\mu$g/$\mu$l of DNA. 5 $\mu$g of this DNA and 5 $\mu$g of DNA fragment (A) described above were treated with 10 units of T4 DNA ligase in 30 $\mu$l of a buffer containing 66 mM Tris-HCl (pH7.6), 6.6 mM MgCl$_2$ , 10 mM DTT and 1 mM ATP at 15°C for 15 hours. After ethanol precipitation, the precipitate was digested with 15 units of ClaI in 30 $\mu$l of a buffer containing 6 mM Tris-HCl (pH7.9), 6 mM MgCl$_2$ and 50 mM NaCl at 37°C for 4 hours. After ethanol precipitation, the precipitate was digested with 15 units of BamHI in 30 $\mu$l of a buffer containing 10 mM Tris-HCl (pH8.0), 7 mM MgCl$_2$ , 100 mM NaCl and 2 mM $\beta$-mercaptoethanol at 37°C for 4 hours. After DNA in the reaction mixture was precipitated with ethanol, the precipitate was treated with 2 units of Klenow fragment in 30 $\mu$l of a buffer containing 50 mM Tris-HCl (pH7.2), 10 mM MgCl$_2$ , 0.1 mM DTT and 80 $\mu$M dNTP at 22°C for 30 minutes. The reaction mixture was subjected to 0.7% agarose gel electrophoresis, and a DNA fragment of about 2,000 bp was recovered by a conventional means. This DNA fragment was designated as DNA fragment (B).

On the other hand, 2 $\mu$g of plasmid pK12 was digested with 10 units of SalI in 20 $\mu$l of a buffer containing 10 mM Tris-HCl (pH7.5), 7 mM MgCl$_2$ , 150 mM NaCl, 0.2 mM EDTA and 7 mM $\beta$-mercaptoethanol at 37°C for 4 hours. After ethanol precipitation, the precipitate was treated with 20 units of Klenow fragment in 20 $\mu$l of a buffer containing 50 mM Tris-HCl (pH7.2), 10 mM MgCl$_2$ , 0.1 mM DTT and 80 $\mu$M dNTP at 22°C for 30 minutes. After ethanol precipitation, the precipitate was treated with one unit of alkaline phosphatase (bovine intestine) in 20 $\mu$l of a buffer containing 50 mM Tris-HCl (pH9.0), 1 mM MgCl$_2$ , 0.1 mM ZnCl$_2$ and 1 mM spermidine at 37°C for 30 minutes. After phenol treatment, ethanol precipitation was carried out. The precipitate was dissolved in 20 $\mu$l of a solution containing 10 mM Tris-HCl (pH7.5) and 1 mM EDTA. This solution contained 0.1 $\mu$g/$\mu$l of DNA. One $\mu$g of this DNA and 1 $\mu$g of the above-mentioned DNA fragment (B) were reacted with 1.5 units of T4 DNA ligase in 20 $\mu$l of a buffer containing 66 mM Tris-HCl (pH7.6), 6.6 mM MgCl$_2$ , 10 mM DTT and 1 mM ATP at 15°C for 15 hours. The reaction mixture was used to transform E. coli JM103 according to a conventional procedure, and obtained ampicillin resistant transformants were screened for colonies containing plasmid pDPAT2 shown in Fig. 13, and the plasmid was isolated according to a conventional procedure.

Example 2. Construction of Plasmid pHA00

18

5 µg of plasmid pPE3 (same as pMUT4L) was digested with 10 units of PvuI in 50 µl of a buffer containing 10 mM Tris-HCl (pH8.0), 7 mM MgCl$_2$ , 150 mM KCl and 7 mM β-mercaptoethanol at 37°C for 5 hours. After ethanol precipitation, the precipitate was digested with 10 units of EcoRI in 50 µl of a buffer containing Tris-HCl (pH7.5), 7 mM MgCl$_2$ , 100 mM NaCl and 7 mM β-mercaptoethanol at 37°C for 2 hours. The reaction mixture was subjected to 1.2% agarose gel electrophoresis, and a DNA fragment of about 2000 bp was recovered by a conventional means. The DNA fragment thus recovered was designated as DNA fragment (A).

Next, 10 µg of plasmid pPE3 was digested with 15 units of PvuI in 50 µl of a buffer containing 10 mM Tris-HCl (pH8.0), 7 mM MgCl$_2$ , 150 mM KCl and 7 mM β-mercaptoethanol at 37°C for 5 hours. After ethanol precipitation, the precipitate was digested with 15 units of BamHI in 50 µl of a buffer containing 10 mM Tris-HCl (pH8.0), 7 mM MgCl$_2$ , 100 mM NaCl and 2 mM β-mercaptoethanol at 30°C for 3 hours. The reaction mixture was subjected to 1.2% agarose gel electrophoresis, and DNA fragments of about 2300 bp and about 1346 bp were recovered according to a conventional means. The DNA fragment of about 2300 bp thus recovered was designated as DNA fragment (B). Two µg of the DNA fragment of about 1346 bp recovered as above was digested with 5 units of BalI in 30 µl of a buffer containing 20 mM Tris-HCl (pH8.5), 7 mM MgCl$_2$ and 7 mM β-mercaptoethanol at 37°C for 8 hours. The reaction mixture was subjected to 1.2% agarose gel electrophoresis, and DNA fragment of about 750 bp was recovered by a conventional procedure. This DNA fragment was designated as DNA fragment (C).

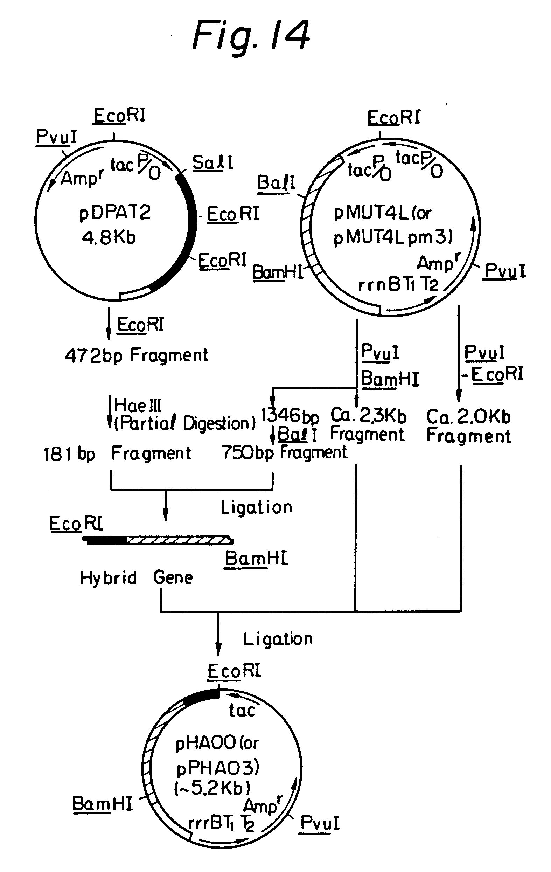

On the other hand, 10 µg of plasmid pDPAT2 constructed in Example 1 was digested with 15 units of EcoRI in 100 µl of a buffer containing 50 mM Tris-HCl (pH7.5), 7 mM MgCl$_2$ , 100 mM NaCl and 7 mM β-mercaptoethanol at 37°C for 3 hours. The reaction mixture was subjected to 1.2% agarose gel electrophoresis, and DNA fragment of about 472 bp was recovered by a conventional procedure. 2 µg of the DNA thus recovered was digested with one unit of HaeIII in 50 µl of a buffer containing 10 mM Tris-HCl (pH7.5), 7 mM MgCl$_2$ , 60 mM NaCl and 7 mM β-mercaptoethanol at 37°C for 30 minutes. The reaction mixture was subjected to 5% polyacrylamide gel electrophoresis, and a DNA fragment of about 180 bp was recovered by a conventional procedure. One µg of this DNA fragment and 1 µg of the above-mentioned DNA fragment (C) were reacted with one unit of T4 DNA ligase in 20 µl of a buffer containing 66 mM Tris-HCl (pH7.6), 6.6 mM MgCl$_2$ , 10 mM DTT and 1 mM ATP at 15°C for 12 hours. The reaction mixture was subjected to 1.2% agarose gel electrophoresis, and a DNA fragment of about 931 bp was recovered by a conventional means. One µg of this DNA fragment, 1 µg of DNA fragment (A) and 1 µg of DNA fragment (B) were ligated with 2 units of T4 DNA ligase in 20 µl of a buffer containing 66 mM Tris-HCl (pH7.6), 6.6 mM MgCl$_2$ , 10 mM DTT and 1 mM ATP at 15°C for 12 hours. The reaction mixture was used to transform E. coli JM103, and resulting ampicillin resistant transformants were screened for colonies containing plasmid pHA00 shown in Fig. 14, and the plasmid was isolated by a conventional means.

Example 3. Construction of Plasmid pHA03

Plasmid pHA03 was constructed according to the same procedure as described in Example 2 except that plasmid pPE3pm3 (same as pMUT4Lpm3) was used in place of plasmid pPE3 (Fig. 14).

Example 4. Construction of Plasmid pHA20

Five µg of the plasmid pHA00 constructed in Example 2 was digested with 7 units of PstI in 20 µl of a buffer containing 20 mM Tris-HCl (pH7.5), 10 mM MgCl$_2$ and 50 mM (NH$_4$)$_2$SO$_4$ at 37°C for 4 hours. The reaction mixture was subjected to ethanol precipitation, and the precipitate was then digested with 10 units of ScaI in 20 µl of a buffer containing 10 mM Tris-HCl (pH7.5), 7 mM MgCl$_2$ , 125 mM NaCl and 7 mM β-mercaptoethanol at 37°C for 8 hours. The reaction mixture was subjected to 1.2% agarose gel electrophoresis, and a DNA fragment of about 1100 bp was recovered by a conventional means. This DNA fragment was designated as DNA fragment (A).

Five µg of plasmid pHA00 was digested with 7 units of PstI in 20 µl of a buffer containing 20 mM Tris-HCl (pH7.6), 10 mM MgCl$_2$ and 50 (NH$_4$)$_2$SO$_4$ mM at 37°C for 4 hours. The reaction mixture was subjected to ethanol precipitation, and the precipitate was then digested with 10 units of EcoRI in 20 µl of a buffer containing 50 mM Tris-HCl (pH7.5), 7 mM MgCl$_2$ , 100 mM NaCl and 9 mM β-mercaptoethanol at 37°C for 3 hours. After ethanol precipitation, the precipitate was subjected to 0.7% agarose gel electrophoresis, and DNA fragment of about 3500 bp was recovered by a conventional means. This DNA fragment was designated as DNA fragment (B).

In the other hand, 15 µg of the plasmid pDPAT2 constructed in Example 1 was digested with 20 units of BglII in 100 µl of a buffer containing 10 mM Tris-HCl (pH7.5), 7 mM MgCl$_2$ , 100 mM NaCl and 7 mM β-

EP 0 231 883 B1

mercaptoethanol at 37°C for 4 hours. After ethanol precipitation, the precipitate was digested with 25 units of ScaI in 100 $\mu$l of a buffer containing 10 mM Tris-HCl (pH7.5), 7 mM MgCl$_2$ , 125 mM NaCl and 7 mM $\beta$-mercaptoethanol at 37°C for 10 hours. The reaction mixture was subjected to 1.2% agarose gel electrophoresis, and DNA fragment of about 625 bp was recovered by conventional means. This DNA fragment was designated as DNA fragment (C).

Ten $\mu$g of plasmid pDPAT2 was digested with 15 units of BglII in 100 $\mu$l of a buffer containing 10 mM Tris-HCl (pH7.5), 7 mM MgCl$_2$ , 100 mM NaCl and 7 mM $\beta$-mercaptoethanol at 37°C for 4 hours. After ethanol precipitation, the precipitate was digested with 10 units of EcoRI in 50 $\mu$l of a buffer containing 50 mM Tris-HCl (pH7.5), 7 mM MgCl$_2$ , 100 mM NaCl and 7 mM $\beta$-mercaptoethanol at 37°C for 3 hours. The reaction mixture was subjected to 5% polyacrylamide gel electrophoresis, and DNA fragment of about 150 bp was recovered according to a conventional procedure. This DNA fragment was designated as DNA fragment (D).

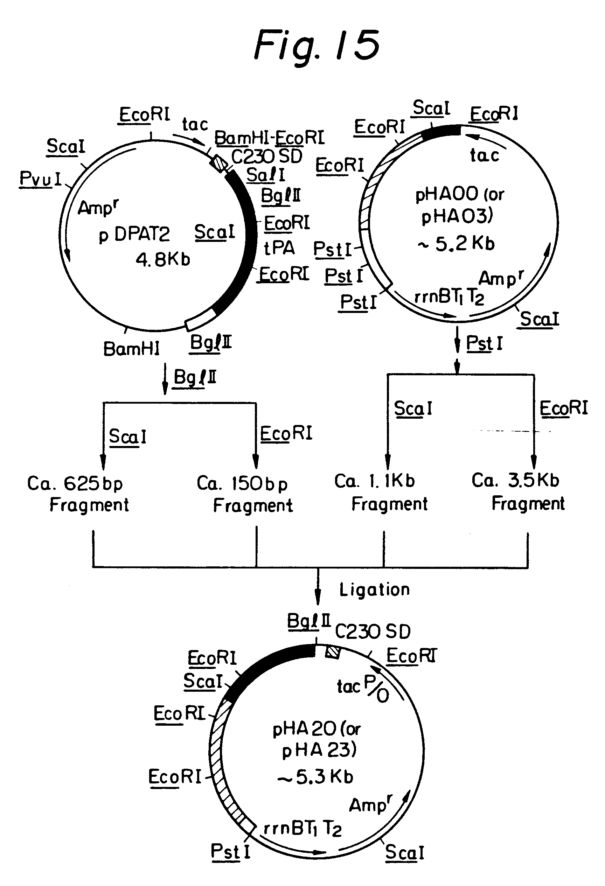

One $\mu$g of DNA fragment (A), 1 $\mu$g of DNA fragment (B), 1 $\mu$g of DNA fragment (C) and 1 $\mu$g of DNA fragment (D), all described above, were ligated with 4 units of T4 DNA ligase in 30 $\mu$l of a buffer containing 66 mM Tris-HCl (pH7.6), 6.6 mM MgCl$_2$ , 10 mM DTT and 1 mM ATP at 15°C for 16 hours. The reaction mixture was used to transform E. coli JM103 according to a conventional procedure, and resulting ampicillin resistant transformants were screened for colonies containing plasmid pHA20 shown in Fig. 15, and the plasmid was recovered by a conventional means.

Example 5. Construction of Plasmid pHA23

Plasmid pHA23 was constructed according to the same procedure as described in Example 4 except that plasmid pHA03 constructed in Example 3 was used in place of plasmid pHA00 used for construction of plasmid pHA20 (Fig. 15).

Example 6. Construction of Plasmid pHA13

Twenty-five $\mu$g of the plasmid pHA23 constructed in Example 5 was digested with 30 units of BglII in 200 $\mu$l of a buffer containing 10 mM Tris-HCl (pH7.5), 7 mM MgCl$_2$ , 100 mM NaCl and 7 mM $\beta$-mercaptoethanol at 37°C for 4 hours. After ethanol precipitation, the precipitate was treated with 4 units of Klenow fragment in 50 $\mu$l of a buffer containing 50 mM Tris-HCl (pH7.2), 10 mM MgCl$_2$ , 0.1 mM DTT and 80 $\mu$M dNTP at 22°C for 30 minutes. After phenol treatment, ethanol precipitation was carried out. The precipitate was digested with 30 units of PstI in 100 $\mu$l of a buffer containing 20 mM Tris-HCl (pH7.5), 10 mM MgCl$_2$ and 50 mM (NH$_4$)$_2$SO$_4$ at 37°C for 4 hours. The reaction mixture was subjected to 0.7% agarose gel electrophoresis, and DNA fragment of about 3500 bp designated as DNA fragment (A), and DNA fragment of about 1700 bp designated as DNA fragment (B) were recovered by a conventional means.

Seven $\mu$g of the DNA fragment (B) was digested with 10 units of RsaI in 50 $\mu$l of a buffer containing 10 mM Tris-HCl (pH8.0), 10 mM MgCl$_2$ , 50 mM NaCl and 7 mM $\beta$-mercaptoethanol at 37°C for 6 hours. The reaction mixture was subjected to 1.2% agarose gel electrophoresis, and DNA fragment of about 1100 bp designated as DNA fragment C, and DNA fragment of about 626 bp designated as DNA fragment D were recovered by a conventional means.

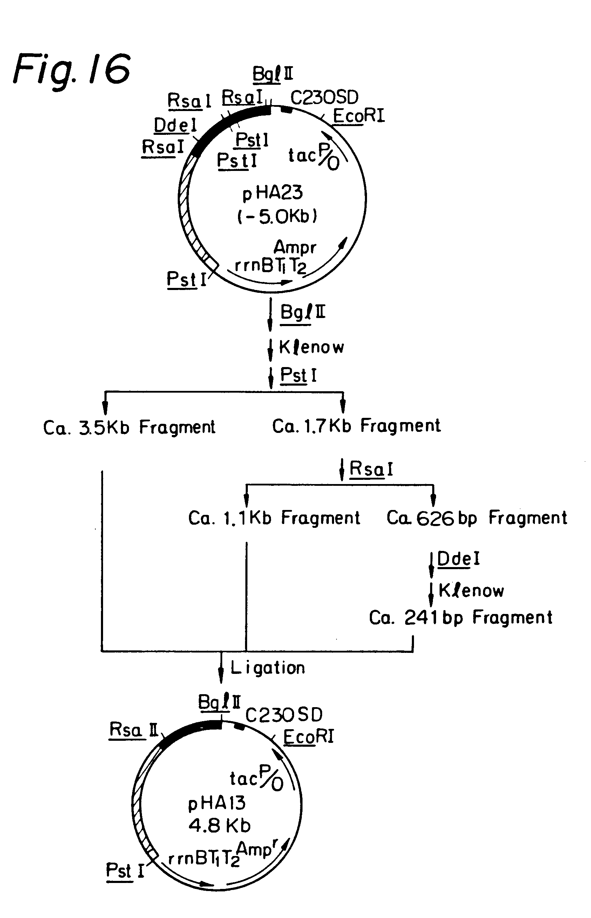

Two $\mu$g of DNA fragment (D) was digested with 5 units of DdeI in 20 $\mu$l of a buffer containing 100 mM Tris-HCl (pH7.5), 5 mM MgCl$_2$ , 100 mM NaCl and 7 mM $\beta$-mercaptoethanol at 37°C for 8 hours. After ethanol precipitation, the precipitate was treated with one unit of Klenow fragment in 20 $\mu$l of a buffer containing 50 mM Tris-HCl (pH7.2), 10 mM MgCl$_2$ , 0.1 mM DTT and 80 $\mu$M dNTP at 22°C for 30 minutes. After phenol treatment, ethanol precipitation was carried out. The precipitate was subjected to 5% polyacrylamide gel electrophoresis, and DNA fragment of about 241 bp was recovered by a conventional procedure. One $\mu$g of this DNA fragment, 0.5 $\mu$g of DNA fragment (A), and 0.5 $\mu$g of DNA fragment (C) were ligated with 3 units of T4 DNA ligase in 20 $\mu$l of a buffer containing 66 mM Tris-HCl (pH7.6), 6.6 mM MgCl$_2$ , 10 mM DTT and 1 mM ATP at 15°C for 15 hours. The reaction mixture was used to transform E. coli JM103, and resulting ampicillin resistant transformants are screened for colonies containing plasmid pHA13 shown in Fig. 16, and the plasmid was isolated by a conventional means.

Example 7. Expression and Extraction of Gene Product

(a) E. coli JM103/pDPAT2, JM103/pHA00, JM103/pHA03, JM103/pHA20, JM103/pHA23 and JM103/pHA13 prepared in Examples 1 to 5, and E. coli JM103/pMUT4L prepared in Reference Example 15 were cultured in 100 ml of LB medium supplemented with 100 $\mu$g/ml ampicillin at 37°C with shaking.

20

When $OD_{600}$ of the culture medium reached to 0.6, isopropyl-$\beta$,D-thiogalactopyranoside was added to the medium to 1 mM of the final concentration, and the culture was continued for additional 5 hours. The cultured broth was then transferred to an ice bath. The cooled broth was centrifuged at 3000 rpm for 10 minutes at 4°C to collect cells, which were then suspended in 50 ml of a buffer containing 50 mM Tris-HCl (pH7.5) and 100 mM NaCl. The suspension was then centrifuged to collect cells, which were resuspended in 10 ml of the same buffer. Next, the cells were disrupted by sonication, and the disrupted cells were centrifuged at 15,000 rpm for 10 minutes at 4°C to obtain a precipitate.

(b) Each of the precipitates prepared in step (a) was suspended in 10 ml of a buffer containing 7.5 M guanidine hydrochloride and 50 mM Tris-HCl (pH7.5), and the suspension was allowed to stand at a room temperature for 90 minutes. Next, the suspension was centrifuged at 10,000 rpm for 10 minutes, and the supernatant was diluted to 5 ml of solution containing 1 M guanidine hydrochloride, 0.05 M Tris-HCl (pH7.5), 2 mM reduced type glutathion and 0.2 mM oxidated type glutathion, and incubated overnight at a room temperature. Next, the solution was dialyzed against 100 volumes of a solution containing 10 mM Tris-HCl (pH7.4) and 0.4 M NaCl at 4°C for 4 hours, and then 100 volumes of a solution containing 10 mM Tris-HCl (pH7.4) and 0.1 M NaCl for 2 hours to obtain an extract.

## Example 8.

Each precipitate obtained from each transformant in Example 7(a), in an amount corresponding to 1 ml of cultured broth, was added with 20 $\mu$l of a sampling solution containing 3 M urea, 0.08 M dithiothreitol and 1% SDS, and the mixture was heated at 95°C for 5 minutes. The heat-treated product was centrifuged to eliminate an insoluble matter, and 8 $\mu$l of the supernatant thus obtained was applied to an SDS-polyacrylamide gradient gel (Nature, 292 128, 1981), and electrophoresis was carried out at 50 V overnight. After the electrophoresis, the gell was stained by soaking it in a staining solution consisting of 1% Coomassie Blue, 10% acetic acid, 25% isopropanol and water for 1.5 hours, and then soaked in a destaining solution consisting of 10% acetic acid, 10% isopropanol and water for 2 hours followed by a solution consisting of 5% methanol and 7% acetic acid and water for 2 hours. An example of gel pattern thus obtained was shown in Fig. 17A. In Fig. 17A, a sample of an expression product from E. coli JM103/pHA03, which produces a hybrid plasminogen activator-like polypeptide of the present invention (HA03) (lane 2), was compared with a sample of an expression product from E. coli JM103/pPE3 (pMUT4L) (lane 1), which produces native human prourokinase (UK), and a sample of an expression product from E. coli JM103/pDPAT2, which produces native human tissue plasminogen activator (t-PA) (lane 3), by electrophoresis.

On the other hand, each sample prepared as above described was subjected to the same SDS-polyacrylamide gradient gel electrophoresis as described above, and separated proteins on the gel were then transferred to a nitrocellulose filter and the filter was subjected to an enzymeimmunoassay (EIA) (S. Tabe, Saibo Kogaku, Vol 2, p1061, 1983). That is, after proteins on the SDS-polyacrylamide gel were transferred to a nitrocellulose filter, to prevent non-specific adsorption, the nitrocellulose filter was soaked in a solution of 3% gelatin in TBS (20 mM Tris-HCl, pH7.5, and 500 mM NaCl), and then the filter was soaked for 2 hours in a solution containing anti-tissue plasminogen activator antiserum obtained from a rabbit immunized with tissue plasminogen activator. The filter was washed throughout with TBS solution, and then soaked in a solution containing a horseradish peroxidase-labeled anti-rabbit antibody IgG (goat) for an hour. After a thorough washing, the filter was soaked in a developing solution containing 20 mM Tris-HCl (pH7.5), 500 mM NaCl, 0.06% 4-chloro-1-naphtol and 0.015% $H_2O_2$ for 5 minutes. An example of the obtained results is shown in Fig. 17C. The same procedure as described above was applied to an anti-urokinase antiserum. An example of the obtained results is shown in Fig. 17B.

From the comparison of the patterns shown in Figs. 17 A, B, and C, it is evident that the hybrid polypeptide of the present invention is immunoreative to both antiserum against tissue plasminogen activator and antiserum against urokinase.

## Example 9. Preparation of Fibrin Affinity Sepharose® Column

Three g of fibrinogen (Daiichi Kagaku Yakuhin Kogyo, Japan; Type 2, plasminogen free) was dissolved in 100 ml of a linking buffer containing 0.1 M $NaHCO_3$ (pH8.3) and 0.5 M NaCl. The solution was centrifuged to at 10000 xg for 10 minutes eliminate insoluble materials, and the supernatant was used for subsequent reaction.

Two g of CNBr-activated Sepharose® 4B (Pharmacia) was swollen with 30 ml of 1 mM HCl to obtain about 7 ml of a carrier. The carrier preparation thus obtained was loaded on a G3 glass filter, and was washed four times with 25 ml of 1 mM HCl. Moreover, the carrier was washed with 20 ml of the linking buffer and the washed carrier was suspended in 20 ml of the same buffer. Immediately after the preparation, the suspension was added to the above-mentioned fibrinogen solution, and the mixture was reacted at a room temperature for two hours with stirring. After the reaction, the supernatant was removed. The residual material was added with 50 ml of 0.2 M glycine (pH8.0) and the mixture was stirred at a room temperature for two hours to inactivate residual active groups. After removing the supernatant, the residual material was washed alternatively five times with 20 ml each of 0.2 M glycine solution (pH8.0) and an acetate buffer containing 0.1 M sodium acetate (pH5.0) and 0.5 M NaCl on a glass filter to finally obtain about 7 ml of a fibrinogen-linked carrier. This carrier was filled in a column ⌀16 mm × 70 mm.

Three-hundred units of bovine thrombin (Mochida Seiyaku, Japan) was dissolved in 3 ml of distilled water, and the solution was gradually run through the column, prepared as above, for two hours to convert the fibrinogen linked to the carrier to fibrin. The column was then equilibrated with 100 μl of a buffer containing 0.02 M Tris-HCl (pH7.5), 0.15 M NaCl and 0.05% Triton X-100.

Example 10.

The extract prepared in Example 7 (b), in an amount corresponding to 5 μg of the expression product, was adjusted to 100 μl of a final liquid volume containing 0.02 M Tris-HCl (pH7.5), 0.15 M NaCl and 0.05% Triton® X-100. The solution thus prepared was run through the column prepared in Example 9 at a flow rate of 0.5 ml/minute allowing the reaction of the expression product in the applied solution with the fibrin linked on the carrier in the column. Subsequently, the column was washed with 40 ml of a buffer containing 0.02 M Tris-HCl (pH7.4), 0.15 M NaCl and 0.05% Triton® X-100 at the above-mentioned flow rate. Next, the column was eluted with an eluting buffer containing 2 M KSCN, 0.02 M Tris-HCl (pH7.4) and 0.05% Triton® X-100. During the above-mentioned washing and elution, all effluent was fractionated by 2 ml using a fraction collector. Each fraction was measured for plasminogen activator activity by fibrinolytic activity on a plasminogen-containing fibrin plate.

Fibrinolytic activity was measured as follows: A solution of 0.1 g of fibrinogen (Daiichi Kagaku Yakuhin, Japan; Fibrinogen Type 1) dissolved in 5 ml of 0.06 M phosphate buffer and a solution of 0.025 g of agarose (Sigma) dissolved in 5 ml of the same buffer was mixed. To the mixture bovine thrombin (Mochida Seiyaku, Japan) to give a final concentration of 3 NIH units/ml (1 mM), and after stirring, the mixture was poured onto a petri dish having an inner diameter of 8.5 cm to prepare a fibrin plate. Ten μl each of samples were spotted on the fibrin plate, and the plate was incubated at 37°C for 14 hours. Diameters of developed lysis circles were measured. According to the same procedure as described above, standard samples having known units prepared from commercial urokinase of urine origin (UK) were spotted on the fibrin plate, and the plate was incubated at 37°C for 14 hours, and the diameters of developed lysis circles were measured. From these measurements, a calibration curve of activity versus diameter of lysis circle was prepared. By applying the measurements of the lysis circle diameter of test samples to the calibration curve, the activity of the test sample was obtained.

Fractions eluted by the washing buffer which did not contain KSCN were designated as non-adsorbed fractions, while fractions eluted by the eluting buffer which contained 2 M KSCN were designated as adsorbed fractions. Elution profiles for each sample are shown in Figs. 18-2 to 18-4. As controls, a commercially available tissue plasminogen activator (TPA), a commercially available urokinase derived from urine (UK) and recombinant TPA (DPAT2) derived from E. coli were treated by the same procedure as described above, and the control elution profiles are shown in Fig. 18-1.

As seen from these figures, while the commercially available urokinase (UK) is not adsorbed to a fibrin-sepharose carrier, hybrid plasminogen activator-like polypeptides of the present invention, which contain an entire or part of kringle region responsible for the affinity to fibrin of human tissue plasminogen activator, as well as a recombinant tissue plasminogen activator produced in E. coli and a commercially available tissue plasminogen activator, have the ability to bond to a fibrin-Sepharose® carrier.

Example 11. Km Determination Using Synthetic Substrate S-2288

S-2288 (Kabi) was dissolved in a reaction buffer containing 0.1 M Tris-HCl (pH8.0), 0.01% Triton® X-100 and 0.5 M NaCl, and finally, each solutions containing 0.1 mM, 0.25 mM, 0.5 mM, 0.75 mM and 1 mM S-2288 were prepared.

The extracts HA03 and HA20 prepared in Example 7(b) and a solution of commercially available urokinase (UK), each 10 μl, was diluted with the reaction buffer to a final volume of 400 μl. To these samples, 2 μl of a solution of 1 mg/ml plasmin (Sigma) was added, and the reaction mixtures were allowed

22

to stand at 37°C for one hour in a incubator. Then, the reaction mixtures were promptly transferred to ice water, and added with 2 μl each of a solution of 5 mg/ml soybean trypsin inhibitor (Sigma) to terminate the reaction with plasmin. Five reaction mixtures were prepared for each test sample, and to each reaction mixture, the above-mentioned S-2288 solution having a different concentration was added. These reaction mixtures were allowed to stand at 37°C for one hour in an incubator. After that, the reaction mixture were immediately transferred to an ice water bath and added with 50 μl each of 10% acetic acid solution to terminate the reaction. As a control, the same procedure as described above was carried out using 400 μl of the reaction buffer in place of the 400 μl of the test sample.

For each reaction mixture, absorbance at 405 nm was measured, and for each sample, the difference between sample absorbance and control absorbance, i.e., A value, was obtained. The reaction rate was v = A/60, therefore 1/v = 60/A, wherein "60" denotes that the reaction was carried out for 60 minutes. A Lineweaver-Burk's reciprocal plot graph was formed by plotting the 1/v value on an ordinate axis and 1/S value on an abscissa axis, wherein S represents a concentration (M) of a substrate in the reaction mixture. From the graph, the Km values set forth in the following table were obtained.

## Table

| Sample | Km measured (mol/l) | Km from Kabi S-2288 data sheet (mol/l) |
|---|---|---|
| HA03 | $2.0 \times 10^{-4}$ | |
| HA20 | $2.0 \times 10^{-4}$ | |
| Commercial UK | $2.0 \times 10^{-4}$ | $2.0 \times 10^{-4}$ |

As seen from the table, the present hybrid polypeptide HA03 consisting of a polypeptide region from 161th Met to 219th Gly corresponding to about a half of the kringle of a human tissue plasminogen activator and a polypeptide region from 150th Gln to 411th Leu of human prourokinase, wherein 157th Phe is replaced by Asp, and the present hybrid polypeptide HA20 consisting of a polypeptide region from first Ser to 219th Gly including an entire kringle region of human tissue plasminogen activator and a polypeptide region from 150th Gln to 411th Leu of human prourokinase exhibit the same Km value as commercially available urokinase.

This means that the region responsible for enzyme activity of the present hybrid polypeptide exhibits the same characteristic as the corresponding part of native urokinase, regardless of the length of the region responsible for fibrin affinity derived from the tissue plasminogen activator, and regardless of the presence or absence of point mutation in a serine protease region, i.e., at 157th amino acid.

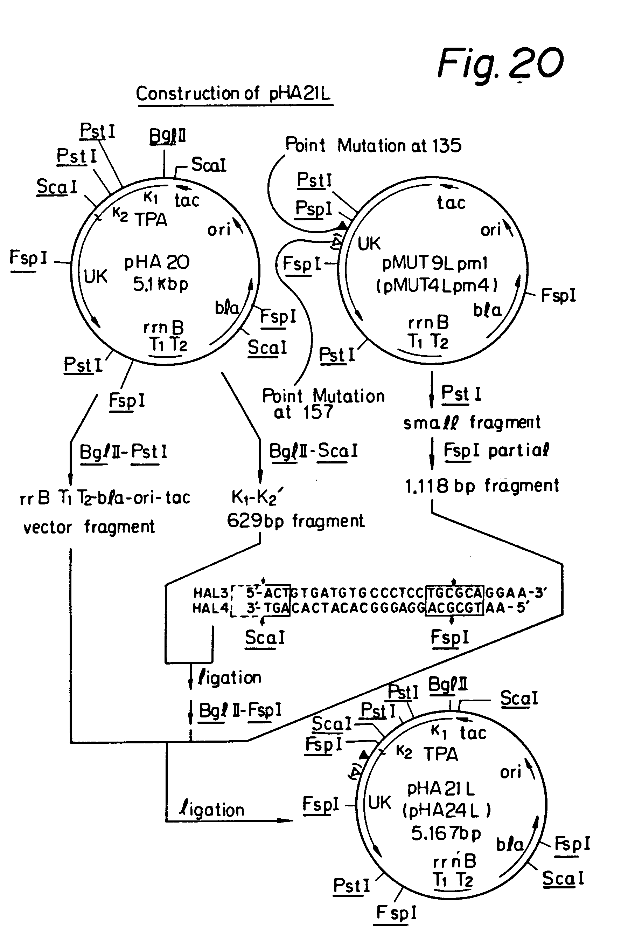

Example 11. Construction of Plasmid pHA21L (Fig. 20)

Ten μg of the plasmid pHA20 constructed in Example 4 was digested with 100 units of BgIII and 96 units of PstI in 200 μl of a buffer containing 20 mM Tris-Hcl (pH7.5), 10 mM MgCl₂ and 100 μM NaCl at 37°C for 2 hours. The reaction mixture was subjected to 0.7% agarose gel electrophoresis, and a DNA fragment of about 3400 bp was recovered by a conventional means. This DNA fragment was designated as DNA fragment (E).

Moreover, 5 μg of the same plasmid pHA20 was digested with 100 units of BgIII and 120 unit of ScaI in 200 μl of a buffer containing 10 mM Tris-Hcl (pH7.5), 7 mM Mgcl₂, 125 mM NaCl and 7 mM β-mercaptoethanol at 37°C for 4 hours. The reaction mixture was then subjected to 0.7% agarose gel electrophoresis, and a DNA fragment of about 630 bp was recovered by a conventional means. This DNA fragment was designated as DNA fragment (F).

On the other hand, 5 μg of the plasmid pMUT9Lpml constructed in Reference Example 18 was digested with 30 units of PstI in 100 μl of a buffer containing 200 mM Tris-HCl (pH7.5), 10 a MgCl₂ and 100 mM NaCl at 37°C for 2 hours. The reaction mixture was then subjected to 0.7% agarose gel electrophoresis, and a DNA fragment of about 1200 bp was recovered by conventional means. This DNA fragment was partially digested with 40 unites of FspI in 100 $\mu$l of a buffer containing 10 mM Tris-HCl (pH7.4), 10 mM MgCl$_2$, 50 mM NaCl and 10 mM ß-mercaptoethanol at 37°C for 10 minutes. The reaction mixture was then subjected to 1.2% agarose gel electrophoresis, and a DNA fragment of about 1100 bp was recovered by conventional means. This DNA fragment was designated as DNA fragment (G).

The following two oligonucleotides:

**5'   ACTGTGATGTGCCCTCCTGCACAGGAA  3'**

**3'   TGACACTACACGGGAGGACGTGTCC    5'**

were synthesized by phospite method. 1 $\mu$g each of these syntetic oligonucleotides were mixed in 30 $\mu$l of n solution containing 50 $\mu$M Tris-HCl (pH7.6), 10 mM MgCl$_2$ and 10 mM ß-mercaptoethanol, heated at 70°C for 5 minutes, and then cooled on ice. The reaction mixture was added with 20 units of T4 polynucleotide kinase, and incubated at 37°C for one hour to phosphorylate 5'-end of the oligonucleotides. The reaction mixture was heated at 70°C for 2 minutes and the allowed to stand at a room temperature for 5 minutes to anneal two synthetic oliganucleotides. After ethanol precipitation of the annealed product, the precipitate was mixed with the above-mentioned DNA fragment (F), and ligated by 5 units of T4 DNA ligase in 20 $\mu$l of a buffer containing 66 mM Tris-HCl (pH7.6), 6.6 mM MgCl$_2$, 10 mM DTT and 10 mM ATP at 12°C for 15 hours. After phenol extraction and ethanol precipitation of the reaction mixture, the reaction mixture was digested with 10 units of FspI and 20 units of Bg1II in 50 $\mu$l of a buffer containing 10 mM Tris-HCl (pH7.4), 10 mM MgCl$_2$, 50 mM NaCl and 10 mM ß-mercaptoethanol at 37°C for 4 hours. The reaction mixture was subjected to 1.0% agarose gel electrophoresis, and DNA fragment of about 650 bp was recovered by a conventional means. This DNA fragment was designated as DNA fragment (H).

The DNA fragments (E), (G) and (H), all prepared as described above, were mixed, and ligated by 5 units of T4 DNA ligase in 20 $\mu$l of a buffer containing 66 mM Tris-HCl (PH7.6), 6.6 mM MgCl$_2$, 10 mM DTT and 10 mM ATP at 12°C for 15 hours. The reaction mixture was used to transform E.coli JM103 according to a conventional means, and ampicillin resistant transformants thus obtained were screened for a colony containing plasmid pHA21L, and the plasmid pHA21L was isolated by a conventional means.

## Example 12. Construction of Plasmid pHA24L

Five $\mu$g of the plasmid pMUT4Lpm4 constructed in Reference Example 17 was digested with 50 units of PstI in 100 $\mu$l of a buffer containing 20 mM Tris-HCl (pH7.5), 10 mM MgCl$_2$ and 100 mM NaCl at 37°c for 2 hours. The reaction mixture was subjected to 0.7% agarose gel electrophoresis, and a DNA fragment of about 1200 bp was recovered by a conventional means. The DNA fragment was partially digested with 40 units of FspI in 100 $\mu$l of a buffer containing 10 mM Tris-HCl (pH7.4), 10 mM MgCl$_2$, 50 mM NaCl and 10 mM $\beta$-mercaptoethanol at 37°C for 10 minutes. The mixture were then subjected to 1.2% agarose gel electrophoresis, and a DNA fragment of about 1100 bp was recovered by a conventional means. This DNA fragment was designated as DNA fragment (I).

On the other hand, DNA fragments (E) and (G) were prepared according to the same procedure as described in Example 11. These DNA fragments (E), (G) and (I) were mixed and ligated by T4 DNA ligase in 20 $\mu$l of a buffer containing 66 mM Tris-HCl (pH7.6), 6.6 mM MgCl$_2$, 10 mM DTT and 10 mM ATP at 12°C for 15 hours. The reaction mixture was used to transform E.coli JM103 according to a conventional means, and ampicillin resistant transformants were screened for a colony containing plasmid pHA24L shown in Fig. 20, and the plasmid pHA24L was isolated according to a conventional means.

## Example 13. Construction of Plasmid pHA11L

Five $\mu$g of the plasmid pHA21L constructed in Example 11 was digested with 50 units of ScaI and 50 units of PstI in 100 $\mu$l of a buffer containing 10 mM Tris-HCl (pH7.5), 7 mM MgCl$_2$, 125 mM NaCl and 7 mM $\beta$-mercaptoethanol at 37°C for 6 hours. The reaction mixture was subjected to 1.0% afarose gel electrophoresis, and a DNA fragment of about 1100 bp was recovered by a conventional means. This DNA fragment was designated as DNA fragment (J).

Moreover, 10 $\mu$g of the same plasmid pHA21L was digested with 100 units of DdeI in 200 $\mu$l of a buffer containing 10 mM Tris-HCl (pH7.5), 7 mM MgCl$_2$, 150 mM NaCl and 7 mM $\beta$-mercaptoethanol at 37°C for 15 hours. The reaction mixture was then subjected to 4.0% agarose gel electrophoresis, and a DNA fragment of about 330 bp was recovered by a conventional method. This DNA fragment was designated as DNA fragment (K).

The following two oligonucleotides:

$$5' \quad \texttt{ATGAAGAGGTGACGTCATGTC} \quad 3'$$

$$3' \quad \texttt{TACTTCTCCACTGCAGTACAGACT} \quad 5'$$

were synthesized by phosphite method. 2 $\mu$g each of these two synthetic oliganucleotides were mixed, and phosphorylation of $5'$-end and annealing ware carried out according to the same procedure described in Example 11. After ethanol precipitation of the annealed oligonucleotides, the precipitate was mixed with the above-prepared DNA fragment (K), and the ligation was carried out according to the same procedure as described in Example 11. After ethanol precipitation, the precipitate was digested with 100 units of ScaI and 100 units of AatII in 100 $\mu$l of a buffer containing 10 mM Tris-HCl (pH7.5), 7 mM MgCl$_2$ , 125 mM NaCl and 7 mM 2-mercaptoethanol at 37°C for 15 hours. The reaction mixture was subjected to 4.0% agarose gel elecrrophoresis, and a DNA fragment of about 250 bp was reocvered by a conventional procedure. This dna fragment was designated as DNA fragment (L).

On the other hand, 5 $\mu$g of the plasmid pMUT9LpmI constructed in Reference Example 18 was digested with 50 units of AatII and 50 units of PstI in 100 $\mu$l of a buffer containing 10 mM Tris-HCl (pH7.5), 7 mM MgCl$_2$ , 60 mM KCl and 7 mM $\beta$-mercaptoethanol at 37°C for 4 hours. The reactin mixture was then subjected to 0.7% agarose gel electrophoresis, and a DNA fragment of about 3000 bp was recovered by a conventional procedure. This DNA fragment was designated as DNA fragment (M).

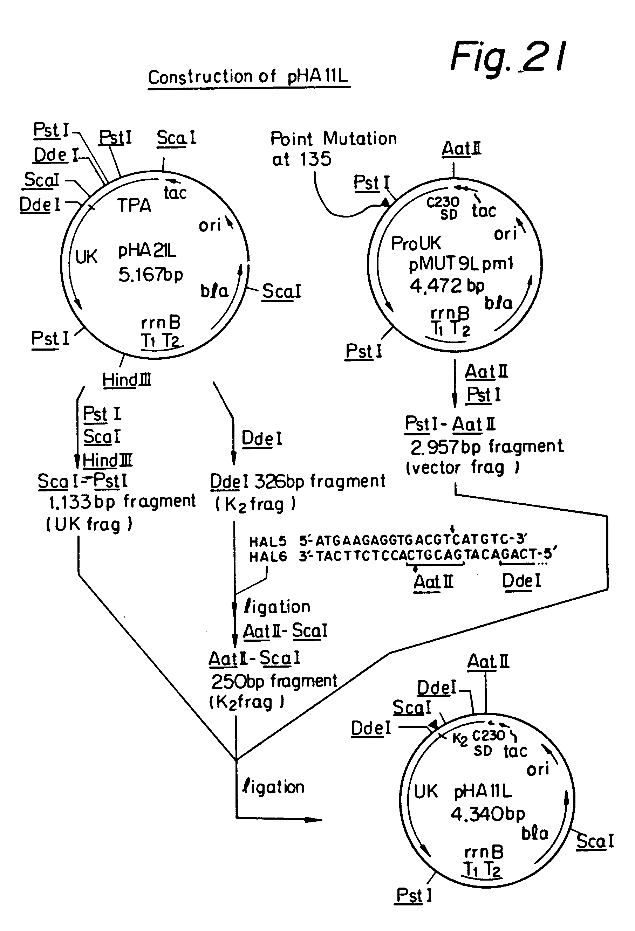

DNA fragments (J), (L) and (M), all prepared as described above, were mixed, and ligated by 5 units of T4 DNA ligase in 20 $\mu$l of a buffer containing 66 mM Tris-HCl (pH7.6), 6.6 mM MgCl$_2$ , 10 M DTT and 10 mM ATP at 12°C for 15 hours. The reaction mixture was used to transform E. coli JM103 according to a conventional procedure, and ampicillin resistant transformants were secreened for a colony containing plasmid pHA11L shown in Fig. 21, and the plasmid pHA11L was isolated according to a conventional procedure.

Example 14. Construction of Plasmid PHA14L

Five $\mu$g of the plasmid pHA24L constructed in Example 12 was digested with 50 units of ScaI and 50 units of PstI in 100 $\mu$l of a buffer containing 10 mM Tris-HCl (pH7.5), 7 mM MgCl$_2$ , 125 mM NaCl and 7 mM ß-mercaptoethanol at 37°C for 6 hours. The reaction mixture was then subjected to 1.0% agarose gel electrophoresis, and a DNA fragment of about 1100 bp was recovered according to a conventional procedure. This DNA fragment was designated as DNA fragment (N).

On the other hand, DNA fragments (L) and (M) were prepared according to exactly the same procedure as decribed in Example 13. These DNA fragments (L), (M) and (N) were mixed, and ligated by 5 units of T4 DNA ligase in 20 $\mu$l of a buffer containing 66 mM Tris-HCl (pH7.6), 6.6 mM MgCl$_2$ , 10 mM DTT and 10 mM ATP at 12°C for 15 hours. The reaction mixture was used to transform E. coli JM103, and ampicillin resistant transformants were screened for a colony containing plasmid pHA14L, and the plasmid pHA14L was isolated according to a conventional procedure.

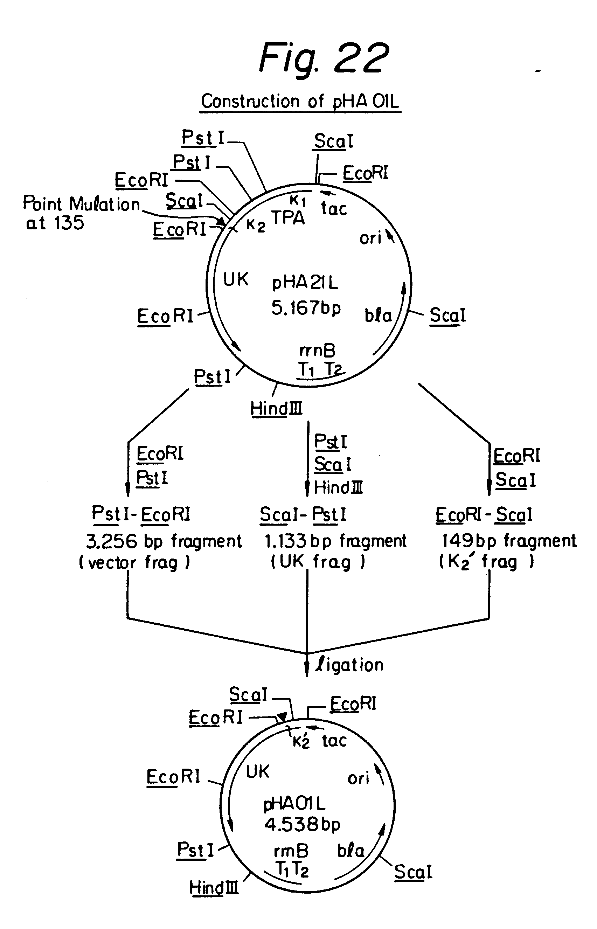

Example 15. Construction of Plasmid pHA01L (Fig. 22)

Five $\mu$g of the plasmid pHA21L constructed in Example 11 was digested with 50 units of EcoRI and 50 unit of PstI in 100 $\mu$l of a buffer containing 20 mM Tris-HCl (pH7.5), 10 mM MgCl$_2$ and 100 mM NaCl at 37°C for 4 hours. The reaction mixture was subjected to 0.7% agarose gel electrophoresis, and a DNA fragment of about 3300 bp was recovered according to a conventional procedure. This DNA fragment was designated as DNA fragment (O).

Five $\mu$g of the same plasmid pHA21L was digested with 50 units of ScaI and 50 units of PstI in 100 $\mu$L of a buffer containing 10 MM Tris-HCl (pH7.5), 7 Mm MgCl$_2$ , 125 mM NaCl and 7 mM ß-mercaptoethanol at 37°C for l5 hours. The reaction mixture was then subjected to 0.7% agarose gel electrophoresis, and a DNA of about 100 bp was recovered according to a conventional procedure. This DNA fragment was designated as DNA fragment (P).

Moreover, 10 $\mu$g of the same plasmid pHA21L was digested with 50 units of EcoRI and 50 units of ScaI in 100 $\mu$l of a buffer containing 10 mM Bris-HCl (pH7.5), 7 mM MgCl$_2$ , 125 mM NaCl and 7 mM $\beta$-mercaptoethanol at 37°C for 15 hours. The reaction mixture was then subjected to 2.0% agarose gel electrophoresis, and a DNA fragment of about 150 bp was recovered according to a conventional procedure. This DNA fragment was designated as DNA fragment (Q).

The DNA fragments (O), )P), and (Q), all prepared as described above, were mixed and ligated by 5 units of T4 DNA ligase in 20 $\mu$l of a buffer containing 66 mM Tris-HCl (pH7.6), 6.6 mM MgCl$_2$ , 10 mM DTT and 10 mM ATP at 12°C for 15 hours. The reaction mixture was used to transform E. coli JM103, and ampicillin resistant tranformants were screened for a colony containing plasmid pHA0l shown in Fig. 22, and the plasmid pHA0l was isolated according to a conventional procedure.

### Example 16. Construction of Plasmid pHA04L

Plasmid pHA04L was constructed according to the same procedure as described in Example 15, except that the plasmid pHA24L constructed in Example 12 was used in place of the plasmid pHA21L.

### Example 17. Expression and Extraction of Hybrid Plasminogen Activator-Like Polypeptid HPA24L

Plasmid pHA24L constructed in Example 12 was used to transform E. coli KY1436 according to a conventional procedure, and the transformant thus obtained was cultured in 5 ml of an L medium supplemented with 50 $\mu$g/ml of ampicillin in a test tube at 30°C over night with shaking. 2 ml of the cultured broth was inoculated to 1 liter of an L medium supplemented with 50 $\mu$g/ml of ampicillin in a 5 liter conical flask, and culturing was carried out at 30°C on an air-shaker at 250 rpm. When absorbance at 600 nm ($OD_{600}$) of the broth reached transfered to an incubator at 37°C, and culturing was carried out at 37°C for an additional 5 hours with shaking to express a hybrid plasminogen activator-like polypeptide HPA24L gene.

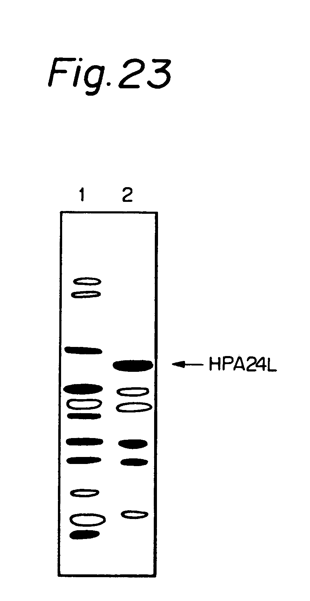

An amount of the cultured broth corresponding to 6 of $OD_{600}$ was transfered to plastic centrifuge tubes, and centrifuged to collect cells. The cells were suspended in 1.0 ml of a buffer containing 0.1 m NaCl and 50 mM Tris-HCl (pH8.0) and the suspension was sonicated to disrupt the cells. The sonicated suspension was then centrifuged to recover an insoluble fraction. An amount of the insoluble fraction corresponding to 1 of $OD_{600}$ was subjected to SDS-polyacrylamide gel electrophoresis according to a conventional procedure. The result is set forth in Fig. 23, wherein lane 2 represents the protein composition from the insoluble fraction and lane 1 represent that from the supernnatant.

On the other hand, an amount of the insoluble fraction corresponding to 4 of $OD_{600}$ was suspended in 160 $\mu$l of a solution containing 6 M guanidine hydrochloride and 25 mM Tris-HCl (pH8.0), and the suspension was allowed to stand at a room temperature for 30 minutes. The suspension was then adjusted to final concentrations of 50 mM Tris-HCl (pH8.0), 1 M guanidine hydrochloride, 2 mM reduced type glutathione, 0.2 mM oxidated type glutathione, 1 mM EDTA and 0.01% Tween 80, and allowed to stand for 15 hours at a room temperature to obtain a crude extract.

### Example 18. Determination of Activity of HPA24L Crude Extract Using Synthetic Substrate S-2444

Ten $\mu$l of the HPA24L crude extract prepared in Example 17 was added to a buffer containing 0.1 M Tris-HCl (pH8.0) and 0.01% Triton® X-100 to make a total volume of 99 $\mu$l. To the mixture 1 $\mu$l of 1 $\mu$g/$\mu$l plasmin solution was added, and the whole was incubated at 37°C for 15 minutes, and then added with 1 $\mu$l of soybeen trypsin inhibitor solution. The mixture was completely mixed. The mixture was then added with 0.7 ml of a buffer containing 2 mM synthetic substrate X-2444, and 0.1 M Tris-HCl (pH8.0) and 0.01% Triton® X-100, and the mixture was incubated at 37°C for 30 minutes, and then 100 $\mu$l of glacial acetic acid was added to terminate the reaction.

Absorbance of the reaction mixture was measured at 405 nm. Enzyme activity in the reaction mixture was calculated according to the formula:

International Unit (I.U.) = $(OD_{405}/0.395) \times 6.5$ and about 120 I.U. of the enzyme activity was obtained.

### Example 19. Expression and Extraction of Other Hyblid Plasminagen Activator-Like Polypeptides

The same procedure as described in Examples 17 and 18 was repeated for plasmids pHA21L, pHA11L, pHA14L, pHA01L, and pHA04L, and similar results as described in Examples 17 and 18 were obtained.

### Literature

(1)　　　Japanese Unexamined Patent Publication No. 60-241889.

(2)     Chirgwin, J.M., Przybyla, A.E., MacDonald, R.J. and Rutter, W.J. (1979) Biochemystry 18, 5294-5299.

(3)     Aviv, H. and Leder, P. (1972) Proc. Natl, Acad. Sci. USA 69, 1408-1412.

(4)     Okayama H. and Berg, P., (1982) Molec. Cell Biol. 2, 101-170.

(5)     Norgard M.V. (1978) Gene 3, 279-292.

(6)     Pennica D. (1983) Nature 301, 214-221.

(7)     Denhart D.T. (1966) Biochem. Biophys. Res. Commun., 23, 643-646.

(8)     A.M. Maxam, and W. Gilbert (1980) Methods Enzymol. 65, 499-560.

(9)     Maniatis, T., Fritsch, E.F. and Sambrook, J. (1982) In: Molecular Cloning, Cold Spring Harbor Laboratory, Cold Spring Harbor, N.Y., pp.254.

(10)     Birnboim, H.C. and Doly, J. (1979) Nucl. Acids Res. 7, 1513-1523.

(11)     Steffens, G.J., Güenzler, W.A. Öetting, F., Frankus, E. and Flohé, L. (1982) Hoppe-Seyler's Z. Physiol. Chem. 363, 1043-1058.

(12)     Güenzler, W.A., supra, (1982) 1055.

(13)     Grunstein M. and Hogness D.S., (1975) Proc. Natl. Acad. Sci., USA, 72, 3961-3965.

(14)     Messing, J., Grec, R. and Seeburg, P.H. (1981) Nucl. Acids Res. 9, 309-321.

(15)     Agarwall, K.L., Brunstedt, J. and Noyes, B.E. (1981) J. Biol. Chem. 256, 1023-1028.

(16)     Stewart A.G., Richards H., Roberts S., Marwick J., Edwards K., I. Bell, J. Smith and R. Derbyshire (1983) Nuc. Acids Res. 11, 6597-6609.

(17)     Brousius J., Ullrich A., Raker M.A., Grey A., Dull T.J., Gutell R.R. and Noller H.F., (1981) plasmid 6, 112-118.

(18)     Brousius, J. (1984) Gene 27, 151-160.

(19)     Brousius J., (1984), Gene, 27 161-172.

(20)     Messing J., (1983) Methods in Enzymology, 101, 20-78.

(21)     Benton W.D. and Davis R.W. (1977), Science 196, 180-182.

## Claims
## Claims for the following Contracting States : BE, CH, DE, FR, GB, IT, LI, NL, SE

1. A hybrid plasminogen activator-like polypeptide comprising a polypeptide region responsible for an affinity to fibrin derived from tissue plasminogen activator polypeptide joined by a peptide bond to a polypeptide region responsible for an enzyme activity derived from prourokinase polypeptide, wherein the hybrid plasminogen activator like-polypeptide exhibits both an affinity to fibrin, and an enzyme activity after the activation.

2. A hybrid plasminogen activator-like polypeptide according to claim 1, wherein the polypeptide region responsible for the affinity to fibrin consists of about two kringles of a N-terminal region of a human tissue plasminogen activator polypeptide.

3. A hybrid plasminogen activator-like polypeptide according to claim 2, wherein the about two kringles is a polypeptide region from an N-terminal first serine to a 215th cysteine or 219th glycine of a human tissue plasminogen activator polypeptide calculated from the first amino acid.

4. A hybrid plasminogen activator-like polypeptide according to claim 1, wherein the polypeptide region responsible for the affinity to fibrin consists of about one kringle of a human tissue plasminogen activator polypeptide.

5. A hybrid plasminogen activator-like polypeptide according to claim 4, wherein the about one kringle is a polypeptide region from the 128th serine to a 215th cysteine or 219th glycine of a human tissue plasminogen activator calculated from the N-terminal amino acid serine as the first amino acid.

6. A hybrid plasminogen activator-like polypeptide according to claim 1, wherein the polypeptide region responsible for the affinity to fibrin consists of about a half of a kringle of a human tissue plasminogen activator polypeptide.

7. A hybrid plasminogen activator-like polypeptide according to claim 6, wherein the about a half kringle is a polypeptide from a 161th methionine to a 215th cysteine or 219th glycine of a human tissue plasminogen activator polypeptide calculated from an N-terminal amino acid serine as the first amino acid.

8. A hybrid plasminogen activator-like polypeptide according to claim 1, wherein the polypeptide region responsible for the enzyme activity consists of a C-terminal enzyme activity region of a human prourokinase polypeptide.

9. A hybrid plasminogen activator-like polypeptide according to claim 8, wherein the polypeptide region responsible for the enzyme activity consists of a polypeptide region from a 150th glutamine to a 411th leucine of a human prourokinase polypeptide calculated from an N-terminal amino acid serine as the first amino acid, wherein 157th phenylalanine is optionally replaced by an acidic amino acid.

10. A hybrid plasminogen activator-like polypeptide according to claim 8, wherein the polypeptide region responsible for the enzyme activity consists of a polypeptide region from a 132th alanine to a 411th leucine of a human prourokinase calculated from an N-terminal amino acid serine as the first amino acid, wherein 157th phenylalanine is optionally replaced by an acidic amino acid, and/or 135th amino acid lysine is optionally replaced by an amino acid other than basic amino acid.

11. A hybrid plasminogen activator-like polypeptide according to claim 9 or 10, wherein the acidic amino acid is aspartic acid, and the amino acid other than basic amino acid is glutamine.

12. A DNA segment coding for the hybrid plasminogen activator-like polypeptide of claim 1, wherein the DNA portion coding for the polypeptide region responsible for the affinity to fibrin is present at the 5'-terminal side and the DNA portion coding for the polypeptide region responsible for the enzyme activity is present at the 3'-terminal side in the DNA segment.

13. A DNA segment according to claim 12, wherein the DNA region coding for the polypeptide region responsible for the affinity to fibrin is a corresponding part of cDNA corresponding to mRNA derived from cells of a human pharyngeal cancer cell line Detroit 562.

14. A DNA segment according to claim 12, wherein the DNA region coding for the polypeptide region responsible for the enzyme activity is a corresponding part of cDNA corresponding to mRNA derived from human kidney tissue cells.

15. A DNA segment according to claim 14, wherein a codon coding for 157th phenylalanine is mutated to a codon coding for an acidic amino acid, and/or a codon coding for 135th lysine is mutated to a codon coding for an amino acid other than basic amino acid.

16. A plasmid containing the DNA segment of claim 12 and capable of expression of the polypeptide.

17. A plasmid according to claim 16 containing a tac promoter/operator and the Shine-Dalgarno sequence of the methapyrocatechase gene derived from Pseudomonas putida.

18. A plasmid according to claim 17 which is a plasmid pHA00, deposited under DSM3628, pHA03, deposited under DSM3627, pHA20, deposited under DSM3626, pHA23, deposited under DSM3625, pHA21L, deposited under DSM3951, pHA24L, deposited under DSM3952;
pHA13 which contains a gene coding for a hybrid plasminogen activator-like polypeptide comprising as its N-terminal half, a polypeptide region from 128th serine to 219th glycine of human tissue plasminogen activator calculated from N-terminal serine as the first amino acid, and as its C-terminal half, a polypeptide region from 150th glutamine to 411th C-terminal leucine of human prourokinase calculated from N-terminal serine on the first amino acid wherein 157th phenylalanine is replaced by aspartic acid, under the control by the tac promoter/operator and the Shine-Dalgarno sequence of the methapyrocatechase gene derived from Pseudomonas putida, and can express the above-mentioned hybrid polypeptide, and which has the structure shown in Fig. 16;
pHA11L which contains a gene coding for a hybrid plasminogen activator-like polypeptide comprising as its N-terminal half, a polypeptide region from 128th serine to 215th cysteine of human tissue plasminogen activator, and as its C-terminal half, a polypeptide region from 132th alanine to 411th C-terminal leucine of human prourokinase calculated from N-terminal serine as the fist amino acid wherein 135th lysine is replaced by glutamine, under the control by the tac promoter and the Shine-Dalgarno

28

sequence of the methapyrocatechase gene derived from Pseudomonas putida, and can express the above-mentioned hybrid polypeptide, and which has the structure shown in Fig. 21;

pHA01L contains a gene coding for a hybrid plasminogen activator-like polypeptide comprising as its N-terminal half, a polypeptide region from 161th methionine to 215th cysteine of human tissue plasminogen activator calculated from N-terminal serine as the first amino acid, and as its C-terminal half, a polypeptide region from 132th alanine to 411th N-terminal leucine of human prourokinase calculated from N-terminal serine as the first amino acid wherein 135th lysine is replaced by glutamine, under the control by the tac promoter/operator and the Shine-Dalgarno sequence of the methapyrocatechase gene derived from Pseudomonas putida, and can express the above-mentioned hybrid polypeptide, and which has the structure shown in Fig. 22;

pHA14L which is identical with the plasmid pHA11L except that the codon for 157th phenylalanine is substituted by the codon for aspartic acid; or

pHA04L which is identical with the plasmid pHA01L except that the codon for 157th phenylalanine is substituted by the codon for aspartic acid.

**19.** Escherichia coli transformed with the plasmid of Claim 16.

**20.** A process for production of the hybrid plasminogen activator-like polypeptide of Claim 1 comprising culturing the E. coli of Claim 19 and recovering the polypeptide from a cultured product.

**Claims for the following Contracting State : ES**

**1.** A process for production of a hybrid plasminogen activator-like polypeptide comprising a polypeptide region responsible for an affinity to fibrin, derived from tissue plasminogen activator polypeptide, joined by a peptide bond to a polypeptide region responsible for an enzyme activity derived from prourokinase polypeptide, wherein the hybrid plasminogen activator-like polypeptide exhibits both an affinity to fibrin, and an enzyme activity after the activation, which process comprises culturing a host transformed with a plasmid containing a DNA segment coding for the polypeptide and recovering the polypeptide from a cultured product.

**2.** A process according to Claim 1, wherein the polypeptide region responsible for the affinity to fibrin consists of about two kringles of a N-terminal region of a human tissue plasminogen activator polypeptide.

**3.** A process according to Claim 2, wherein the about two kringles is a polypeptide region from an N-terminal first serine to a 215th cysteine or 219th glycine of a human tissue plasminogen activator polypeptide calculated from the first amino acid.

**4.** A process according to Claim 1, wherein the polypeptide region responsible for the affinity to fibrin consists of about one kringle of a human tissue plasminogen activator polypeptide.

**5.** A process according to Claim 4, wherein the about one kringle is a polypeptide region from the 128th serine to a 215th cysteine or 219th glycine of a human tissue plasminogen activator calculated from the N-terminal amino acid serine as the first amino acid.

**6.** A process according to Claim 1, wherein the polypeptide region responsible for the affinity to fibrin consists of about a half of a kringle of a human tissue plasminogen activator polypeptide.

**7.** A process according to Claim 6, wherein the about a half kringle is a polypeptide from a 161th methionine to a 215th cysteine or 219th glycine of a human tissue plasminogen activator polypeptide calculated from an N-terminal amino acid serine as the first amino acid.

**8.** A process according to Claim 1, wherein the polypeptide region responsible for the enzyme activity consists of a C-terminal enzyme activity region of a human prourokinase polypeptide.

**9.** A process according to Claim 8, wherein the polypeptide region responsible for the enzyme activity consists of a polypeptide region from a 150th glutamine to a 411th leucine of a human prourokinase polypeptide calculated from an N-terminal amino acid serine as the first amino acid, wherein the 157th

EP 0 231 883 B1

phenylalanine is optionally replaced by an acidic amino acid.

10. A process according to Claim 8, wherein the polypeptide region responsible for the enzyme activity consists of a polypeptide region from a 132th alanine to a 411th leucine of human prourokinase calculated from an N-terminal amino acid serine as the first amino acid, wherein the 157th phenylalanine is optionally replaced by an acidic amino acid, and/or the 135th amino acid lysine is optionally replaced by an amino acid other than a basic amino acid.

11. A process according to Claim 9 or 10, wherein the acidic amino acid is aspartic acid, and the amino acid other than a basic amino acid is glutamine.

12. A process for preparing a DNA segment coding for the hybrid plasminogen activator-like polypeptide of Claim 1, wherein the DNA portion coding for the polypeptide region responsible for the affinity to fibrin is present at the 5'-terminal side and the DNA portion coding for the polypeptide region responsible for the enzyme activity is present at the 3'-terminal side in the DNA segment, which process comprises:
    (1) preparing a gene coding for a tissue plasminogen activator, and obtaining a DNA fragment coding for a polypeptide region having an affinity to fibrin;
    (2) preparing a gene coding for prourokinase, and obtaining a DNA fragment coding for a polypeptide region having enzyme activity;
    (3) joining together the DNA fragments obtained in steps (1) and (2).

13. A process according to Claim 12, wherein the DNA region coding for the polypeptide region responsible for the affinity to fibrin is a corresponding part of cDNA corresponding to mRNA derived from cells of a human pharyngeal cancer cell line Detroit 562.

14. A process according to Claim 12, wherein the DNA region coding for the polypeptide region responsible for the enzyme activity is a corresponding part of cDNA corresponding to mRNA derived from human kidney tissue cells.

15. A process according to Claim 14, wherein a codon coding for the 157th phenylalanine is mutated to codon coding for an acidic amino acid, and/or a codon coding for 135th lysine is mutated to a codon coding for an amino acid other than a basic amino acid.

16. A process for preparing a plasmid containing the DNA segment of Claim 12 and capable of expression of the polypeptide, which process comprises inserting the DNA segment in a plasmid containing an expression control region.

17. A process according to Claim 16, wherein the plasmid contains a tac promoter/operator and the Shine-Dalgarno sequence of the methapyrocatechase gene derived from Pseudomonas putida.

18. A process according to Claim 17, wherein the plasmid is a plasmid pHA00, deposited under DSM3628, pHA03, deposited under DSM3627, pHA20, deposited under DSM3626, pHA23, deposited under DSM3625, pHA21L, deposited under DSM3951, pHA24L, deposited under DSM3952;
    pHA13 which contains a gene coding for a hybrid plasminogen activator-like polypeptide comprising as its N-terminal half, a polypeptide region from 128th serine to 219th glycine of human tissue plasminogen activator calculated from N-terminal serine as the first amino acid, and as its C-terminal half, a polypeptide region from 150th glutamine to 411th C-terminal leucine of human prourokinase calculated from N-terminal serine as the first amino acid wherein 157th phenylalanine is replaced by aspartic acid, under the control of the tac promotor/operator and the Shine-Dalgarno sequence of the methapyrocatechase gene derived from Pseudomonas putida, and can express the above-mentioned hybrid polypeptide, and which has the structure shown in Fig. 16;
    pHA11L which contains a gene coding for a hybrid plasminogen activator-like polypeptide comprising as its N-terminal half, a polypeptide region from 128th serine to 215th cysteine of human tissue plasminogen activator and as its C-terminal half, a polypeptide region from 132th alanine to 411th C-terminal leucine of human prourokinase calculated from N-terminal serine as the first amino acid wherein 135th lysine is replaced by glutamine, under the control of the tac promotor and the Shine-Dalgarno sequence of the methapyrocatechase gene derived from Pseudomonas putida, and can express the above-mentioned hybrid polypeptide, and which has the structure shown in Fig. 21;

30

pHA01L contains a gene coding for a hybrid plasminogen activator-like polypeptide comprising as its N-terminal half, a polypeptide region from 161th methionine to 215th cysteine of human tissue plasminogen activator calculated from N-terminal serine as the first amino acid, and as its C-terminal half, a polypeptide region from 132th alanine to 411th N-terminal leucine of human prourokinase calculated from N-terminal serine as the first amino acid wherein 135th lysine is replaced by glutamine, under the control of the tac promotor/operator and the Shine-Dalgarno sequence of the methapyrocatechase gene derived from Pseudomonas putida, and can express the above-mentioned hybrid polypeptide, and which has the structure shown in Fig. 22;

pHA14L which is identical with the plasmid pHA11L except that the codon for 157th phenylalanine is substituted by the codon for aspartic acid; or

pHA04L which is identical with the plasmid pHA01L except that the codon for the 157th phenylalanine is substituted by the codon for aspartic acid.

19. A process for preparing Escherichia coli transformed with the plasmid of Claim 16 by introducing the plasmid into the micro-organism in a manner known per se.

20. A process according to Claim 1, wherein the cultured host is E. coli.

**Patentansprüche**

**Patentansprüche für folgende Vertragsstaaten : BE, CH, DE, FR, GB, IT, NL, SE**

1. Ein hybrides Plasminogenaktivator-ähnliches Polypeptid umfassend eine für die Affinität zu Fibrin verantwortliche Polypeptidregion, die vom Gewebsplaminogenaktivatorpolypeptid stammt, verknüpft durch eine Peptidbindung mit einer für eine Enzymaktivität verantwortlichen Polypeptidregion, die vom Prourokinasepolypeptid stammt, worin das hybride Plasminogenaktivator-ähnliche Polypeptid eine Affinität zu Fibrin und eine Enzymaktivität nach der Aktivierung besitzt.

2. Ein hybrides Plasminogenaktivator-ähnliches Polypeptid gemäß Anspruch 1, worin die für die Affinität zu Fibrin verantwortliche Polypeptidregion aus ungefähr zwei Domänen einer N-terminalen Region eines menschlichen Gewebsplasminogenaktivatorpolypeptids besteht.

3. Ein hybrides Plasminogenaktivator-ähnliches Polypeptid gemäß Anspruch 2, worin die ungefähr zwei Domänen eine Polypeptidregion von einem N-terminalen ersten Serin bis zu einem Cystein in Position 215 oder Glycin in Position 217 eines menschlichen Gewebsplasminogenaktivatorpolypeptids, berechnet von der ersten Aminosäure, darstellt.

4. Ein hybrides Plasminogenaktivator-ähnliches Polypeptid gemäß Anspruch 1, worin die für die Affinität zu Fibrin verantwortliche Polypeptidregion aus ungefähr einer Domäne eines menschlichen Gewebsplasminogenaktivatorpolypeptids besteht.

5. Ein hybrides Plasminogenaktivator-ähnliches Polypeptid gemäß Anspruch 4, worin die ungefähr eine Domäne eine Polypeptidregion von dem Serin in Positon 128 bis zu einem Cystein in Position 215 oder Glycin in Position 219 eines menschlichen Gewebsplasminogenaktivatorpolypeptids, berechnet von der N-terminalen Aminosäure Serin als der ersten Aminosäure, darstellt.

6. Ein hybrides Plasminogenaktivator-ähnliches Polypeptid gemäß Anspruch 1, worin die für die Affinität zu Fibrin verantwortliche Polypeptidregion aus ungefähr einer halben Domäne eines menschlichen Gewebsplasminogenaktivatorpolypeptids besteht.

7. Ein hybrides Plasminogenaktivator-ähnliches Polypeptid gemäß Anspruch 6, worin die ungefähr halbe Domäne ein Polypeptid von einem Methionin in Position 161 bis zu einem Cystein in Position 215 oder Glycin in Position 219 eines menschlichen Gewebsplasminogenaktivatorpolypeptids, berechnet von der N-terminalen Aminosäure Serin als der ersten Aminosäure, darstellt.

8. Ein hybrides Plasminogenaktivator-ähnliches Polypeptid gemäß Anspruch 1, worin die für die Enzymaktivität verantwortliche Polypeptidregion aus einer C-terminalen Region mit Enzymaktivität eines menschlichen Prourokinasepolypeptids besteht.

9. Ein hybrides Plasminogenaktivator-ähnliches Polypeptid gemäß Anspruch 8, worin die für die Enzymaktivität verantwortliche Polypeptidregion aus einer Polypeptidregion von einem Glutamin in Position 150 bis zu einem Leucin in Position 411 eines menschlichen Prourokinasepolypeptids, berechnet von einer N-terminalen Aminosäure Serin als der ersten Aminosäure, besteht, worin das Phenylalanin in Position 157 gegebenenfalls durch eine saure Aminosäure ersetzt ist.

10. Ein hybrides Plasminogenaktivator-ähnliches Polypeptid gemäß Anspruch 8, worin die für die Enzymaktivität verantwortliche Polypeptidregion aus einer Polypeptidregion von einem Alanin in Position 132 bis zu einem Leucein in Position 411 einer menschlichen Prourokinase, berechnet von einer ersten N-terminalen Aminosäure Serin als der ersten Aminosäure, besteht, worin das Phenylalanin in Position 157 gegebenenfalls durch eine saure Aminosäure ersetzt ist und/oder die Aminosäure Lysin in Position 135 gegebenenfalls durch eine andere als ein basische Aminosäure ersetzt ist.

11. Ein hybrides Plasminogenaktivator-ähnliches Polypeptid gemäß Anspruch 9 oder 10, worin die saure Aminosäure Aparaginsäure ist, und die andere als die basische Aminosäure Glutamin ist.

12. Ein DNA-Segment, das für das hybride Plasminogenaktivator-ähnliche Polypeptid von Anspruch 1 kodiert, worin der DNA-Abschnitt, der die Polypeptidregion kodiert, die für die Affinität zu Fibrin verantwortlich ist, an der 5'-terminalen Seite vorhanden ist, und der DNA-Abschnitt, der für die Polypeptidregion kodiert, die für die Enzymaktivität verantwortlich ist, ist an der 3'-terminalen Seite des DNA-Segments vorhanden.

13. Ein DNA-Segment gemäß Anspruch 12, worin die DNA-Region, die für die Polypeptidregion kodiert, die für die Affinität zu Fibrin verantwortlich ist, ein entsprechender Abschnitt von cDNA ist, der mRNA entspricht, die von Zellen einer menschlichen pharyngealen Krebszellinie Detroit 562 stammt.

14. Ein DNA-Segment gemäß Anspruch 12, worin die DNA-Region, die für die Polypeptidregion kodiert, die für die Enzymaktivität verantwortlich ist, ein entsprechender Abschnitt von cDNA darstellt, die mRNA entspricht, die von menschlichen Nierengewebszellen stammt.

15. Ein DNA-Segment gemäß Anspruch 14, worin ein Codon, das für Phenylalanin in Position 157 kodiert, zu einem Codon mutiert wird, das für eine saure Aminosäure kodiert, und/oder ein Codon, das für Lysin in Position 135 kodiert, ist zu einem Codon mutiert, das für eine andere Aminosäure als eine basische Aminosäure kodiert.

16. Ein Plasmid welches das DNA-Segment nach Anspruch 12 enthält und fähig ist, das Polypeptid zu exprimieren.

17. Ein Plasmid gemäß Anspruch 16, enthaltend einen TacPromotor/Operator und die Shine-Dalgarno-Sequenz des von Pseudomonas putida stammenden Methapyrocatechasegens.

18. Ein Plasmid gemäß Anspruch 17, das ein Plasmid darstellt, pHA00 hinterlegt unter DSM3628, pHA03, hinterlegt unter DSM3627, pHA20, hinterlegt unter DSM3626, pHA23, hinterlegt unter DSM3625, pHA21L, hinterlegt unter DSM3951, pHA24L, hinterlegt unter DSM3952;
pHA13, das ein Gen enthält, das für ein hybrides Plsminogenaktivator-ähnliches Polypeptid kodiert, das als seine N-terminale Hälfte eine Polypeptidregion von Serin in Position 128 bis Glycin in Position 219 von menschlichem Gewebsplaminogenaktivator, berechnet vom N-terminalen Serin als der ersten Aminosäure, und als seine C-terminale Hälfte eine Polypeptidregion von Glutamin in Position 150 bis zu dem C-terminalen Leucin in Position 411 von menschlicher Prourokinase, berechnet vom N-terminalen Serin als der ersten Aminosäure, enthält, worin Phenylalanin in Position 157 durch Asparaginsäure ersetzt ist, unter der Kontrolle des Tac-Promotors/Operators und der Shine-Dalgarno-Sequenz des von Pseudomonas putida stammenden Methapyrocatechasegens, und das das oben erwähnte hybride Polypeptid exprimieren kann und das die in Figur 16 gezeigte Struktur besitzt;
pHA11L, das ein Gen enthält, das für ein hybrides Plsminogenaktivator-ähnliches Polypeptid kodiert, das als seine N-terminale Hälfte eine Polypeptidregion von Serin in Position 128 bis zu Cystein in Position 215 eines menschlichen Gewebsplaminogenaktivators, und als seine C-terminale Hälfte eine Polypeptidregion von Alanin in Postion 132 bis zu dem C-terminalen Leucin in Position 411 von menschlicher Prourokinase, berechnet vom N-terminalen Serin als der ersten Aminosäure, enthält, worin Lysin in Position 135 durch Glutamin ersetzt ist, unter der Kontrolle des Tac-Promotors und der Shine-Dalgarno-Sequenz des von Pseudomonas putida stammenden Methapyrocatechasegens, und das das oben erwähnte hybride Polypeptid exprimieren kann und das die in Figur 21 gezeigte Struktur besitzt;

pHA01L, das ein Gen enthält, das für ein hybrides Plsminogenaktivator-ähnliches Polypeptid kodiert, das als seine N-terminale Hälfte eine Polypeptidregion von Methionin in Position 161 bis Cystein in Position 215 von menschlichem Gewebsplaminogenaktivator, berechnet vom N-terminalen Serin als der ersten Aminosäure, und als seine C-terminale Hälfte eine Polypeptidregion von Alanin in Position 132 bis zu dem N-terminalen Leucin in Position 411 von menschlicher Prourokinase, berechnet vom N-terminalen Serin als der ersten Aminosäure, enthält, worin Lysin in Position 135 durch Glutamin ersetzt ist, unter der Kontrolle des Tac-Promotors/Operators und der Shine-Dalgarno-Sequenz des von Pseudomonas putida stammenden Methapyrocatechasegens, und das das oben erwähnte hybride Polypeptid exprimieren kann und das die in Figur 22 gezeigte Struktur besitzt;

pHA14L, das identisch mit Plasmid pHA11L ist, außer daß das Codon für Phenylalanin in Positon 157 durch ein Codon für Asparaginsäure ersetzt ist,

pHA04L, das identisch mit Plasmid pHA01L ist, außer daß das Codon für Phenylalanin in Position 157 durch ein Codon für Asparaginsäure ersetzt ist.

19. Escherichia coli, transformiert mit dem Plasmid von Anspruch 16.

20. Ein Verfahren zum Herstellen des hybriden Plasminongeaktivator-ähnlichen Polypeptids von Anspruch 1, umfassend Kultivieren des E.Coli von Anspruch 19 und Gewinnen des Polypeptids von einem kultivierten Produkt.

**Patentansprüche für folgenden Vertragsstaat : ES**

1. Ein Verfahren zum Herstellen eines hybriden Plasminogenaktivator-ähnlichen Polypeptids umfassend eine für die Affinität zu Fibrin verantwortliche Polypeptidregion, die vom Gewebsplaminogenaktivatorpolypeptid stammt, verknüpft durch eine Peptidbindung mit einer für eine Enzymaktivität verantwortlichen Polypeptidregion, die vom Prourokinasepolypeptid stammt, worin das hybride Plasminogenaktivator-ähnliche Polypeptid eine Affinität zu Fibrin und eine Enzymaktivität nach der Aktivierung besitzt, wobei das Verfahren umfaßt:
   Kultivieren eines Wirts, der mit einem Plasmid transformiert ist, das ein für das Polypeptid kodierendes DNA-Segment enthält, und Gewinnen des Polypeptids aus einem kultivierten Produkt.

2. Ein Verfahren gemäß Anspruch 1, worin die für die Affinität zu Fibrin verantwortliche Polypeptidregion aus ungefähr zwei Domänen einer N-terminalen Region eines menschlichen Gewebsplasminogenaktivatorpolypeptids besteht.

3. Ein Verfahren gemäß gemäß Anspruch 2, worin die ungefähr zwei Domänen eine Polypeptidregion von einem N-terminalen ersten Serin bis zu einem Cystein in Position 215 oder Glycin in Position 217 eines menschlichen Gewebsplasminogenaktivatorpolypeptids, berechnet von der ersten Aminosäure, darstellt.

4. Ein Verfahren gemäß Anspruch 1, worin die für die Affinität zu Fibrin verantwortliche Polypeptidregion aus ungefähr einer Domäne eines menschlichen Gewebsplasminogenaktivatorpolypeptids besteht.

5. Ein Verfahren gemäß Anspruch 4, worin die ungefähr eine Domäne eine Polypeptidregion von dem Serin in Positon 128 bis zu einem Cystein in Position 215 oder Glycin in Position 219 eines menschlichen Gewebsplasminogenaktivatorpolypeptids, berechnet von der N-terminalen Aminosäure Serin als der ersten Aminosäure, darstellt.

6. Ein Verfahren gemäß Anspruch 1, worin die für die Affinität zu Fibrin verantwortliche Polypeptidregion aus ungefähr einer halben Domäne eines menschlichen Gewebsplasminogenaktivatorpolypeptids besteht.

7. Ein Verfahren gemäß Anspruch 6, worin die ungefähr halbe Domäne ein Polypeptid von einem Methionin in Position 161 bis zu einem Cystein in Position 215 oder Glycin in Position 219 eines menschlichen Gewebsplasminogenaktivatorpolypeptids, berechnet von der N-terminalen Aminosäure Serin als der ersten Aminosäure, darstellt.

**8.** Ein Verfahren gemäß Anspruch 1, worin die für die Enzymaktivität verantwortliche Polypeptidregion aus einer C-terminalen Region mit Enzymaktivität eines menschlichen Prourokinasepolypeptids besteht.

**9.** Ein Verfahren gemäß Anspruch 8, worin die für die Enzymaktivität verantwortliche Polypeptidregion aus einer Polypeptidregion von einem Glutamin in Position 150 bis zu einem Leucin in Position 411 eines menschlichen Prourokinasepolypeptids, berechnet von einer N-terminalen Aminosäure Serin als der ersten Aminosäure, besteht, worin das Phenylalanin in Position 157 gegebenenfalls durch eine saure Aminosäure ersetzt ist.

**10.** Ein Verfahren gemäß Anspruch 8, worin die für die Enzymaktivität verantwortliche Polypeptidregion aus einer Polypeptidregion von einem Alanin in Position 132 bis zu einem Leucein in Position 411 einer menschlichen Prourokinase, berechnet von einer ersten N-terminalen Aminosäure Serin als der ersten Aminosäure, besteht, worin das Phenylalanin in Position 157 gegebenenfalls durch eine saure Aminosäure ersetzt ist und/oder die Aminosäure Lysin in Position 135 gegebenenfalls durch eine andere als ein basische Aminosäure ersetzt ist.

**11.** Ein Verfahren gemäß Anspruch 9 oder 10, worin die saure Aminosäure Aparaginsäure ist, und die andere als die basische Aminosäure Glutamin ist.

**12.** Ein Verfahren zum Herstellen eines DNA-Segments, das für das hybride Plasminogenaktivator-ähnliche Polypeptid von Anspruch 1 kodiert, worin der DNA-Abschnitt, der die Polypeptidregion kodiert, die für die Affinität zu Fibrin verantwortlich ist, an der 5'-terminalen Seite vorhanden ist, und der DNA-Abschnitt, der für die Polypeptidregion kodiert, die für die Enzymaktivität verantwortlich ist, ist an der 3'-terminalen Seite des DNA-Segments vorhanden, wobei das Verfahren umfaßt:
(1) Herstellen eines Gens, das für einen Gewebsplasminogenaktivator kodiert, und Erhalten eines DNA-Fragments, das für eine Polypeptidregion mit einer Affinität zu Fibrin kodiert;
(2) Herstellen eines Gens, das für Prourokinase kodiert, und Erhalten eines DNA-Fragments, das für eine Polypeptidregion mit Enzymaktivität kodiert;
(3) Verknüpfen der beiden in Schritten (1) und (2) erhaltenen DNA-Fragmente.

**13.** Ein Verfahren gemäß Anspruch 12, worin die DNA-Region, die für die Polypeptidregion kodiert, die für die Affinität zu Fibrin verantwortlich ist, ein entsprechender Abschnitt von cDNA ist, der mRNA entspricht, die von Zellen einer menschlichen pharyngealen Krebszellinie Detroit 562 stammt.

**14.** Ein Verfahren gemäß Anspruch 12, worin die DNA-Region, die für die Polypeptidregion kodiert, die für die Enzymaktivität verantwortlich ist, ein entsprechender Abschnitt von cDNA darstellt, die mRNA entspricht, die von menschlichen Nierengewebszellen stammt.

**15.** Ein Verfahren gemäß Anspruch 14, worin ein Codon, das für Phenylalanin in Position 157 kodiert, zu einem Codon mutiert wird, das für eine saure Aminosäure kodiert, und/oder ein Codon, das für Lysin in Position 135 kodiert, ist zu einem Codon mutiert, das für eine andere Aminosäure als eine basische Aminosäure kodiert.

**16.** Ein Verfahren zum Herstellen eines Plasmids enthaltend das DNA-Segment von Anspruch 12 und das das Polypeptid exprimieren kann, wobei das Verfahren umfaßt Einfügen des DNA-Segments in ein Plasmid enthaltend eine Kontrollregion zur Expression.

**17.** Ein Verfahren gemäß Anspruch 16, worin das Plasmid einen Tac-Promotor/Operator und die Shine-Dalgarno-Sequenz des von Pseudomonas putida stammenden Methapyrocatechasegens enthält.

**18.** Ein Verfahren gemäß Anspruch 17, worin das Plasmid darstellt ein Plasmid pHA00, hinterlegt unter DSM3628, pHA03, hinterlegt unter DSM3627, pHA20, hinterlegt unter DSM3626, pHA23, hinterlegt unter DSM3625, pHA21L, hinterlegt unter DSM3951, pHA24L, hinterlegt unter DSM3952;
pHA13, das ein Gen enthält, das für ein hybrides Plsminogenaktivator-ähnliches Polypeptid kodiert, das als seine N-terminale Hälfte eine Polypeptidregion von Serin in Position 128 bis Glycin in Position 219 von menschlichem Gewebsplaminogenaktivator, berechnet vom N-terminalen Serin als der ersten Aminosäure, und als seine C-terminale Hälfte eine Polypeptidregion von Glutamin in Position 150 bis zu dem C-terminalen Leucin in Position 411 von menschlicher Prourokinase, berechnet vom N-terminalen Serin als der ersten Aminosäure, enthält, worin Phenylalanin in Position 157 durch Asparaginsäure ersetzt ist, unter der Kontrolle des Tac-Promotors/Operators und der Shine-Dalgarno-Sequenz des von Pseudomonas putida stammenden Methapyrocatechasegens, und das das oben erwähnte hybride Polypeptid exprimieren kann und das die in Figur 16 gezeigte Struktur besitzt;

pHA11L, das ein Gen enthält, das für ein hybrides Plsminogenaktivator-ähnliches Polypeptid kodiert, das als seine N-terminale Hälfte eine Polypeptidregion von Serin in Position 128 bis zu Cystein in Position 215 eines menschlichen Gewebsplaminogenaktivators, und als seine C-terminale Hälfte eine Polypeptidregion von Alanin in Postion 132 bis zu dem C-terminalen Leucin in Position 411 von menschlicher Prourokinase, berechnet vom N-terminalen Serin als der ersten Aminosäure, enthält, worin Lysin in Position 135 durch Glutamin ersetzt ist, unter der Kontrolle des Tac-Promotors und der Shine-Dalgarno-Sequenz des von Pseudomonas putida stammenden Methapyrocatechasegens, und das das oben erwähnte hybride Polypeptid exprimieren kann und das die in Figur 21 gezeigte Struktur besitzt;

pHA01L, das ein Gen enthält, das für ein hybrides Plsminogenaktivator-ähnliches Polypeptid kodiert, das als seine N-terminale Hälfte eine Polypeptidregion von Methionin in Position 161 bis Cystein in Position 215 von menschlichem Gewebsplaminogenaktivator, berechnet vom N-terminalen Serin als der ersten Aminosäure, und als seine C-terminale Hälfte eine Polypeptidregion von Alanin in Position 132 bis zu dem N-terminalen Leucin in Position 411 von menschlicher Prourokinase, berechnet vom N-terminalen Serin als der ersten Aminosäure, enthält, worin Lysin in Position 135 durch Glutamin ersetzt ist, unter der Kontrolle des Tac-Promotors/Operators und der Shine-Dalgarno-Sequenz des von Pseudomonas putida stammenden Methapyrocatechasegens, und das das oben erwähnte hybride Polypeptid exprimieren kann und das die in Figur 22 gezeigte Struktur besitzt;

pHA14L, das identisch mit Plasmid pHA11L ist, außer daß das Codon für Phenylalanin in Positon 157 durch ein Codon für Asparaginsäure ersetzt ist,

pHA04L, das identisch mit Plasmid pHA01L ist, außer daß das Codon für Phenylalanin in Position 157 durch ein Codon für Asparaginsäure ersetzt ist.

19. Ein Verfahren zum Herstellen von Escherichia coli, transformiert mit dem Plasmid von Anspruch 16, durch Einbringen des Plasmids in den Mikroorganismus in bekannter Weise.

20. Ein Verfahren gemäß Anspruch 1, worin der kultivierte Wirt E.Coli ist.

**Revendications**
**Revendications pour les Etats contractants suivants : BE, CH, DE, FR, GB, IT, NL, SE**

1. Un polypeptide hybride de type activateur de plasminogène comprenant une région polypeptidique responsable d'une affinité pour la fibrine dérivée d'un polypeptide d'activateur de plasminogène de tissu réunie par une liaison peptide à une région polypeptidique responsable d'une activité enzymatique dérivée d'un polypeptide de prourokinase, dans lequel le polypeptide hybride de type activateur de plasminogène présente à la fois une affinité pour la fibrine et une activité enzymatique après activation.

2. Un polypeptide hybride de type activateur de plasminogène suivant la revendication 1, dans lequel la région polypeptidique responsable d'une affinité pour la fibrine consiste en environ deux kringles d'une région N-terminale d'un polypeptide d'activateur de plasminogène de tissu humain.

3. Un polypeptide hybride de type activateur de plasminogène suivant la revendication 2, dans lequel les deux kringles environ constituent une région polypeptidique allant d'une première sérine N-terminale jusqu'à la 215ème cystéine ou la 219ème glycine d'un polypeptide d'activateur de plasminogène de tissu humain, en comptant à partir du premier acide aminé.

4. Un polypeptide hybride de type activateur de plasminogène suivant la revendication 1, dans lequel la région polypeptidique responsable d'une affinité pour la fibrine consiste en environ un kringle d'un polypeptide d'activateur de plasminogène de tissu humain.

5. Un polypeptide hybride de type activateur de plasminogène suivant la revendication 4, dans lequel le kringle environ est une région polypeptidique allant de la 128ème sérine jusqu'à la 215ème cystéine ou la 219ème glycine d'un polypeptide d'activateur de plasminogène de tissu humain, en comptant à partir de l'acide aminé sérine N-terminal en tant que premier acide aminé.

6. Un polypeptide hybride de type activateur de plasminogène suivant la revendication 1, dans lequel la région polypeptidique responsable d'une affinité pour la fibrine consiste en environ la moitié d'un kringle d'un polypeptide d'activateur de plasminogène de tissu humain.

7. Un polypeptide hybride de type activateur de plasminogène suivant la revendication 6, dans lequel environ la moitié d'un kringle est un polypeptide allant de la 161ème méthionine jusqu'à la 215ème cystéine ou la 219ème glycine d'un polypeptide d'activateur de plasminogène de tissu humain, en comptant à partir de l'acide aminé sérine N-terminal en tant que premier acide aminé.

8. Un polypeptide hybride de type activateur de plasminogène suivant la revendication 1, dans lequel la région polypeptidique responsable d'une activité enzymatique consiste en une région d'activité enzymatique C-terminale d'un polypeptide de prourokinase humaine.

9. Un polypeptide hybride de type activateur de plasminogène suivant la revendication 8, dans lequel la région polypeptidique responsable de l'activité enzymatique consiste en une région polypeptidique allant d'une 150ème glutamine jusqu'à une 411ème leucine d'un polypeptide de prourokinase humaine, en comptant à partir d'un acide aminé sérine N-terminal en tant que premier acide aminé, dans lequel la 157ème phénylalanine est éventuellement remplacée par un acide aminé acide.

10. Un polypeptide hybride de type activateur de plasminogène suivant la revendication 8, dans lequel la région polypeptidique responsable de l'activité enzymatique consiste en une région polypeptidique allant d'une 132ème alanine jusqu'à une 411ème leucine d'un polypeptide de prourokinase humaine, en comptant à partir d'un acide aminé sérine M-terminal en tant que premier acide aminé, dans lequel la 157ème phénylalanine est éventuellement remplacée par un acide aminé acide, et/ou le 135ème acide aminé lysine est éventuellement remplacé par un acide aminé autre qu'un acide aminé basique.

11. Un polypeptide hybride de type activateur de plasminogène suivant les revendications 9 ou 10, dans lequel l'acide aminé acide est l'acide aspartique, et l'acide aminé autre qu'un acide aminé basique est la glutamine.

12. Un segment d'ADN codant pour le polypeptide hybride de type activateur de plasminogène suivant la revendication 1, dans lequel la portion d'ADN codant pour la région polypeptidique responsable de l'affinité pour la fibrine est présente du côté 5'-terminal et la portion d'ADN codant pour la région polypeptidique responsable de l'activité enzymatique est présente du côté 3'-terminal dans le segment d'ADN.

13. Un segment d'ADN suivant la revendication 12, dans lequel la région d'ADN codant pour la région polypeptidique responsable de l'affinité pour la fibrine est une partie correspondante d'ADNc correspondant à l'ARNm dérivé de cellules d'une lignée cellulaire Detroit 562 de cancer du pharynx humain.

14. Un segment d'ADN suivant la revendication 12, dans lequel la région d'ADN codant pour la région polypeptidique responsable de l'activité enzymatique est une partie correspondante d'ADNc correspondant à l'ARNm dérivé de cellules de tissu de rein humain.

15. Un segment d'ADN suivant la revendication 14, dans lequel un codon codant pour la 157ème phénylalanine est muté en un codon codant pour un acide aminé acide, et/ou un codon codant pour la 135ème lysine est muté en un codon codant pour un acide aminé autre qu'un acide aminé basique.

16. Un plasmide contenant le segment d'ADN suivant la revendication 12 et capable d'exprimer le polypeptide.

17. Un plasmide suivant la revendication 16, contenant un promoteur/opérateur tac et la séquence de Shine-Dalgarno du gène de la méthapyrocatéchase dérivé de Pseudomonas putida.

**18.** Un plasmide suivant la revendication 17, qui est un plasmide pHAOO, déposé sous le numéro DSM3628, pHAO3, déposé sous le numéro DSM3627, pHA20, déposé sous le numéro DSM3626, pHA23, déposé sous le numéro DSM3625, pHA21L, déposé sous le numéro DSM3951, pHA24L, déposé sous le numéro DSM3952;

pHA13 qui contient un gène codant pour un polypeptide hybride de type activateur de plasminogène comprenant en tant que moitié N-terminale, une région polypeptidique allant de la 128ème sérine à la 219ème glycine d'un activateur de plasminogène de tissu humain en comptant à partir d'une sérine N-terminale en tant que premier acide aminé, et en tant que moitié C-terminale, une région polypeptidique allant de la 150ème glutamine jusqu'à la 411ème leucine C-terminale de prourokinase humaine en comptant à partir d'une sérine N-terminale en tant que premier acide aminé, dans laquelle la 157ème phénylalanine est remplacée par l'acide aspartique, sous le contrôle du promoteur/opérateur tac et de la séquence de Shine-Dalgarno du gène de la méthapyrocatéchase dérivé de Pseudomonas putida, et peut exprimer le polypeptide hybride mentionné plus haut, et qui a la structure représentée dans la figure 16;

pHA11L qui contient un gène codant pour un polypeptide hybride de type activateur de plasminogène comprenant en tant que moitié N-terminale, une région polypeptidique allant de la 128ème sérine à la 215ème cystéine d'un activateur de plasminogène de tissu humain, et en tant que moitié C-terminale, une région polypeptidique allant de la 132ème alanine jusqu'à la 411ème leucine C-terminale de prourokinase humaine, en comptant à partir d'une sérine N-terminale en tant que premier acide aminé, dans laquelle la 135ème lysine est remplacée par la glutamine, sous le contrôle du promoteur tac et de la séquence de Shine-Dalgarno du gène de la méthapyrocatéchase dérivé de Pseudomonas putida, et peut exprimer le polypeptide hybride mentionné plus haut, et qui a la structure représentée dans la figure 21;

pHA01L qui contient un gène codant pour un polypeptide hybride de type activateur de plasminogène comprenant en tant que moitié N-terminale, une région polypeptidique allant de la 161ème méthionine à la 215ème cystéine d'un activateur de plasminogène de tissu humain en comptant à partir d'une sérine N-terminale en tant que premier acide aminé, et en tant que moitié C-terminale, une région polypeptidique allant de la 132ème alanine à la 411ème leucine N-terminale de prourokinase humaine en comptant à partir d'une sérine N-terminale en tant que premier acide aminé, dans laquelle la 135ème lysine est remplacée par la glutamine, sous le contrôle du promoteur/opérateur tac et de la séquence de Shine-Dalgarno du gène de la méthapyrocatéchase dérivé de Pseudomonas putida, et peut exprimer le polypeptide hybride mentionné plus haut, et qui a la structure représentée dans la figure 22;

pHA14L qui est identique au plasmide pHA11L sauf que le codon pour la 157ème phénylalanine est remplacé par le codon pour l'acide aspartique; ou

pHA04L qui est identique au plasmide pHA01L sauf que le codon pour la 157ème phénylalanine est remplacé par le codon pour l'acide aspartique.

**19.** Escherichia coli transformé avec le plasmide suivant la revendication 16.

**20.** Un procédé pour la production du polypeptide hybride de type activateur de plasminogène suivant la revendication 1, comprenant la culture de E. coli suivant la revendication 19 et la récupération du polypeptide à partir du produit cultivé.

**Revendications pour l'Etat contractant suivant : ES**

**1.** Un procédé pour la production d'un polypeptide hybride de type activateur de plasminogène comprenant une région polypeptidique responsable d'une affinité pour la fibrine dérivée d'un polypeptide d'activateur de plasminogène de tissu réunie par une liaison peptide à une région polypeptidique responsable d'une activité enzymatique dérivée d'un polypeptide de prourokinase, dans lequel le polypeptide hybride de type activateur de plasminogène présente à la fois une affinité pour la fibrine et une activité enzymatique après activation, lequel procédé comprend la culture d'un hôte transformé avec un plasmide contenant un segment d'ADN codant pour le polypeptide et la récupération du polypeptide à partir du produit cultivé.

**2.** Un procédé suivant la revendication 1, dans lequel la région polypeptidique responsable d'une affinité pour la fibrine consiste en environ deux kringles d'une région N-terminale d'un polypeptide d'activateur de plasminogène de tissu humain.

EP 0 231 883 B1

3. Un procédé suivant la revendication 2, dans lequel les deux kringles environ constituent une région polypeptidique allant d'une première sérine N-terminale jusqu'à la 215ème cystéine ou la 219ème glycine d'un polypeptide d'activateur de plasminogène de tissu humain, en comptant à partir du premier acide aminé.

4. Un procédé suivant la revendication 1, dans lequel la région polypeptidique responsable d'une affinité pour la fibrine consiste en environ un kringle d'un polypeptide d'activateur de plasminogène de tissu humain.

5. Un procédé suivant la revendication 4, dans lequel le kringle environ est une région polypeptidique allant de la 128ème sérine jusqu'à la 215ème cystéine ou la 219ème glycine d'un polypeptide d'activateur de plasminogène de tissu humain, en comptant à partir de l'acide aminé sérine N-terminal en tant que premier acide aminé.

6. Un procédé suivant la revendication 1, dans lequel la région polypeptidique responsable d'une affinité pour la fibrine consiste en environ la moitié d'un kringle d'un polypeptide d'activateur de plasminogène de tissu humain.

7. Un procédé suivant la revendication 6, dans lequel environ la moitié d'un kringle est un polypeptide allant de la 161ème méthionine jusqu'à la 215ème cystéine ou la 219ème glycine d'un polypeptide d'activateur de plasminogène de tissu humain, en comptant à partir de l'acide aminé sérine N-terminal en tant que premier acide aminé.

8. Un procédé suivant la revendication 1, dans lequel la région polypeptidique responsable d'une activité enzymatique consiste en une région d'activité enzymatique C-terminale d'un polypeptide de prourokinase humaine.

9. Un procédé suivant la revendication 8, dans lequel la région polypeptidique responsable de l'activité enzymatique consiste en une région polypeptidique allant d'une 150ème glutamine jusqu'à une 411ème leucine d'un polypeptide de prourokinase humaine, en comptant à partir d'un acide aminé sérine N-terminal en tant que premier acide aminé, dans lequel la 157ème phénylalanine est éventuellement remplacée par un acide aminé acide.

10. Un procédé suivant la revendication 8, dans lequel la région polypeptidique responsable de l'activité enzymatique consiste en une région polypeptidique allant d'une 132ème alanine jusqu'à une 411ème leucine d'un polypeptide de prourokinase humaine, en comptant à partir d'un acide aminé sérine N-terminal en tant que premier acide aminé, dans lequel la 157ème phénylalanine est éventuellement remplacée par un acide aminé acide, et/ou le 135ème acide aminé lysine est éventuellement remplacé par un acide aminé autre qu'un acide aminé basique.

11. Un procédé suivant les revendications 9 ou 10, dans lequel l'acide aminé acide est l'acide aspartique, et l'acide aminé autre qu'un acide aminé basique est la glutamine.

12. Un procédé pour la préparation d'un segment d'ADN codant pour le polypeptide hybride de type activateur de plasminogène suivant la revendication 1, dans lequel la portion d'ADN codant pour la région polypeptidique responsable de l'affinité pour la fibrine est présente du côté 5'-terminal et la portion d'ADN codant pour la région polypeptidique responsable de l'activité enzymatique est présente du côté 3'-terminal dans le segment d'ADN, lequel procédé comprend:
   (1) la préparation d'un gène codant pour un activateur de plasminogène de tissu et l'obtention d'un fragment d'ADN codant pour une région polypeptidique ayant une affinité pour la fibrine;
   (2) la préparation d'un gène codant pour la prourokinase et l'obtention d'un fragment d'ADN codant pour une région polypeptidique ayant une activité enzymatique;
   (3) la réunion des fragments d'ADN obtenus dans les étapes (1) et (2).

13. Un procédé suivant la revendication 12, dans lequel la région d'ADN codant pour la région polypeptidique responsable de l'affinité pour la fibrine est une partie correspondante d'ADNc correspondant à l'ARNm dérivé de cellules d'une lignée cellulaire Detroit 562 de cancer du pharynx humain.

38

**14.** Un procédé suivant la revendication 12, dans lequel la région d'ADN codant pour la région polypeptidique responsable de l'activité enzymatique est une partie correspondante d'ADNc correspondant à l'ARNm dérivé de cellules de tissu de rein humain.

**15.** Un procédé suivant la revendication 14, dans lequel un codon codant pour la 157ème phénylalanine est muté en un codon codant pour un acide aminé acide, et/ou un codon codant pour la 135ème lysine est muté en un codon codant pour un acide aminé autre qu'un acide aminé basique.

**16.** Un procédé pour la préparation d'un plasmide contenant le segment d'ADN suivant la revendication12 et capable d'exprimer le polypeptide, lequel procédé comprend l'insertion du segment d'ADN dans un plasmide contenant une région de contrôle d'expression.

**17.** Un procédé suivant la revendication 16, dans lequel le plasmide contient un promoteur/opérateur tac et la séquence de Shine-Dalgarno du gène de la méthapyrocatéchase dérivé de Pseudomonas putida.

**18.** Un procédé suivant la revendication 17, dans lequel le plasmide est un plasmide pHA00, déposé sous le numéro DSM3628, pHA03, déposé sous le numéro DSM3627, pHA20, déposé sous le numéro DSM3626, pHA23, déposé sous le numéro DSM3625, pHA21L, déposé sous le numéro DSM3951, pHA24L, déposé sous le numéro DSM3952;

pHA13 qui contient un gène codant pour un polypeptide hybride de type activateur de plasminogène comprenant en tant que moitié N-terminale, une région polypeptidique allant de la 128ème sérine à la 219ème glycine d'un activateur de plasminogène de tissu humain en comptant à partir d'une sérine N-terminale en tant que premier acide aminé, et en tant que moitié C-terminale, une région polypeptidique allant de la 150ème glutamine jusqu'à la 411ème leucine C-terminale de prourokinase humaine en comptant à partir d'une sérine N-terminale en tant que premier acide aminé, dans laquelle la 157ème phénylalanine est remplacée par l'acide aspartique, sous le contrôle du promoteur/opérateur tac et de la séquence de Shine-Dalgarno du gène de la méthapyrocatéchase dérivé de Pseudomonas putida, et peut exprimer le polypeptide hybride mentionné plus haut, et qui a la structure représentée dans la figure 16;

pHA11L qui contient un gène codant pour un polypeptide hybride de type activateur de plasminogène comprenant en tant que moitié N-terminale, une région polypeptidique allant de la 128ème sérine à la 215ème cystéine d'un activateur de plasminogène de tissu humain, et en tant que moitié C-terminale, une région polypeptidique allant de la 132ème alanine jusqu'à la 411ème leucine C-terminale de prourokinase humaine, en comptant à partir d'une sérine N-terminale en tant que premier acide aminé, dans laquelle la 135ème lysine est remplacée par la glutamine, sous le contrôle du promoteur tac et de la séquence de Shine-Dalgarno du gène de la méthapyrocatéchase dérivé de Pseudomonas putida, et peut exprimer le polypeptide hybride mentionné plus haut, et qui a la structure représentée dans la figure 21;

pHA01L qui contient un gène codant pour un polypeptide hybride de type activateur de plasminogène comprenant en tant que moitié N-terminale, une région polypeptidique allant de la 161ème méthionine à la 215ème cystéine d'un activateur de plasminogène de tissu humain en comptant à partir d'une sérine N-terminale en tant que premier acide aminé, et en tant que moitié C-terminale, une région polypeptidique allant de la 132ème alanine à la 411ème leucine N-terminale de prourokinase humaine en comptant à partir d'une sérine N-terminale en tant que premier acide aminé, dans laquelle la 135ème lysine est remplacée par la glutamine, sous le contrôle du promoteur/opérateur tac et de la séquence de Shine-Dalgarno du gène de la méthapyrocatéchase dérivé de Pseudomonas putida, et peut exprimer le polypeptide hybride mentionné plus haut, et qui a la structure représentée dans la figure 22;

pHA14L qui est identique au plasmide pHA11L sauf que le codon pour la 157ème phénylalanine est remplacé par le codon pour l'acide aspartique; ou

pHA04L qui est identique au plasmide pHA01L sauf que le codon pour la 157ème phénylalanine est remplacé par le codon pour l'acide aspartique.

**19.** Un procédé pour la préparation de Escherichia coli transformé avec le plasmide suivant la revendication 16 par introduction du plasmide dans le microorganisme d'une façon connue en soi.

**20.** Un procédé suivant la revendication 1, dans lequel l'hôte cultivé est E. coli.

# Fig 1-1

CCACCGACCCCCACCCCCTGCCTGGAAACTTAAAGGAGGCCGGAGCTGTGGGGAGCTCAGAGCTGAGATCCTACAGGAGT

CCAGGGCTGGAGAGAAAACCTCTGCGAGGAAAGGGAAGGAGCAAGCCGTGAATTTAAGGGACGCTGTGAAGCAATCATG

```
asp ala met lys arg gly leu cys cys val leu leu leu cys gly ala val phe val ser
GAT GCA ATG AAG AGA GGG CTC TGC TGT GTG CTG CTG CTG TGT GGA GCA GTC TTC GTT TCG

pro ser gln glu ile his ala arg phe arg arg gly ala arg SER TYR GLN GLY CYS SER
CCC AGC CAG GAA ATC CAT GCC CGA TTC AGA AGA GGA GCC AGA TCT TAC CAA GGT TGC AGC

GLU PRO ARG CYS PHE ASN GLY GLY THR CYS GLN GLN ALA LEU TYR PHE SER ASP PHE VAL
GAG CCA AGG TGT TTC AAC GGG GGC ACC TGC CAG CAG GCC CTG TAC TTC TCA GAT TTC GTG

CYS GLN CYS PRO GLU GLY PHE ALA GLY LYS CYS CYS GLU ILE ASP THR ARG ALA THR CYS
TGC CAG TGC CCC GAA GGA TTT GCT GGG AAG TGC TGT GAA ATA GAT ACC AGG GCC ACG TGC

TYR GLU ASP GLN GLY ILE SER TYR ARG GLY THR TRP SER THR ALA GLU SER GLY ALA GLU
TAC GAG GAC CAG GGC ATC AGC TAC AGG GGC ACG TGG AGC ACA GCG GAG AGT GGC GCC GAG
```

EP 0 231 883 B1

# Fig. 1-2

```
CYS THR ASN TRP ASN SER SER ALA LEU ALA GLN LYS PRO TYR SER GLY ARG ARG PRO ASP     86
TGC ACC AAC TGG AAC AGC AGC GCG TTG GCC CAG AAG CCC TAC AGT GGG CGG AGG CCA GAC
                                                    500

ALA ILE ARG LEU GLY LEU GLY ASN HIS ASN TYR CYS ARG ASN PRO ASP ARG ASP SER LYS    106
GCC ATC AGG CTG GGC CTG GGG AAC CAC AAC TAC TGC AGA AAC CCA GAT CGA GAC TCA AAG
                                            550

PRO TRP CYS TYR VAL PHE LYS ALA GLY LYS TYR SER SER GLU PHE CYS SER THR PRO ALA    126
CCC TGG TGC TAC GTC TTT AAG GCG GGG AAG TAC AGC TCA GAG TTC TGC AGC ACC CCT GCC
                            600

CYS SER GLU GLY ASN SER ASP CYS TYR PHE GLY ASN GLY SER ALA TYR ARG GLY THR HIS    146
TGC TCT GAG GGA AAC AGT GAC TGC TAC TTT GGG AAT GGG TCA GCC TAC CGT GGC ACG CAC
                650

SER LEU THR GLU SER GLY ALA SER CYS LEU PRO TRP ASN SER MET ILE LEU ILE GLY LYS    166
AGC CTC ACC GAG TCG GGT GCC TCC TGC CTC CCG TGG AAT TCC ATG ATC CTG ATA GGC AAG
700                                                                750

VAL TYR THR ALA GLN ASN PRO SER ALA GLN ALA LEU GLY LEU GLY LYS HIS ASN TYR CYS    186
GTT TAC ACA GCA CAG AAC CCC AGT GCC CAG GCA CTG GGC CTG GGC AAA CAT AAT TAC TGC .
                                                    800

ARG ASN PRO ASP GLY ASP ALA LYS PRO TRP CYS HIS VAL LEU LYS ASN ARG ARG LEU THR    206
CGG AAT CCT GAT GGG GAT GCC AAG CCC TGG TGC CAC GTG CTG AAG AAC CGC AGG CTG ACG
                                        850
```

EP 0 231 883 B1

## Fig. 1-3

```
                                                      ↓
TRP GLU TYR CYS ASP VAL PRO SER CYS SER THR CYS GLY LEU ARG GLN TYR SER GLN PRO    226
TGG GAG TAC TGT GAT GTG CCC TCC TGC TCC ACC TGC GGC CTG AGA CAG TAC AGC CAG CCT
                                900

GLN PHE ARG ILE LYS GLY GLY LEU PHE ALA ASP ILE ALA SER HIS PRO TRP GLN ALA ALA    246
CAG TTT CGC ATC AAA GGA GGG CTC TTC GCC GAC ATC GCC TCC CAC CCC TGG CAG GCT GCC
                950

ILE PHE ALA LYS HIS ARG ARG SER PRO GLY GLU ARG PHE LEU CYS GLY GLY ILE LEU ILE    266
ATC TTT GCC AAG CAC AGG AGG TCG CCC GGA GAG CGG TTC CTG TGC GGG GGC ATA CTC ATC
1000                                                                       1050

SER SER CYS TRP ILE LEU SER ALA ALA HIS CYS PHE GLN GLU ARG PHE PRO PRO HIS HIS    286
AGC TCC TGC TGG ATT CTC TCT GCC GCC CAC TGC TTC CAG GAG AGG TTT CCG CCC CAC CAC
                                            1100

LEU THR VAL ILE LEU GLY ARG THR TYR ARG VAL VAL PRO GLY GLU GLU GLU GLN LYS PHE    306
CTG ACG GTG ATC TTG GGC AGA ACA TAC CGG GTG GTC CCT GGC GAG GAG GAG CAG AAA TTT
                                        1150

GLU VAL GLU LYS TYR ILE VAL HIS LYS GLU PHE ASP ASP ASP THR TYR ASP ASN ASP ILE    326
GAA GTC GAA AAA TAC ATT GTC CAT AAG GAA TTC GAT GAT GAC ACT TAC GAC AAT GAC ATT
                                1200

ALA LEU LEU GLN LEU LYS SER ASP SER SER ARG CYS ALA GLN GLU SER SER VAL VAL ARG    346
GCG CTG CTG CAG CTG AAA TCG GAT TCG TCC CGC TGT GCC CAG GAG AGC AGC GTG GTC CGC
              1250
```

EP 0 231 883 B1

Fig. I-4

```
THR VAL CYS LEU PRO PRO GLU ASP LEU GLN LEU PRO ASP TRP THR GLU CYS GLU LEU SER   366
ACT GTG TGC CTT CCC CCG GAG GAC CTG CAG CTG CCG GAC TGG ACG GAG TGT GAG CTC TCC
1300                                                                 1350

GLY TYR GLY LYS HIS GLU ALA LEU SER PRO PHE TYR SER GLU ARG LEU LYS GLU ALA HIS   386
GGC TAC GGC AAG CAT GAG GCC TTG TCT CCT TTC TAT TCG GAG CGG CTG AAG GAG GCT CAT
                                                1400

VAL ARG LEU TYR PRO SER SER ARG CYS THR SER GLN HIS LEU LEU ASN ARG THR VAL THR   406
GTC AGA CTG TAC CCA TCC AGC CGC TGC ACA TCA CAA CAT TTA CTT AAC AGA ACA GTC ACC
                                        1450

ASP ASN MET LEU CYS ALA GLY ASP THR ARG SER GLY GLY PRO GLN ALA ASN LEU HIS ASP   426
GAC AAC ATG CTG TGT GCT GGA GAC ACT CGG AGC GGC GGG CCC CAG GCA AAC TTG CAC GAC
                            1500

ALA CYS GLN GLY ASP SER GLY GLY PRO LEU VAL CYS LEU ASN ASP GLY ARG MET THR LEU   446
GCC TGC CAG GGC GAT TCG GGA GGC CCC CTG GTG TGT CTG AAC GAT GGC CGC ATG ACT TTG
                1550

VAL GLY ILE ILE SER TRP GLY LEU GLY CYS GLY GLN LYS ASP VAL PRO GLY VAL TYR THR   466
GTG GGC ATC ATC AGC TGG GGC CTG GGC TGT GGA CAG AAG GAT GTC CCG GGT GTG TAC ACC
1600                                                                 1650

                                                            481
LYS VAL THR ASN TYR LEU ASP TRP ILE ARG ASP ASN MET ARG PRO XXX
AAG GTT ACC AAC TAC CTA GAC TGG ATT CGT GAC AAC ATG CGA CCG TGA CCAGGAACACCCGA
                                                1700
```

EP 0 231 883 B1

## Fig. I-5

CTCCTCAAAAGCAAATGAGATCCCGCCTCTTCTTCTTCAGAAGACACTGCAAAGGCGCAGTGCTTCTCTACAGACTTCTC

CAGACCCACCACACCGCAGAAGCGGGACGAGACCCTACAGGAGAGGGAAGAGTGCATTTTCCCTGATACTTCCCATTTTG

GAAGTTTTCAGGACTTGGTCTGATTTCAGGATACTCTGTCAGATGGGAAGACATGAATGCACACTAGCCTCTCCAGGAAT

GCCTCCTCCCTGGGCAGAAGTGGCCATGCCACCCTGTTTTCGCTAAAGCCCAACCTCCTGACCTGTCACCGTGAGCAGCT

TTGGAAACAGGACCACAAAAATGAAAGCATGTGTCAATAGTAAAAGATAACAAGATCTTTCAGGAAAGACGGATTGCATT

AGAAATAGACAGTATATTTATAGTCACAAGAGCCCAGCAGGGCTCAAAGTTGGGGCAGGCTGGCTGGCCCGTCATGTTCC

TCAAAAGCACCCTTGACGTCAAGTCTCCTTCCCCTTTCCCCACTCCCTGGCTCTCAGAAGGTATTCCTTTTGTGTACAGT

GTGTAAAGTGTAAATCCTTTTTTCTTTATAAACTTTAGAGTAGCATGAGAGAATTGTATCATTTGAACAACTAGGCTTCAG

CATATTTATAGCAATCCATGTTAGTTTTTACTTTCTGTTGCCACAACCCTGTTTTATACTGTACTTAATAAATTCAGATA

TATTTTTCACAGTTTTTCC

EP 0 231 883 B1

## Fig. 2-1

```
                                                                          met arg ala
CAGCGCCGGCTCGCGCCCTCCTGCCGCAGCCACCGAGCCGCCGTCTAGCGCCCCGACCTCGCCACC  ATG AGA GCC
1                                                          50

                                                                            1
leu leu ala arg leu leu leu cys val leu val val ser asp ser lys gly SER ASN GLU    3
CTG CTG GCG CGC CTG CTT CTC TGC GTC CTG GTC GTG AGC GAC TCC AAA GGC AGC AAT GAA
                            100


LEU HIS GLN VAL PRO SER ASN CYS ASP CYS LEU ASN GLY GLY THR CYS VAL SER ASN LYS   23
CTT CAT CAA GTT CCA TCG AAC TGT GAC TGT CTA AAT GGA GGA ACA TGT GTG TCC AAC AAG
                150


TYR PHE SER ASN ILE HIS TRP CYS ASN CYS PRO LYS LYS PHE GLY GLY GLN HIS CYS GLU   43
TAC TTC TCC AAC ATT CAC TGG TGC AAC TGC CCA AAG AAA TTC GGA GGG CAG CAC TGT GAA
       200                                                          250


ILE ASP LYS SER LYS THR CYS TYR GLU GLY ASN GLY HIS PHE TYR ARG GLY LYS ALA SER   63
ATA GAT AAG TCA AAA ACC TGC TAT GAG GGG AAT GGT CAC TTT TAC CGA GGA AAG GCC AGC
                                                    300


THR ASP THR MET CLY ARG PRO CYS LEU PRO TRP ASN SER ALA THR VAL LEU GLN GLN THR   83
ACT GAC ACC ATG GGC CGG CCC TGC CTG CCC TGG AAC TCT GCC ACT GTC CTT CAG CAA ACG
                                    350


TYR HIS ALA HIS ARG SER ASP ALA LEU GLN LEU GLY LEU GLY LYS HIS ASN TYR CYS ARG  103
TAC CAT GCC CAC AGA TCT GAT GCT CTT CAG CTG GGC CTG GGG AAA CAT AAT TAC TGC AGG
                        400
```

EP 0 231 883 B1

# Fig. 2-2

```
ASN PRO ASP ASN ARG ARG ARG PRO TRP CYS TYR VAL GLN VAL GLY LEU LYS LEU PRO VAL    123
AAC CCA GAC AAC CGG AGG CGA CCC TGG TGC TAT GTG CAG GTG GGC CTA AAG CCG CTT GTC
             450
                                        ⇩
GLN GLU CYS MET VAL HIS ASP CYS ALA ASP GLY LYS LYS PRO SER SER PRO PRO GLU GLU    143
CAA GAG TGC ATG GTG CAT GAC TGC GCA GAT GGA AAA AAG CCC TCC TCT CCT CCA GAA GAA
             500                                                          550
                          ⇩                              ⇩
LEU LYS PHE GLN CYS GLY GLN LYS THR LEU ARG PRO ARG PHE LYS ILE ILE GLY GLY GLU    163
TTA AAA TTT CAG TGT GGC CAA AAG ACT CTG AGG CCC CGC TTT AAG ATT ATT GGG GGA GAA
                                                          600
PHE THR THR ILE GLU ASN GLN PRO TRP PHE ALA ALA ILE TYR ARG ARG HIS ARG GLY GLY    183
TTC ACC ACC ATC GAG AAC CAG CCC TGG TTT GCG GCC ATC TAC AGG AGG CAC CGG GGG GGC
                                             650
SER VAL THR TYR VAL CYS GLY GLY SER LEU ILE SER PRO CYS TRP VAL ILE SER ALA THR    203
TCT GTC ACC TAC GTG TGT GGA GGC AGC CTC ATC AGC CCT TGC TGG GTG ATC AGC GCC ACA
                              700
HIS CYS PHE ILE ASP TYR PRO LYS LYS GLU ASP TYR ILE VAL TYR LEU GLY ARG SER ARG    223
CAC TGC TTC ATT GAT TAC CCA AAG AAG GAG GAC TAC ATC GTC TAC CTG GGT CGC TCA AGG
                 750
LEU ASN SER ASN THR GLN GLY GLU MET LYS PHE GLU VAL GLU ASN LEU ILE LEU HIS LYS    243
CTT AAC TCC AAC ACG CAA GGG GAG ATG AAG TTT GAG GTG GAA AAC CTC ATC CTA CAC AAG
         800                                                          850
```

EP 0 231 883 B1

## Fig. 2-3

```
ASP TYR SER ALA ASP THR LEU ALA HIS HIS ASN ASP ILE ALA LEU LEU LYS ILE ARG SER    263
GAC TAC AGC GCT GAC ACG CTT GCT CAC CAC AAT GAC ATT GCC TTG CTG AAG ATC CGT TCC
                                                        900

LYS GLU GLY ARG CYS ALA GLN PRO SER ARG THR ILE GLN THR ILE CYS LEU PRO SER MET    283
AAG GAG GGC AGG TGT GCG CAG CCA TCC CGG ACT ATA CAG ACC ATC TGC CTG CCC TCG ATG
                                            950

TYR ASN ASP PRO GLN PHE GLY THR SER CYS GLN ILE THR GLY PHE GLY LYS GLU ASN SER    303
TAT AAC GAT CCC CAG TTT GGC ACA AGC TGT GAG ATC ACT GGC TTT GGA AAA GAG AAT TCT
                                1000

THR ASP TYR LEU TYR PRO GLU GLN LEU LYS MET THR VAL VAL LYS LEU ILE SER HIS ARG    323
ACC GAC TAT CTC TAT CCG GAG CAG CTG AAA ATG ACT GTT GTG AAG CTG ATT CCC CAC CGG
                    1050

GLU CYS GLN GLN PRO HIS TYR TYR GLY SER GLU VAL THR THR LYS MET LEU CYS ALA ALA    343
GAG TGT CAG CAG CCC CAC TAC TAC GGC TCT GAA GTC ACC ACC AAA ATG CTG TGT GCT GCT
    1100                                                              1150

ASP PRO GLN TRP LYS THR ASP SER CYS GLN GLY ASP SER GLY GLY PRO LEU VAL CYS SER    363
GAC CCA CAG TGG AAA ACA GAT TCC TGC CAG GGA GAC TCA GGG GGA CCC CTC GTC TGT TCC
                                                    1200

LEU GLN GLY ARG MET THR LEU THR GLY ILE VAL SER TRP GLY ARG GLY CYS ALA LEU LYS    383
CTC CAA GGC CGC ATG ACT TTG ACT GGA ATT GTG AGC TGG GGC CGT GGA TGT GCC CTG AAG
                                        1250
```

EP 0 231 883 B1

Fig. 2-4

```
ASP LYS PRO GLY VAL TYR THR ARG VAL SER HIS PHE LEU PRO TRP ILE ARG SER HIS THR      403
GAC AAG CCA GGC GTC TAC ACG AGA GTC TCA CAC TTC TTA CCC TGG ATC CGC AGT CAC ACC
            1300


                                    411
LYS GLU GLU ASN GLY LEU ALA LEU XXX
AAG GAA GAG AAT GGC CTG GCC CTC TGA GGGTCCCCAGGGAGGAAACGGGCACCACCCGCTTTCTTGCTGG
                1350                                                        1400

TTGCTATTTTGCAGTAGAGTCATCTCCATCAGCTGTAAGAAGAGACTGGGAAGATAGGCTCTGCACAGATGGATTTGCC
                                                        1450

TGTGCCCACCACCAGGGCGAACGACAATAGCTTTACCCTCAGGCATAGGCCTGGGTGCTGGCTGCCCAGACCCCTCTGG
                1500                                                    1550

CCAGGATGGAGGGGTGGTCCTGACTCAACATGTTACTGACCAGCAACTTGTCTTTTTCTGGACTGAAGCCTGCAGGAGT
                                            1600

TAAAAAGGGCAGGGCATCTCCTGTGCATGGGCTCGAAGGGAGAGCCAGCTCCCCCGAGCGGTGGGCATTTGTGAGGCCC
        1650                                                1700

ATGGTTGAGAAATGAATAATTTCCCAATTAGGAAGTGTAAGCAGCTGAGGTCTCTTGAGGGAGCTTAGCCAATGTGGGA
                            1750

GCAGCGGTTTGGGGAGCAGAGACACTAACGACTTCAGGGCAGGGCTCTGATATTCCATGAATGTATCAGGAAATATATA
1800                                                        1850

TGTGTGTGTATGTTTGCACACTTGTGTGTGGGCTGTGAGTGTAAGTGTGAGAAAGAGCTGGTGTCTGATTGTTAAGTCT
                    1900                                                1950
```

EP 0 231 883 B1

Fig. 2- 5

AAATATTTCCTTAAACTGTGTGGACTGTGATGCCACACAGAGTGGTCTTTCTGGAGAGGTTATAGGTCACTCCTGGGGC
2000

CTCTTGGGTCCCCCACGTGACAGTGCCTGGGAATGTATTATTCTGCAGCATGACCTGTGACCAGCACTGTCTCAGTTTC
2050                                                                      2100

ACTTTCACATAGATGTCCCTTTCTTGGCCAGTTATCCCTTCCTTTTAGCCTAGTTCATCCAATCCTCACTGGGTGGGGT
2150

GAGGACCACTCCTGTACACTGAATATTTATATTTCACTATTTTTATTTATATTTTTGTAATTTTAAATAAAAGTGATCA
2200                                                                      2250

ATAAAATGTGATTTTTCTGATGAAAAA
2294

EP 0 231 883 B1

# Fig. 3

EP 0 231 883 B1

N-Terminal Fibrin-
Affinity Region
Corresponding to Tissue
Plasminogen Activator

C-Terminal Fibrinolytic
Active Region Corresponding
to Urokinase

Symbol of
Polypeptide

Fig. 3 (Continue)

| N-Terminal Fibrin-Afinity Region Corresponding to Plasminogen Activator | C-Terminal Fibrinolytic Active Region Corresponding to Urokinase | Symbol of Polypeptide |

1 ——————————— 215 132 135 ————————————————— 411
Ser                    Cys Ala Gln                        Pro        HPA 21L

1 ——————————— 215 132 135   157 ——————————— 411
Ser                    Cys Ala Gln  Acidic Amino Acid     Pro        HPA 24L

128 ————— 215 132 135 ————————————————— 411
Ser            Cys Ala Gln                        Pro        HPA11L

128 ————— 215 132 135   157 ——————————— 411
Ser            Cys Ala Gln  Acidic Amino Acid     Pro        HPA 14L

161 —— 215 132 135 ————————————————— 411
Met       Cys Ala Gln                        Pro        HPA 01L

161 —— 215 132 135   157 ——————————— 411
Met       Cys Ala Gln  Acidic Amino Acid     Pro        HPA 04L

EP 0 231 883 B1

# Fig. 4

# Fig. 5

# Fig. 6

# Fig. 7

# Fig. 8

met ser asn glu leu his gln val pro ser

5′  C ATG AGC AAC GAG CTC CAC CAC GTT CCGT    3′

3′TGCAG TAC TCG TTG CTC GAG GTG GTC CAA GGC AGC 5′

Aat II                                                      Tag I

EP 0 231 883 B1

Fig. 9

# *Fig. 10*

# Fig. 11

```
                    157
        Pro  Arg  Asp  Lsy  Ile
5'··· G GCCC CGC GAT AAG ATTA···3'
3'··TCCGGG GCG AAATT CTAAT···5'
        Arg Pro Arg Phe Lys Ile
                    157
```

Single Strand
Template
DNA

Anneal 60°C, 20min.; 20°C, 20min.
DNA Polymerase I Klenow Fragment + dNTPs
T4 DNA Ligase

Transformation into E. coli JM103

Screening by
Plaque Hybridization

Plaque

# Fig. 12

EP 0 231 883 B1

# Fig. 13

61

# Fig. 14

# Fig. 15

Fig. 16

Fig. 17

EP 0 231 883 B1

# Fig. 18-1

COMMERCIAL TPA

COMMERCIAL UK

# Fig. 18-2

# Fig. 18-3

# Fig. 18-4

HA23

UNIT OF ACTIVITY

ADSORBED FRACTION

←—NON - ADSORBED FRACTION

10

5          10          15          20

FRACTION NO.

EP 0 231 883 B1

Fig. 19

Fig. 20

Construction of pHA21L

Fig. 21

Construction of pHA11L

# Fig. 22

Construction of pHA 01L

# Fig.23